# EUROPÄISCHE PATENTSCHRIFT

(11) **EP 3 041 820 B1**
(45) Veröffentlichungstag und Bekanntmachung des Hinweises auf die Patenterteilung: **03.07.2019**
(21) Anmeldenummer: 14765893.4
(22) Anmeldetag: 02.09.2014
(51) Int. Cl.: C07C 227/18, C07K 14/195

(54) **[S,S]-EDDS BIOSYNTHESEGENE UND -PROTEINE UND VERFAHREN ZUR BIOSYNTHESE VON [S,S]-EDDS**
[S,S]-EDDS BIOSYNTHESIS GENES AND PROTEINS AND METHOD FOR THE BIOSYNTHESIS OF [S,S]-EDDS
GÈNE ET PROTÉINE DE BIOSYNTHÈSE DE [S,S]-EDDS, ET PROCÉDÉ POUR LA BIOSYNTHÈSE DE [S,S]-EDDS

(30) Priorität: 03.09.2013 DE 102013217543
(43) Veröffentlichungstag der Anmeldung: 13.07.2016
(73) Patentinhaber: Stegmann, Efthimia, 72108 Rottenburg/Wurmlingen (DE); Wohlleben, Wolfgang, 72074 Tübingen (DE); Spohn, Marius, 88069 Tettnang (DE); Weber, Tilman, 72074 Tübingen (DE)
(72) Erfinder: Stegmann, Efthimia, 72108 Rottenburg/Wurmlingen (DE); Wohlleben, Wolfgang, 72074 Tübingen (DE); Spohn, Marius, 88069 Tettnang (DE); Weber, Tilman, 72074 Tübingen (DE)
(74) Vertreter: Patentanwälte Ruff, Wilhelm, Beier, Dauster & Partner mbB
(86) Internationale Anmeldenummer: PCT/EP2014/068612
(87) Internationale Veröffentlichungsnummer: WO 2015/032751

(56) Entgegenhaltungen:
- WO-A1-96/36725
- EFTHIMIA STEGMANN ET AL: "Development of three different gene cloning systems for genetic investigation of the new species Amycolatopsis japonicum MG417-CF17, the ethylenediaminedisuccinic acid producer", JOURNAL OF BIOTECHNOLOGY, Bd. 92, Nr. 2, 1. Dezember 2001 (2001-12-01), Seiten 195-204, XP055154530, ISSN: 0168-1656, DOI: 10.1016/S0168-1656(01)00360-1
- Michael Goodfellow ET AL: "Amycolatopsis japonicum sp. nov., an Actinomycete Producing (S,S)-N,N'-Ethylenediaminedisuccinic Acid", Systematic and Applied Microbiology, 1. Januar 1997 (1997-01-01), Seiten 78-84, XP055154526, DOI: 10.1016/S0723-2020(97)80051-3 Gefunden im Internet: URL:http://www.sciencedirect.com/science/a rticle/pii/S0723202097800513 [gefunden am 2014-11-24]
- TAKAHASHI R ET AL: "PRODUCTION OF (S,S)-ETHYLENEDIAMINE-N,N'-DISUCCINIC ACID FROM ETHYLENEDIAMINE AND FUMARIC ACID BY BACTERIA", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, Bd. 63, Nr. 7, 1. Januar 1999 (1999-01-01) , Seiten 1269-1273, XP009033534, ISSN: 0916-8451, DOI: 10.1271/BBB.63.1269
- BIAO TANG ET AL: "ContigScape: a Cytoscape plugin facilitating microbial genome gap closing", BMC GENOMICS, BIOMED CENTRAL LTD, LONDON, UK, Bd. 14, Nr. 1, 30. April 2013 (2013-04-30) , Seite 289, XP021147878, ISSN: 1471-2164, DOI: 10.1186/1471-2164-14-289 -& DATABASE UniProt [Online] 24. Juli 2013 (2013-07-24), "SubName: Full=Fur family transcriptional regulator, ferric uptake regulator {ECO:0000313|EMBL:AGM04681.1};", XP002732911, gefunden im EBI accession no. UNIPROT:R4SX04 Database accession no. R4SX04
- EVI STEGMANN ET AL: "Complete genome sequence of the actinobacterium Amycolatopsis japonica MG417-CF17T (=DSM 44213T) producing (S,S)-N,N'-ethylenediaminedisuccinic acid", JOURNAL OF BIOTECHNOLOGY, Bd. 189, 3. September 2014 (2014-09-03), Seiten 46-47, XP055154510, ISSN: 0168-1656, DOI: 10.1016/j.jbiotec.2014.08.034

### SEQUENCE LISTING

<110> Eberhard Karls Universitaet Tuebingen
<120> S,S-EDDS Biosynthesegencluster und Verfahren zur Biosynthese von S,S-EDDS
<130> P 53 563 WO
<160> 64
<170> BiSSAP 1.2
<210> 1
   <211> 541
   <212> PRT
   <213> Amycolatopsis japonicum
<220>
   <223> ORF 1639
<400> 1
   Met Thr Arg Lys Glu Pro Ala Asp Thr Pro Leu Gly Asp Ala Phe His
   1               5                   10                  15
   Leu Tyr Asp Thr Thr Leu Arg Asp Gly Ala Gln Arg Glu Gly Ile Ser
               20                  25                  30
   Tyr Ser Val Ala Asp Lys Leu Ala Val Ala Arg Leu Leu Asp Glu Leu
           35                  40                  45
   Gly Val Gly Phe Ile Glu Gly Gly Trp Pro Gly Ala Leu Pro Lys Asp
       50                  55                  60
   Thr Glu Phe Phe Ala Arg Ala Ala Ser Gly Glu Leu Lys Leu Arg His
   65                  70                  75                  80
   Ala Ala Leu Val Ala Phe Gly Ala Thr Arg Lys Ala Gly Thr Lys Ala
                   85                  90                  95
   Glu Gln Asp Gln Gln Val Arg Ala Leu Leu Asp Ser Gln Ala Pro Val
               100                 105                 110
   Val Thr Leu Val Ala Lys Ser Asp Leu Arg His Ile Glu Arg Ala Leu
           115                 120                 125
   Arg Val Asp Val Asp Glu Ala Cys Glu Met Val Arg Asp Thr Val Ala
       130                 135                 140
   Phe Leu Val Gly Glu Gly Arg Arg Val Phe Leu Asp Ala Glu His Phe
   145                 150                 155                 160
   Phe Asp Gly Tyr Ala Phe Ser Pro Asp Thr Ala Leu Lys Val Leu Asp
                   165                 170                 175
   Ala Gly Val Asn Ala Gly Ala Asp Val Ala Val Leu Cys Asp Thr Asn
               180                 185                 190
   Gly Gly Gln Leu Pro Leu Gly Leu Ala Glu Thr Val Arg Glu Val Lys
           195                 200                 205
   Glu Arg Thr Gly Phe Arg Leu Gly Ile His Cys Gln Asp Asp Thr Ser
       210                 215                 220
   Cys Ala Val Ala Asn Ser Val Ala Ala Val Gln Ala Gly Val Thr His
   225                 230                 235                 240
   Val Gln Cys Thr Ala Asn Gly Tyr Gly Glu Arg Ala Gly Asn Ala Asp
                   245                 250                 255
   Leu Phe Ala Val Thr Gly Asn Leu Val Thr Lys Leu Gly Met Glu Val
               260                 265                 270
   Leu Pro Thr Gly Gly Ala Ala Glu Leu Thr Arg Val Ser His Ala Leu
           275                 280                 285
   Ala Glu Ile Ala Asn Ile Ala Pro Tyr Thr His Gln Ala Tyr Val Gly
       290                 295                 300
   Ala Ser Ala Phe Ala His Lys Ala Gly Leu His Ala Ser Ala Ile Lys
   305                 310                 315                 320
   Val Asp Pro Leu Leu Tyr Asn His Ile Asp Pro Ala Ser Val Gly Asn
                   325                 330                 335
   Asp Met Arg Val Leu Val Thr Glu Met Ala Gly Arg Ala Ser Leu Glu
               340                 345                 350
   Leu Lys Gly Arg Glu Leu Gly Val Asp Leu Ala Gly Gln Pro Glu Ala
           355                 360                 365
   Leu Thr Ser Ala Val Lys Lys Val Lys Arg Leu Glu Ser Glu Gly Trp
       370                 375                 380
   Ser Phe Glu Ala Ala Asp Ala Ser Leu Glu Leu Leu Leu Arg Arg Glu
   385                 390                 395                 400
   Val Asp Gly Pro Gln Ser Asp Val Leu Asp Glu Pro Pro Phe Glu Leu
                   405                 410                 415
   Glu Ser Tyr Arg Val Val Leu Asp His Arg Ser Asp Gly Glu Val Val
               420                 425                 430
   Ser Glu Ala Thr Val Lys Val His Val Ala Gly Gln Arg Val Ile Ala
           435                 440                 445
   Thr Ala Glu Gly Asn Gly Pro Val His Ala Leu Asp Ala Ala Leu Arg
       450                 455                 460
   Glu Ala Leu Ser Pro His Leu Ser Trp Leu Asp Ser Val Glu Leu Ala
   465                 470                 475                 480
   Asp Tyr Lys Val Arg Ile Leu Pro Gly His Pro Gly Thr Asp Ala Val
                   485                 490                 495
   Thr Arg Val Leu Val Glu Thr Ser Asp Gly Glu Arg Glu Trp Thr Thr
               500                 505                 510
   Val Gly Val His Gly Asn Ile Val Glu Ala Ser Trp Leu Ala Leu Cys
           515                 520                 525
   Asp Ala Leu Val His Lys Ser Leu Ser Glu Gly Pro Ala
       530                 535                 540
<210> 2
   <211> 1626
   <212> DNA
   <213> Amycolatopsis japonicum
<220>
   <223> orf 1639
<400> 2
   gtgacccgca aggagcccgc cgatacaccg ctcggcgacg ccttccacct gtacgacacg        60
   acgttgcgcg acggtgcgca gcgtgagggg atctcctact ccgtcgcgga caaactggcg       120
   gtggcgaggc tgctcgacga actcggggtc gggttcatcg aaggcggatg gccgggcgcg       180
   ctgcccaagg acacggaatt cttcgcccgt gcggcttcgg gcgagctgaa acttcgccat       240
   gccgcgctcg tcgcgttcgg cgcgacgcgg aaggcgggca ccaaggccga gcaggatcag       300
   caggtgcggg ccctgctcga ctcgcaggca ccggtggtca cgctcgtggc caaatccgat       360
   ctgcggcaca tcgaacgcgc gctgcgggtc gacgtcgacg aagcttgcga gatggtgcgc       420
   gacaccgtcg cgttcctcgt cggtgaagga cgtcgcgtct tccttgacgc ggaacacttt       480
   ttcgacggct acgcgttctc cccggacacc gcgctgaagg tgctcgacgc gggcgtgaac       540
   gcgggtgccg acgtcgccgt gctgtgcgac accaacggcg ggcaactgcc gctcggactc       600
   gcggaaaccg tccgcgaggt caaggaaagg accggattcc ggctcggaat ccattgtcag       660
   gacgacactt cctgcgcggt ggcgaattcc gtcgccgccg tgcaggccgg cgtgacgcac       720
   gtccagtgca ccgccaatgg ttacggcgag cgggcgggta acgccgatct tttcgccgtg       780
   acagggaatt tggtgaccaa gctcggtatg gaggtgctcc cgaccggagg cgccgccgag       840
   ctcacccggg tctcccatgc ccttgccgaa atcgcgaaca tcgcccccta cacccaccag       900
   gcttacgtag gggcgtcggc cttcgcccac aaggcgggac tgcacgcgag cgcgatcaag         960
   gtggatccgt tgctgtacaa ccacatcgat ccagcttctg tcggcaatga catgcgggta      1020
   ctggtcaccg agatggccgg cagggccagc ctggagctca agggacgtga gctcggggtc      1080
   gaccttgccg gccagcccga ggcgctgacg agcgccgtga agaaggtgaa acgcctcgaa      1140
   tccgaaggct ggtccttcga agccgcggac gcttcactgg aactgctgct gcggcgggag      1200
   gtcgacggcc cgcagagcga cgtcctcgac gaaccgccgt tcgaactcga gtcgtaccgg      1260
   gtcgtcctcg accaccggtc cgacggcgag gtcgtctccg aggcgacggt gaaggtgcac      1320
   gtcgccgggc agcgcgtgat cgcgaccgcc gagggcaacg ggcccgtgca cgcgctcgac      1380
   gccgccctgc gcgaggcgct cagcccgcat ctgtcctggt tggacagtgt cgagctggcc      1440
   gactacaagg tgcggatcct gcccggccat ccgggcaccg acgccgtcac ccgtgtgctc      1500
   gtcgagacca gcgacggcga gcgcgagtgg accaccgtcg gcgtgcacgg caacatcgtc      1560
   gaggcgagct ggctggcgct gtgcgacgcg ctcgtgcaca agtccctcag cgagggcccg      1620
   gcgtga                                                                  1626
<210> 3
   <211> 256
   <212> PRT
   <213> Amycolatopsis japonicum
<220>
   <223> ORF 1640
<400> 3
   Met Arg Leu Ala Arg Ile Ala His Pro Gly Gly Val Ala Phe Ala Ser
   1               5                   10                  15
   Ile Glu Gly Asp Gly Asp Asp Ala Gln Val Leu Glu Ile Ala Glu His
               20                  25                  30
   Pro Phe Gly Asn Pro Asn Phe Thr Gly Lys Arg Trp Pro Leu Ala Asp
           35                  40                  45
   Val Arg Leu Leu Ala Pro Ile Leu Pro Ser Lys Val Ile Ala Val Gly
       50                  55                  60
   Arg Asn Tyr Ala Lys His Ala Ala Glu Phe Gly Asn Glu Val Pro Gln
   65                  70                  75                  80
   Asp Pro Met Leu Phe Ile Lys Pro Ser Thr Thr Val Ile Gly Pro Asn
                   85                  90                  95
   Val Pro Ile Arg Arg Pro Arg Gly Val Gly Arg Val Asp Phe Glu Gly
               100                 105                 110
   Glu Leu Ala Ile Val Ile Gly Gln Pro Val Lys Asn Val Pro Ala Ala
           115                 120                 125
   Arg Ala Ala Ser Ala Ile Leu Gly Tyr Thr Val Ala Asn Asp Val Ser
       130                 135                 140
   Ala Arg Asp Leu Gln Lys Ser Asp Gly Gln Trp Gly Arg Ala Lys Gly
   145                 150                 155                 160
   Phe Asp Thr Phe Cys Pro Leu Gly Pro Trp Ile Glu Thr Ser Leu Asp
                   165                 170                 175
   Ala Ala Asp Leu Ala Leu Lys Ser Glu Val Asp Gly Val Leu Lys Gln
               180                 185                 190
   Asp Gly Arg Thr Ser Asp Leu Val His Lys Ile Pro Glu Leu Val Glu
           195                 200                 205
   Phe Val Ser Gly Val Met Thr Leu Leu Pro Gly Asp Val Ile Leu Thr
       210                 215                 220
   Gly Thr Pro Glu Gly Val Gly Pro Ile Glu Asp Gly Gln Ser Val Ser
   225                 230                 235                 240
   Ile Thr Ile Glu Gly Ile Gly Thr Leu Thr Asn Pro Val Gln Asp Ile
                   245                 250                 255
<210> 4
   <211> 771
   <212> DNA
   <213> Amycolatopsis japonicum
<220>
   <223> orf 1640
<400> 4
   gtgcgtctag cccgaattgc tcatcccggt ggtgtcgcgt tcgcttcgat cgaaggggac      60
   ggcgacgacg cccaggtact ggaaatcgcc gagcacccct tcggcaaccc caacttcacc     120
   ggcaaacgct ggccgctcgc cgacgtccgg ctgctcgcgc cgatcctgcc gtcgaaggtg     180
   atcgcggtcg gccggaacta cgccaagcac gcggccgagt tcggcaacga ggtcccgcag     240
   gacccgatgc tgttcatcaa gccgtcgacc acggtgatcg ggccgaacgt gccgatccgc     300
   cgtccgcgcg gtgtcggccg tgtcgacttc gagggcgaac tggcgatcgt catcggccag     360
   ccggtcaaga acgtgcccgc cgcccgcgcg gcgagcgcga tcctcggcta caccgtggcg     420
   aacgacgtca gcgcgcgtga cctgcagaag tccgacggcc agtggggcag ggcgaagggt     480
   ttcgacacct tctgcccgct gggcccgtgg atcgagacct cgctcgacgc ggccgatctc     540
   gcgctcaagt ccgaggtgga cggtgtgctc aagcaggacg gccggacgtc ggacctggtg     600
   cacaagatcc ccgagctggt cgaattcgtg tccggggtga tgaccctgtt gcccggtgac     660
   gtcatcctga ccggcacgcc cgagggcgtc ggcccgatcg aggacggcca gtccgtgtcg     720
   atcaccatcg agggcatcgg caccctgacg aacccggttc aagacatcta g              771
<210> 5
   <211> 411
   <212> PRT
   <213> Amycolatopsis japonicum
<220>
   <223> ORF 1641
<400> 5
   Met Asn Glu Arg Thr Asn Cys Val Val Val Gly Gly Gly Pro Ala Gly
   1               5                   10                  15
   Met Val Ala Gly Leu Leu Leu Ala Arg Ala Gly Val Glu Val Thr Val
               20                  25                  30
   Leu Glu Lys His Gln Asp Phe Leu Arg Asp Phe Arg Gly Asp Thr Val
           35                  40                  45
   His Pro Ser Thr Leu Thr Leu Leu Asp Glu Leu Gly Leu Ser Glu Lys
       50                  55                  60
   Phe Leu Ser Leu Pro His Ser Glu Ile Ala Tyr Ala Gly Phe Pro Ala
   65                  70                  75                  80
   Glu Asp Gly Thr Met Met Arg Leu Ala Asp Leu Thr Arg Leu Lys Val
                   85                  90                  95
   Ala His Pro Tyr Ile Ala Met Val Pro Gln Trp Asp Phe Leu Asp Leu
               100                 105                 110
   Leu Ala Asp Ala Gly Ala Lys Glu Pro Thr Phe Thr Leu Arg Gln Ser
           115                 120                 125
   Thr Glu Met Thr Gly Leu Ile Phe Glu His Gly Arg Val Ser Gly Val
       130                 135                 140
   Arg Tyr Arg Asp Ala Asp Gly Lys Ala Gly Glu Leu Arg Ala Asp Leu
   145                 150                 155                 160
   Val Ile Ala Ala Asp Gly Arg Trp Ser Leu Ala Arg Arg Glu Ala Gly
                   165                 170                 175
   Leu Met Thr Lys Asp Tyr Pro Ile Pro Phe Asp Ala Trp Trp Phe Arg
               180                 185                 190
   Ile Ser Arg Arg Asp Gly Glu Arg Glu Gly Met Leu Thr Pro Lys Met
           195                 200                 205
   Arg Asp Arg Arg Phe Ala Val Pro Leu Pro Arg Lys Gly Tyr Phe Gln
       210                 215                 220
   Ile Ala Tyr Leu Ser Pro Lys Gly Gln Asp Leu Arg Asp Lys Gly Ile
   225                 230                 235                 240
   Glu Ala Phe Arg Glu Asn Val Ala Ala Ile Leu Pro Glu Leu Ala Asp
                   245                 250                 255
   Arg Val Asp Glu Leu Lys Thr Thr Asp Asp Val Lys Phe Leu Asp Val
               260                 265                 270
   Lys Met Asn Leu Leu Arg Lys Trp His Leu Asp Gly Leu Leu Cys Ile
           275                 280                 285
   Gly Asp Ala Ala His Ala Met Ser Pro Val Gly Gly Val Gly Ile Asn
       290                 295                 300
   Leu Ala Val Gln Asp Ala Val Ala Ala Ala Thr Leu Leu Ala Glu Pro
   305                 310                 315                 320
   Leu Arg Arg Gly Arg Pro Ser Val Ala Asp Leu Ala Lys Val Arg Lys
                   325                 330                 335
   Arg Arg Leu Ala Pro Thr Met Leu Val Gln Gly Leu Gln Arg Ile Leu
               340                 345                 350
   His Lys Asn Val Met Gly Pro Val Met Ala Gly Lys Arg Asn Gly Pro
           355                 360                 365
   Pro Ala Pro Met Val Lys Leu Phe Ser Arg Phe Pro Ala Leu Ser Tyr
       370                 375                 380
   Ile Pro Ala Arg Leu Leu Gly Leu Gly Leu Arg Pro Glu His Ala Pro
   385                 390                 395                 400
   Ala Phe Ala Arg Arg Ala Met Glu Pro Ala Gly
                   405                 410
<210> 6
   <211> 1236
   <212> DNA
   <213> Amycolatopsis japonicum
<220>
   <223> orf 1641
<400> 6
   atgaacgaac gtacgaactg cgtggtcgtc ggcggtggcc ccgcgggtat ggtcgccgga      60
   ttgctgctgg ccagggccgg ggtcgaggtc accgtgctgg agaagcacca ggacttcctg     120
   cgggacttcc gcggcgacac cgtgcacccg tcgacgttga cgttgctgga cgagctgggc     180
   ctcagcgaga aattcctcag cctgccgcac agcgagatcg cctacgccgg cttccccgcc     240
   gaggacggca cgatgatgcg cctcgccgac ctgaccaggc tgaaggtggc gcacccgtac     300
   atcgccatgg tcccgcagtg ggacttcctg gacctgctcg ccgacgccgg ggcgaaggaa     360
   cccacgttca cgctgcggca gagcaccgag atgaccgggc tgatcttcga acacggccgg     420
   gtgagcgggg tccgctaccg cgacgccgac gggaaggccg gcgaactgcg cgccgacctg     480
   gtgatcgccg ccgacggacg ctggtcgctg gcccgccgcg aggccgggct catgacgaag     540
   gactacccca tcccgttcga cgcgtggtgg ttccggatct cacggcggga cggggaacgc     600
   gaaggcatgc tgaccccgaa gatgcgggat cgccgcttcg ccgtgccgct gccccgcaag     660
   ggctacttcc agatcgccta cctcagcccc aagggccagg acctgcgcga caagggcatc     720
   gaggcgttcc gggagaacgt ggccgcgatc ctgcccgagc tggccgaccg cgtggacgag     780
   ctgaagacga cggacgacgt caaattcctc gacgtcaaga tgaacctgct gcggaagtgg     840
   catctcgacg ggctgctgtg catcggtgac gcggcgcacg cgatgtcgcc ggtcggcggg     900
   gtcggcatca acctcgcggt gcaggacgcg gtcgcggccg ccaccctgct cgccgaaccg     960
   ctgcgccgcg gcaggccgtc ggtggcggat ctggcgaagg tccgcaagcg ccgtctcgcg    1020
   cccacgatgc tggtgcaggg gctgcagcgg attctgcaca agaacgtgat ggggccggtg    1080
   atggcgggca agcgcaacgg cccgcccgcg ccgatggtga agctgttcag ccggttcccc    1140
   gcgctgtcct acatccccgc gcggctgctg ggcctggggc tgcggcccga gcacgccccg    1200
   gccttcgcgc ggcgggcgat ggaaccggcc ggttag                              1236
<210> 7
   <211> 244
   <212> PRT
   <213> Amycolatopsis japonicum
<220>
   <223> ORF 1642
<400> 7
   Met Cys Leu Asp Ile Asp Asp Thr Leu Ile Asp Cys Thr Ala Ala Ile
   1               5                   10                  15
   Arg Leu Ser Leu Ser Ala Leu Thr Gly Gln Asn Asp Leu Trp Pro Leu
               20                  25                  30
   Trp Asp Leu Ile Thr Glu Glu His Val Ala Leu Val Val Ala Gly Glu
           35                  40                  45
   Ile Asp Tyr Ala Thr Met His Tyr Lys Arg Thr Glu Cys Phe Leu Ala
       50                  55                  60
   Glu Ile Gly Ile Leu Ala Asp Glu Gln Gln Val Ser Gly Phe Glu Arg
   65                  70                  75                  80
   Arg Arg Arg Glu Ile Leu Thr Arg Ser Trp Gln Leu Phe Glu Asp Val
                   85                  90                  95
   Leu Pro Cys Leu Glu Trp Leu Arg Ala Ala Gly Val Leu Val Ala Ala
               100                 105                 110
   Val Thr Asn Ala Ser Gly Ala His Gln Arg Lys Lys Ile Ala Asp Leu
           115                 120                 125
   Gly Leu Ala Arg Phe Phe Asp His Val Ala Ile Ala Gly Glu Leu Gly
       130                 135                 140
   Val Ala Lys Pro Asp Pro Ala Met Phe His Ser Val Cys Leu Gly Leu
   145                 150                 155                 160
   Gly Cys Asp Pro Ala Gln Ala Val His Val Gly Asp Lys Leu Asp Thr
                   165                 170                 175
   Asp Ala Ile Gly Ala Arg Asp Ala Gly Leu Gly Ala Val Trp Leu Asp
               180                 185                 190
   Arg Asp Gly Ile Ala Glu Arg Ala Pro Ala Gly Val His Thr Ile Ser
           195                 200                 205
   Gly Leu Asp Glu Leu Pro Glu Leu Leu Val Ser Glu Phe Ala Lys Leu
       210                 215                 220
   Gly Val Pro Ala Gln Arg Ala Thr Glu Thr Pro Ala Phe Thr Val Arg
   225                 230                 235                 240
   Asn Ser Val Leu
<210> 8
   <211> 735
   <212> DNA
   <213> Amycolatopsis japonicum
<220>
   <223> orf 1642
<400> 8
   gtgtgcctcg acatcgatga cacgttgatc gactgcacgg cggcgatccg cctcagcctg       60
   agcgccctca ctggccagaa cgacctttgg ccgctgtggg atctcatcac cgaagagcat      120
   gtcgcgctgg tggtcgcggg cgaaatcgat tacgcgacca tgcattacaa gcgtacggaa      180
   tgtttcctcg ccgagatcgg cattttggcc gacgaacagc aggtcagcgg cttcgaaaga      240
   cgtcgtcgtg aaattctcac ccgttcgtgg caattgttcg aagatgtcct cccatgcctt      300
   gaatggctgc gggccgccgg tgttctcgtc gccgcggtca cgaacgcctc gggcgcgcat      360
   cagcgcaaga agatcgccga cctcggcctc gcccggttct tcgatcacgt ggccatcgcc      420
   ggtgaactcg gagtagccaa acccgacccg gcgatgttcc actcggtctg cctcggcctc      480
   ggctgcgacc cggcgcaggc cgtccacgtc ggcgacaaac tcgacaccga cgccatcggc      540
   gcgcgcgacg ccggcctggg ggccgtctgg ctcgaccggg acggcatcgc cgagcgcgcc      600
   ccggcgggcg tgcacaccat ctcgggcctc gacgagctgc ctgagctgct cgtttccgag      660
   ttcgcgaagc tcggcgtgcc cgctcagcgc gctactgaga cccccgcttt cacggtgcgg      720
   aacagcgtgc tctag                                                       735
<210> 9
   <211> 70
   <212> PRT
   <213> Amycolatopsis japonicum
<220>
   <223> ORF 1643
<400> 9
   Met Val Glu Gly Arg Gln Thr Thr Pro Ala Thr Phe Asp His Pro Leu
   1               5                   10                  15
   Val Thr Ser Gly Ser Arg Leu Asn Asp Gly Gly Val Val Val Glu His
               20                  25                  30
   Gly Phe Ala Gly Ala Gly Ala Gly Leu Thr Leu Ser Val Ala Ala Ile
           35                  40                  45
   Ala Thr Ile Pro Ala Ala Phe Asp Ala His Val Pro Val Thr Pro Val
       50                  55                  60
   Leu Lys Ala Glu Thr Ala
   65                  70
<210> 10
   <211> 213
   <212> DNA
   <213> Amycolatopsis japonicum
<220>
   <223> orf 1643
<400> 10
   gtggttgaag gccgccagac gacaccagcc accttcgacc acccgctggt gacttccgga     60
   agccggctga acgacggcgg cgtggtggtc gaacacggtt tcgccggggc cggggccggc    120
   ctcacgctct ccgtcgccgc gatcgccacc atccccgccg ccttcgacgc acacgtcccg    180
   gtcactccgg tactcaaagc ggagaccgct tag                                  213
<210> 11
   <211> 135
   <212> PRT
   <213> Amycolatopsis japonicum
<220>
   <223> ORF 1644
<400> 11
   Met Arg Gln Ala Gly Phe Asp Gly Glu Leu Pro Asp Arg Ile Arg Ala
   1               5                   10                  15
   Phe Asp Glu Ile Val Lys Thr His Glu Trp Ser Glu Ser Ala Gly Ser
               20                  25                  30
   Ile Thr Gln Pro Ile Asp Tyr Tyr Arg Gln Phe Arg Gly Arg Pro Glu
           35                  40                  45
   Gly Pro Asn Arg Val Tyr Asp Ala Ser Asp Leu Pro Gly Val Gly Tyr
       50                  55                  60
   Thr Arg Gln Val Ala Asp Thr Thr Arg Met Gly Leu Gly Glu Ser Trp
   65                  70                  75                  80
   Thr Glu Ala Gly Gln Pro Val Arg Glu His Pro Glu Pro Ser Ser Asp
                   85                  90                  95
   Leu Phe Thr His Thr Ser Gly Pro Asn Gln His Ala Leu Ile Thr Glu
               100                 105                 110
   Gln Leu Thr Arg His His Tyr Val Met Thr Leu Thr Met Ser Thr Arg
           115                 120                 125
   Gln Thr Tyr Pro Thr Cys Arg
       130                 135
<210> 12
   <211> 408
   <212> DNA
   <213> Amycolatopsis japonicum
<220>
   <223> orf 1644
<400> 12
   gtgcggcaag caggcttcga cggcgaactg cctgaccgga tccgagcttt cgacgagatc      60
   gtcaagacac acgagtggtc tgaaagtgca ggctcgatca cacaacccat cgactactac     120
   cgccagttcc ggggtcgccc ggaaggaccg aaccgggtct acgacgcgag cgacctcccc     180
   ggcgtcggct acacccggca ggtggccgac accacccgca tgggactcgg cgagtcctgg     240
   accgaagcgg gacagccggt acgggagcac cccgagccgt cgtcagacct tttcacccat     300
   accagcggac cgaaccagca tgccttgatc accgagcagc tgacccgaca ccattacgtg     360
   atgaccttga ccatgagcac caggcagacc tatccgacgt gtcggtga                  408
<210> 13
   <211> 325
   <212> PRT
   <213> Amycolatopsis japonicum
<220>
   <223> ORF 1645
<400> 13
   Met Thr Met Arg Ala Ala Tyr Leu Ala Glu Pro Gly Val Val Ala Val
   1               5                   10                  15
   Gly Arg Phe Pro Ile Pro Arg Pro Glu Glu Gly Gln Val Leu Val Glu
               20                  25                  30
   Met Arg His Ala Ser Ile Cys Gly Ser Asp Val His Ile Val Phe Asp
           35                  40                  45
   Gly Phe His Asp Gln Glu Arg Leu Gly Ser Pro Gly Tyr Pro Gly His
       50                  55                  60
   Glu Gly Ile Gly Val Val Ala Glu Ser Arg Ser Arg Ala Phe Ala Ala
   65                  70                  75                  80
   Gly Asp Pro Val Leu Thr Val Pro Pro Val Glu His Thr Gly Cys Phe
                   85                  90                  95
   Ala Glu Tyr Gln Val Val Asp Glu Gly Ser Leu Val Pro Val Pro Arg
               100                 105                 110
   Glu Gly Asp Leu Ser Arg Leu Leu Leu Ala Gln Gln Leu Gly Thr Thr
           115                 120                 125
   Val Phe Gly Met Ala Lys Phe Val Asp Gly Pro Pro Pro Arg Thr Ala
       130                 135                 140
   Ala Val Ile Gly Ala Gly Ser Ala Gly Leu Phe Phe Leu Gln Leu Leu
   145                 150                 155                 160
   Arg Arg Met Gly Cys Glu Ser Val Leu Val Ser Asp Leu Asp Pro Gly
                   165                 170                 175
   Arg Leu Val Val Ala Asp Arg Leu Gly Ala Gln Val Val Asp Ala Arg
               180                 185                 190
   Arg Ala Ala Leu Ala Asp Thr Ala Arg Glu Met Thr Gly Gly Ile Gly
           195                 200                 205
   Val Asp Leu Val Val Glu Ala Ala Gly Pro Asp Ala Cys Arg Val Glu
       210                 215                 220
   Ala Val Glu Ala Val Arg Glu Arg Gly Thr Ile Gly Phe Phe Gly Phe
   225                 230                 235                 240
   Pro Glu Arg Lys Gly Thr Ala Pro Phe Pro Val Glu Arg Ala Phe Arg
                   245                 250                 255
   Lys Ser Val Arg Met Glu Trp Val Asn Gly Thr Gln Lys Glu Pro Gly
                   260                 265                 270
   Leu Val Ser Phe Arg Arg Ala Val Asp Leu Ile Arg Ser Gly Glu Ile
               275                 280                 285
   Val Val Asp His Cys Leu Glu His Ala Phe Asp Leu Gly Asp Ile Ala
           290                 295                 300
   Glu Ala Phe Ala Ser Ala Arg Arg His Gly Asp Gly His Pro Lys Val
   305                 310                 315                 320
   Gly Ile Ile Leu Arg
                   325
<210> 14
   <211> 978
   <212> DNA
   <213> Amycolatopsis japonicum
<220>
   <223> orf 1645
<400> 14
   atgaccatgc gggcggcata tctggcagag cccggagtgg tcgcggtcgg gcggttcccg      60
   ataccgcgcc ccgaggaagg gcaagtgctc gtcgaaatgc ggcacgcctc gatctgcggc     120
   tcggacgtgc acatcgtgtt cgacggcttc cacgatcaag agcggttggg cagccctggc     180
   tatccagggc acgagggcat cggagtggtg gcggaaagcc gcagccgagc cttcgccgcc     240
   ggcgatcctg tgctcaccgt gccgcctgtg gaacacaccg gttgcttcgc cgaataccag     300
   gtcgtcgacg aggggagcct ggtaccggtt ccgcgcgagg gcgacctgtc ccgcctgctg     360
   ctcgcacaac aactcgggac cacggtgttc gggatggcga aattcgtcga cggaccccca     420
   ccccggaccg cggccgtgat cggtgcgggc tccgcgggat tgttcttctt gcagttgctg     480
   cgccgcatgg gatgtgagtc cgtgctcgtg tcggacctcg atcccggcag gctcgtcgtc     540
   gcggaccggc tgggcgcaca agtggtcgac gcacgacgag cggctctcgc ggacacggca     600
   cgcgagatga ccggcggtat cggcgtggac ctcgtcgtcg aagccgccgg gcccgacgcc     660
   tgcagggtgg aggcggtcga agccgtccgg gagcgcggca cgatcgggtt cttcggcttc     720
   cccgaacgca agggcacggc cccgttcccg gtggagcggg cattccgcaa gtcggtacgg     780
   atggaatggg tcaacggaac gcagaaggaa ccgggcctgg tgtcgttccg gcgagccgtg     840
   gatctgatcc gaagcggcga gatagtggtc gaccattgcc tcgagcacgc cttcgacctc     900
   ggagacatcg ccgaggcgtt cgcgtccgcc cgccgccacg gtgatggcca cccgaaggtg     960
   ggaatcattc tgcgctga                                                   978
<210> 15
   <211> 448
   <212> PRT
   <213> Amycolatopsis japonicum
<220>
   <223> ORF 1646
<400> 15
   Met Asn Trp Ser Gln Leu Leu Val Pro Val Thr Ser Gly Val Ser Val
   1               5                   10                  15
   Ala Leu Ile Val Ala Thr Val Arg Phe Leu Trp Arg Arg Thr Arg Leu
               20                  25                  30
   Pro Thr Gly Leu Ser Gln Arg Met Thr Arg Arg Asn Tyr Leu Ala Glu
           35                  40                  45
   Met Leu Val Ile Ser Lys Asn Asp Asn Val Asp Arg Leu Asp Ala Leu
       50                  55                  60
   Val Pro Asn Leu Leu Pro Ala Gln Gly Ser Pro Val Leu Val Glu Ile
   65                  70                  75                  80
   Gln Glu Ser Trp Thr Gln Ile Asn Gly Arg Arg Gly Val Arg Val Ile
                   85                  90                  95
   Thr Arg Thr Glu Pro Ala Ser Leu Thr Ala Gly Ala Glu Leu Leu Ala
               100                 105                 110
   Ala Gly Ile Glu Val Arg Val Ser Arg Ala Leu Thr Ala Asp His Leu
           115                 120                 125
   Ser Tyr His Leu Phe Ser Gly Ser Glu His His Thr Val Leu Asn Arg
       130                 135                 140
   Arg Asn Gly Gly Lys Asp Arg Pro Glu Arg Leu Asp Glu Ile Ser Ala
   145                 150                 155                 160
   Ala Lys Val Phe Arg Ser His Phe Asp Glu Thr Trp Ser Glu Ser Glu
                   165                 170                 175
   Pro Ile Glu Ser Phe Leu Ala Gly Gln Leu Leu Ala Gly Pro Asp Ser
               180                 185                 190
   Gly Leu Ala Asp Arg Ile Arg Glu Gln Arg Ala Ala Tyr Gln Leu Pro
           195                 200                 205
   Ala Lys Ala Glu Asp Ala Ile Leu Arg His Leu Ala Phe Arg His Arg
       210                 215                 220
   Ala Pro Val Val Phe Val Thr Gly Leu Pro Gly Ala Gly Lys Ser Leu
   225                 230                 235                 240
   Val Arg Arg Ala Leu Ala Glu Glu Leu Arg Arg Trp Arg Met Gln Val
                   245                 250                 255
   Asp Glu Leu Asn Asp Tyr Val Tyr Ala Phe Glu Glu Phe Leu His Ala
               260                 265                 270
   Leu Met Leu Leu Gly Asp Gly Arg Gly Ala Gly Phe Ser Ala Gln Gln
           275                 280                 285
   Gly Gly Ala Phe Gln Val Glu Arg Glu Asp Asp Leu Arg Pro Ala Leu
       290                 295                 300
   His Thr Leu Gly His Arg Val Trp Glu Asn Arg Arg Ser Asn Pro Ile
   305                 310                 315                 320
   Thr Ile Val Glu Phe Ala Arg Ala Asp Thr Ile Gly Ala Leu Gln Val
                   325                 330                 335
   Phe Gly Asp Glu Val Leu Ala Ala Ser Gln Ile Ile His Val Arg Ala
               340                 345                 350
   Ser Asp Thr Glu Arg Ser Ala Arg Leu Ala Arg Arg Gly Glu Pro Pro
           355                 360                 365
   Lys Ile Ser Val Ala Gly Gln Ser Ile Ser Leu Glu Val Ser Asp Glu
       370                 375                 380
   His Arg Leu Pro Ser Asn Ala Ala Asp Thr Leu Tyr Ser Arg Asp Asp
   385                 390                 395                 400
   Phe Ala Leu Leu Lys Ala Gln Lys Asn Ile Ala Asn Arg Leu Phe Leu
                   405                 410                 415
   Val Glu Asn Asp Ala Glu Glu Lys Ala His Val Asp Glu Gln Val Thr
               420                 425                 430
   Ala Phe Val Glu Ala Val Val Arg Pro Tyr Arg Val Leu Ala Arg Thr
           435                 440                 445
<210> 16
   <211> 1347
   <212> DNA
   <213> Amycolatopsis japonicum
<220>
   <223> orf 1646
<400> 16
   atgaactgga gtcagctcct cgttcccgtg accagcggtg tgtccgtcgc cctcatcgtg      60
   gcgaccgtca ggttcctgtg gcggcgcacc cgtctgccga ccggcctctc ccagcggatg     120
   acccggcgga actacctggc ggagatgctc gtcatctcca agaacgacaa cgtcgaccgg     180
   ctcgacgcac tggtcccgaa tctgctgccg gcacaaggct cccccgtgct cgtcgagatc     240
   caggagtcgt ggacgcagat caacggccgg cgcggcgtcc gggtgatcac ccggaccgag     300
   cccgcgtccc tgaccgccgg agccgagttg ctcgcagcgg ggatcgaggt tcgcgtgtcc     360
   cgcgcactga ccgcggatca cctgtcgtac cacctgttct ccggttcgga acaccacacg     420
   gtgctcaacc gccgcaacgg cgggaaggac cggcccgaac gactggacga gatctccgca     480
   gccaaggtgt tccgcagcca cttcgacgag acgtggagcg agtccgaacc catcgaatcg     540
   ttcctggccg ggcaactcct ggccgggccc gactccggcc tcgcggaccg gatccgggag     600
   cagcgcgcgg cgtaccagct gcccgccaag gcggaggacg cgatcctgcg gcatctggcg     660
   ttccgccacc gggcgccggt cgtcttcgtc accggcctgc cgggtgccgg caagtcactg     720
   gttcgacgag ctctcgccga agagcttcgg cggtggcgga tgcaggtcga cgagctgaac     780
   gactacgtct acgcgttcga ggaattcctc cacgccttga tgctcctcgg cgacggtcgc     840
   ggtgccggct tcagcgccca gcagggcggc gcgttccagg tggagcgcga ggacgatctc     900
   cggcccgccc tgcacacgct cggccaccgc gtctgggaaa accgccggag caacccgatc     960
   acgatcgtcg agttcgccag ggccgacacg atcggagcgc tccaggtgtt cggcgacgag    1020
   gtactggcgg cgtcgcagat catccacgtc cgcgcctccg acaccgagcg ctccgcccgg    1080
   ctggccagac ggggcgaacc accgaagatc tcggtggcgg gtcagtcgat cagtctcgaa    1140
   gtgtccgacg agcatcggct gcccagcaac gccgcggaca ccctgtacag cagggatgac    1200
   ttcgcactcc tgaaggcaca gaagaacatc gccaaccgcc ttttcctcgt cgagaacgac    1260
   gccgaggaaa aggcgcacgt cgacgaacag gtcacggcgt tcgtcgaggc ggtcgtgcgc    1320
   ccctaccgag tactcgcgcg tacctag                                        1347
<210> 17
   <211> 110
   <212> PRT
   <213> Amycolatopsis japonicum
<220>
   <223> ORF 1647
<400> 17
   Met Thr Glu Gln Ala Phe Phe Val Leu Thr Ala Leu Val Asp Thr Pro
   1               5                   10                  15
   Arg His Gly Tyr Gly Ile Val Gly Glu Val Ser Gly Leu Ser Asp Gly
               20                  25                  30
   Arg Val Lys Leu Lys Val Gly Thr Leu Tyr Gly Val Leu Asp Arg Leu
           35                  40                  45
   Val Ala Asp Gly Leu Val Glu Pro Asp Arg Glu Glu Val Ala Asn Gly
       50                  55                  60
   Arg Leu Arg Arg Tyr Tyr Arg Leu Thr Glu Asp Gly Arg Thr Ala Leu
   65                  70                  75                  80
   Ala Ala Glu Val Gln Arg Gln Ala Ser Asn Ala Arg Ala Ala Ser Glu
                   85                  90                  95
   Arg Leu Arg Ala Trp Arg Pro Ala Asp Thr Gly Gly Leu Ala
               100                 105                 110
<210> 18
   <211> 333
   <212> DNA
   <213> Amycolatopsis japonicum
<220>
   <223> orf 1647
<400> 18
   atgacggagc aggcgttctt cgtgctcacc gcgctggtgg acacgccgcg gcatgggtac      60
   gggatcgtcg gcgaggtgag cggtctctcg gacggccggg tgaagctgaa ggtcggcacg     120
   ctgtacggcg tgctggatcg gctggtcgcg gacggtttgg tcgagccgga tcgggaggag     180
   gtcgccaacg ggcggctccg ccggtactac cggctgaccg aggacggccg cacggcgctc     240
   gcggccgagg tccaacggca ggcctcgaac gcccgcgccg cctcggaacg gctgcgcgcg     300
   tggcggccgg cggacaccgg gggcctggcg tga                                  333
<210> 19
   <211> 331
   <212> PRT
   <213> Amycolatopsis japonicum
<220>
   <223> ORF 1648
<400> 19
   Met Asn Arg Leu Glu Glu Arg Tyr Arg Arg Val Leu Arg Leu Leu Pro
   1               5                   10                  15
   Ala Ser Tyr Arg Gln Ala Trp Glu Glu Asp Met Val Ala Thr Phe Leu
               20                  25                  30
   His Ser Glu Met Pro Glu Asp Ala Glu Asp Ala Glu Phe Ala Val Asp
           35                  40                  45
   His Gly Arg Pro Ser Trp Pro Glu Val Ala Ser Val Val Ala Leu Ala
       50                  55                  60
   Val Arg Leu Arg Leu Gly Gly Asp Gly Ala Pro Leu Arg Tyr Leu Val
   65                  70                  75                  80
   Trp Gly Glu Thr Val Arg Arg Leu Ala Leu Val Gly Leu Leu Val Gln
                   85                  90                  95
   Ala Val Ala Ala Leu Val Gly Ile Gly Ala Trp Leu Trp Ser Pro Ser
               100                 105                 110
   Val Thr Asn Pro Ile Pro Ala Asp Val Gln Thr Leu Leu Gly Leu Val
           115                 120                 125
   Trp Val Ala Ala Tyr Leu Cys Leu Val Thr Gly Arg Arg Arg Ala Ala
       130                 135                 140
   Arg Trp Leu Ala Val Thr Ala Leu Ala Gln Ala Ser Val Phe Phe Gly
   145                 150                 155                 160
   Val Asp Leu Val Ala Val Glu Gly Ala Tyr Gly Leu Ser Gly Thr Val
                   165                 170                 175
   Gly Leu Leu Leu Glu Ala Leu Pro Val Val Ala Leu Val Ala Phe His
               180                 185                 190
   Arg Gln Ala Pro Pro Val Lys Ala Arg Pro Trp Leu Val Ala Leu Leu
           195                 200                 205
   Ala Gly Val Gly Leu Leu Thr Val Ala Met Ser Val Thr Tyr Val Ser
       210                 215                 220
   Val Pro Ala Leu Val Asp Trp Pro Gly Val Asp Cys Ala Ala Val Phe
   225                 230                 235                 240
   Val Ala Ala Val Val Tyr Leu Ser Val Pro Arg Leu Gly Trp Arg Ser
                   245                 250                 255
   Ala Ala Val Ser Trp Ser Thr Thr Leu Ala Ser Leu Ala Leu Val Ala
               260                 265                 270
   Leu Val Gln Arg Val Val Thr Leu Leu Asp Tyr Leu Arg Leu Ala Ala
           275                 280                 285
   Arg Thr Ala Glu Leu Asp Ile Met Ile Ala Ile Ala Ala Gly Gln Thr
       290                 295                 300
   Val Ala Val Leu Ala Ile Ser Val Pro Leu Ala Met Leu Ala Val Lys
   305                 310                 315                 320
   Ala Val Arg Ala Leu Pro Pro Val Ala Ala Arg
                   325                 330
<210> 20
   <211> 996
   <212> DNA
   <213> Amycolatopsis japonicum
<220>
   <223> orf 1648
<400> 20
   gtgaaccgcc tggaggagcg gtaccggcgg gtgctgcggc tgctccccgc ctcctatcgt      60
   caggcgtggg aggaggacat ggtcgccacc ttcctgcaca gcgagatgcc ggaggacgcc     120
   gaggacgcgg agttcgccgt cgaccacggc aggccgagct ggccggaggt ggcgagcgtc     180
   gtggcgctgg cggtgcggct gcggctggga ggcgacggcg cgcccctccg ctacctcgtg     240
   tggggtgaga cggtccggcg gctggcgctg gtggggttgc tcgtgcaggc ggtggccgcg     300
   ctggtcggta tcggggcgtg gctgtggtct ccctcggtga cgaatccgat cccggccgac     360
   gtgcagaccc tgctcggctt ggtctgggtg gcggcttatc tgtgcctggt gaccggcagg     420
   cggcgcgcgg cgcggtggct cgcggtgacg gcgctggctc aagcctcggt cttcttcggc     480
   gtggatctcg tggccgtcga gggcgcgtac gggctgtccg ggaccgtcgg actcctgctg     540
   gaggcgctgc cggtggtggc actggtcgcg ttccatcggc aggccccacc ggtgaaagcg     600
   cggccgtggc tcgtcgcgct gcttgccggt gtcggcctcc tgacggtcgc gatgtcggtc     660
   acctatgtca gcgttccggc gctcgtggac tggccgggcg tggactgtgc ggcggttttc     720
   gtcgccgcgg tcgtctactt gagcgtgcct cgcctgggct ggcggagtgc cgcggtgtcg     780
   tggtcgacga cgctcgcttc gctcgcgctg gtcgccctcg tgcagcgggt cgtgacgctg     840
   ctggactacc tccggctcgc ggcccgcacc gcggaactgg acatcatgat cgcgatcgcc     900
   gccgggcaga ccgtggcggt tctggcgata tcggtgccgc tcgcgatgct cgccgtcaag     960
   gccgtgcgcg cgctgccgcc ggtggctgcc cggtga                               996
<210> 21
   <211> 339
   <212> PRT
   <213> Amycolatopsis japonicum
<220>
   <223> ORF 1649
<400> 21
   Met Val Leu Ala Leu Pro Phe Val Asp Gln Val Trp Gln Ile Tyr Val
   1               5                   10                  15
   Leu Ile Val Ile Leu Gln Ser Ala Ser Ala Ser Phe Thr Pro Ala Phe
               20                  25                  30
   Gln Ala Val Leu Pro Asp Leu Leu Pro Asp Glu Arg Asp Tyr Thr Lys
           35                  40                  45
   Ala Leu Ser Leu Ser Gln Leu Ala Ser Thr Met Glu Ser Leu Leu Ser
       50                  55                  60
   Pro Leu Leu Ala Ala Ala Val Leu Ser Leu Val Ser Phe His Trp Leu
   65                  70                  75                  80
   Phe Thr Gly Thr Ser Leu Gly Phe Ala Ala Ser Ala Ala Leu Val Leu
                   85                  90                  95
   Ser Thr Arg Ile Pro Asp Ala Ala Arg Gly Asp Arg Gly Gly Ala Trp
               100                 105                 110
   Asp Arg Thr Met Ala Gly Ile Lys Met Tyr Leu Ala Thr Pro Arg Leu
           115                 120                 125
   Arg Gly Thr Leu Gly Leu Asp Leu Val Val Ala Ala Ala Gly Ser Val
       130                 135                 140
   Val Met Val Asn Thr Val Asn Tyr Val Arg Asp Thr Leu Gly Gly Thr
   145                 150                 155                 160
   Gln Ser Asp Val Ala Leu Leu Leu Ala Ala Asn Gly Ala Gly Thr Ile
                   165                 170                 175
   Leu Ala Ala Leu Leu Leu Pro Arg Val Leu Asp Gly Ile Pro Asp Arg
               180                 185                 190
   Thr Val Met Leu Thr Gly Gly Gly Thr Leu Leu Ala Gly Leu Thr Gly
           195                 200                 205
   Ala Ile Ala Leu Ser Thr Val Glu His Gly Ala Ala Ala Pro Val Val
       210                 215                 220
   Trp Ala Val Ile Gly Leu Gly Thr Gly Leu Val Leu Thr Pro Val Gly
   225                 230                 235                 240
   Arg Val Leu Arg Arg Ser Ser Arg Pro Ala Asp Arg Pro Ala Ile Phe
                   245                 250                 255
   Ala Ala Arg Phe Ser Leu Ser His Ala Cys Trp Leu Leu Ala Tyr Pro
               260                 265                 270
   Ile Ala Gly Arg Leu Ala Thr Asp Ala Gly Phe Thr Val Thr Trp Leu
           275                 280                 285
   Val Leu Gly Ala Leu Gly Val Ile Gly Leu Val Thr Ala Val Arg Ala
       290                 295                 300
   Trp Pro Arg Asn Asp Asp Glu Asp Arg Pro Arg Pro Lys Ser Arg Pro
   305                 310                 315                 320
   Gly Gln Pro Phe Glu Asp Ile Thr Gly Gln Pro Pro Ala Ala Ala Arg
                   325                 330                 335
   Ala Arg Pro
<210> 22
   <211> 1020
   <212> DNA
   <213> Amycolatopsis japonicum
<220>
   <223> orf 1649
<400> 22
   gtggtgctcg ccctgccctt cgtcgaccag gtctggcaga tctacgtcct gatcgtgatc      60
   ctccagtccg cgtcggcctc gttcaccccg gcgttccagg ccgtgctccc cgatctcctt     120
   cccgacgaac gcgactacac gaaggcactg tcgctgtccc agctggcttc gacgatggag     180
   agcctcctca gtccgctcct ggccgccgcc gtgctcagtc tcgtgtcctt ccactggctg     240
   ttcaccggca cttcgctcgg tttcgcggcc tccgccgcac tggtgctgag cacccggatc     300
   ccggacgcgg cgcgcggcga ccgcggcggg gcctgggatc gcaccatggc cgggatcaag     360
   atgtacctcg cgaccccgcg cctgcggggc accctcggcc tcgacctggt ggtcgcggcg     420
   gccggctccg tggtcatggt caataccgtg aactacgtcc gagacaccct cggcggaaca     480
   cagtccgacg tcgccctgct cctggcggcc aacggcgccg gcaccatcct ggccgcgctc     540
   ctgctccccc gtgtactcga cgggatcccc gaccgcaccg tcatgctcac cggcggcgga     600
   accctgctcg ccggactgac cggcgccatc gcactgtcca cagtggagca cggggccgcg     660
   gcgccggtcg tatgggccgt catcgggctc ggcaccgggc tcgtcctcac tcccgtcggc     720
   cgggtactgc gcagatccag ccgtcccgcc gaccggcccg cgatcttcgc cgcccggttc     780
   tccctttccc acgcctgctg gctactcgcc tacccgatcg ccggacggct cgccaccgac     840
   gccgggttca ccgtcacctg gctcgtcctc ggcgcactcg gcgtcatcgg cctcgtcacg     900
   gccgtccgcg cgtggccacg aaacgacgac gaggaccgac cgcggccgaa atcccggcca     960
   ggtcaaccct tcgaggacat caccgggcag ccaccggcgg cagcgcgcgc acggccttga    1020
<210> 23
   <211> 103
   <212> PRT
   <213> Amycolatopsis japonicum
<220>
   <223> ORF 1650
<400> 23
   Met Ser Leu Phe Gly His Arg Asp Tyr Arg His Leu Phe Gly Ala Gln
   1               5                   10                  15
   Leu Val Ala Leu Phe Gly Thr Gly Leu Thr Thr Val Ala Leu Gly Leu
               20                  25                  30
   Leu Ala Tyr Asp Leu Ala Gly Ser Asp Ala Gly Ala Val Leu Gly Thr
           35                  40                  45
   Ala Leu Ala Ile Lys Met Ile Thr Tyr Val Thr Val Ala Pro Leu Ala
       50                  55                  60
   Val Ala Tyr Ala Ala Thr Gly Cys Arg Ala Val Ser Ser Ser Ser Arg
   65                  70                  75                  80
   Trp Thr Arg Ser Ala Arg Trp Trp Cys Ser Pro Cys Pro Ser Ser Thr
                   85                  90                  95
   Arg Ser Gly Arg Ser Thr Ser
               100
<210> 24
   <211> 312
   <212> DNA
   <213> Amycolatopsis japonicum
<220>
   <223> orf 1650
<400> 24
   atgtcgctgt tcggtcatcg tgactatcgg catctgttcg gggcgcagct ggtcgcgctg     60
   ttcggcactg gattgacgac cgtagcgctc gggctgctgg cctacgactt ggcggggtcc    120
   gacgcgggcg ccgtcctggg caccgcgctg gccatcaaga tgatcaccta cgtcacggtg    180
   gcgccgctcg ccgtcgcgta cgccgcgacc gggtgccgcg ccgtttcttc ctcgtcacgc    240
   tggacgcgat ccgcgcgctg gtggtgctcg ccctgccctt cgtcgaccag gtctggcaga    300
   tctacgtcct ga                                                         312
<210> 25
   <211> 116
   <212> PRT
   <213> Amycolatopsis japonicum
<220>
   <223> ORF 1651
<400> 25
   Met Tyr Ala His Asp Glu Ser Gly Gly Gln Trp Ser Arg Leu Pro Pro
   1               5                   10                  15
   Asp Asp Gln Ile Glu Ala Ala Ser Thr Ala Leu Arg Met Leu Ala Asp
               20                  25                  30
   Pro Thr Arg Met Arg Met Leu Trp Leu Ile Ser Gly Glu Glu Tyr Asp
           35                  40                  45
   Val Ala Ser Leu Ala Ala Ala Val Gly Ile Ala Arg Pro Ala Val Ser
       50                  55                  60
   Gln His Leu Ala Lys Leu Lys Leu Ala Gly Leu Val Thr Gln Arg Arg
   65                  70                  75                  80
   Asp Gly Arg Arg Ile Leu Tyr Arg Ala Arg Gly Gly His Val Arg Arg
                   85                  90                  95
   Leu Leu Ala Glu Val Met Asn Ala Ala Asp His His Leu His Gly Ile
               100                 105                 110
   Pro Asp His Asp
           115
<210> 26
   <211> 351
   <212> DNA
   <213> Amycolatopsis japonicum
<220>
   <223> orf 1651
<400> 26
   atgtacgcac atgacgaatc aggcggccaa tggtcccggc tgccgccgga cgaccagatc 60
   gaagccgcgt ccacggcgtt gcggatgctc gcggatccca cccggatgcg gatgctgtgg 120
   ctgatcagcg gcgaggagta cgacgtcgcg tcactggcgg ccgcggtggg catcgcgcgt 180
   ccggcggtgt cccagcatct ggccaagctc aagctggccg gattggtcac ccagcgcagg 240
   gacggccgcc ggatccttta ccgggcccgc ggcgggcacg tccggaggct gctggcggag 300
   gtcatgaacg cggccgatca ccaccttcac gggatccccg accacgactg a 351
<210> 27
   <211> 375
   <212> PRT
   <213> Amycolatopsis japonicum
<220>
   <223> ORF 1652
<400> 27
   Met Asp Gly Pro Asp Ala Thr Arg Ser Leu Leu Pro Ala Gly Phe Gln
   1               5                   10                  15
   Arg Tyr Gly Arg Thr Lys Ala Thr Ala Ala Ala Ala Gly Asp Asp Leu
               20                  25                  30
   Leu Asp Ile Ile Gly Leu Ser Glu Thr Asp Val Glu Val Tyr Asp Ala
           35                  40                  45
   Phe Ala Gly Thr Gly Ser Leu Thr Val Ala Glu Ile Arg Arg Arg Thr
       50                  55                  60
   Gly Ile Pro Gln Gln Arg Leu Leu Arg Val Leu Gly Ala Leu Thr Glu
   65                  70                  75                  80
   Lys Gly Leu Leu Val Arg Leu Pro Gly Arg Ala Glu Val Tyr Ser Ala
                   85                  90                  95
   Val Arg Pro Glu Ile Ala Leu Glu Ala Met Leu Arg Leu Lys Glu Gln
               100                 105                 110
   Glu Leu Ala Ser Ala Arg Leu Val Ala Asp Arg Leu Arg Glu Arg Tyr
           115                 120                 125
   Arg Ala Thr Ser Gly Glu Arg Ala Val Asp Leu Ile Glu Val Ile His
       130                 135                 140
   Gly Asp Ala Leu Ile Ala Glu Arg Ala Asp Gln Leu Leu Arg Ser Ala
   145                 150                 155                 160
   Glu Arg Glu Val Ala Phe Val Asp Lys Pro Pro Tyr Ala Arg Thr Pro
                   165                 170                 175
   Ser Val Leu His Pro Ala Glu Arg Asp Leu Leu Gly Arg Gly Val Arg
               180                 185                 190
   Phe Arg Gly Val Tyr Glu Arg Ser Ala Leu Glu Met His Asp Leu Ser
           195                 200                 205
   Ser Asp Leu Glu Ala Gly Leu Ala Leu Gly Glu Glu Ala Arg Val Val
       210                 215                 220
   Thr Ser Ala Pro Leu Lys Met Ile Val Val Asp Gln Arg Val Gly Leu
   225                 230                 235                 240
   Val Pro Leu Arg Ser Asp Arg Pro Glu Val Gly Ala Ala Leu Val Ile
                   245                 250                 255
   His Pro Cys Ala Leu Leu Asp Ala Leu Gly Ala Val Phe Ala Phe Leu
               260                 265                 270
   Trp Gln Ser Gly Leu Pro Leu Arg Leu Pro Gly Ser Ala Glu Leu Ala
           275                 280                 285
   Asp Val Ala Pro Ser Asp Asp Ala Arg Leu Leu Ala Leu Leu Thr Thr
       290                 295                 300
   Gly Leu Pro Asp Arg Ser Ile Ala Lys Gln Leu Gly Met Ser Tyr Arg
   305                 310                 315                 320
   Thr Phe Gln Arg Arg Leu Arg Asp Leu Met Thr Ala Leu Gly Ala Thr
                   325                 330                 335
   Thr Arg Phe Gln Ala Gly Leu Gln Val Ala Thr Arg Gly Trp Val Thr
               340                 345                 350
   Leu Pro Ser Thr Gln Pro Pro Ile Glu Asp Asp Val Ser Glu Arg Ala
           355                 360                 365
   Gly Ser Pro Ser Pro Arg Pro
       370                 375
<210> 28
   <211> 1128
   <212> DNA
   <213> Amycolatopsis japonicum
<220>
   <223> orf 1652
<400> 28
   gtggacggtc cggacgcgac gcggtccctt ttgcccgccg gattccagcg gtatggtcgg      60
   acgaaggcga ccgcggctgc ggcgggagac gatttgctgg acatcatcgg tctgagtgaa     120
   accgacgtcg aggtctacga tgccttcgcc ggcaccggtt cgctgaccgt cgccgaaatc     180
   cggcggcgaa ccgggattcc gcaacaacgg ctgctgcgcg tactgggcgc gctgacggaa     240
   aaaggtttgc tcgtcaggct tcccggccgg gcggaggtgt attcggcggt gcggccggag     300
   atcgcgctgg aggcgatgct ccggctgaag gaacaggaac tcgcgagcgc gcggctggtc     360
   gccgaccggt tgcgcgagcg gtaccgggcg acgtccggcg aacgcgcggt cgatctcatc     420
   gaagtgatcc acggcgacgc gctgatcgcc gagcgggcgg atcagctgct gcgctcggcc     480
   gagcgggagg tcgccttcgt cgacaaaccg ccttacgcgc gaacgccgag tgtgctgcat     540
   cccgccgaac gtgatctcct cggccggggg gtgcgctttc gcggggttta cgagcgcagt     600
   gcgctggaaa tgcacgacct gtcatccgac ctggaggcag gcctcgcgct cggcgaggag     660
   gcccgcgtgg tcaccagcgc cccgctcaag atgatcgtgg tcgaccagcg cgtcggactg     720
   gtaccactgc ggtccgatcg ccccgaggtc ggcgcggcct tggtgatcca cccgtgcgca     780
   ctgctcgacg cgctcggcgc ggtcttcgct ttcctttggc agagcgggct tccgctgcgc     840
   ctgcccggtt cggccgaact ggcggacgtc gccccttccg acgacgcccg cctgctcgcc     900
   ttgctgacca cgggcctgcc ggaccggagc atcgccaaac aactcggcat gagctaccgg     960
   accttccagc gacggctccg cgacctcatg accgcgctcg gcgcgaccac ccggttccag    1020
   gccgggcttc aggtcgccac gcggggttgg gtcaccttgc cgtcgaccca acccccgatc    1080
   gaggacgacg tcagcgagcg cgcgggttcg ccttcgccac gtccgtga                 1128
<210> 29
   <211> 1347
   <212> PRT
   <213> Amycolatopsis japonicum
<220>
   <223> ORF 1653
<400> 29
   Met Phe Pro Arg Ser Pro His Leu Thr Arg His Pro Trp His Pro Val
   1               5                   10                  15
   Ala Ile Leu Ala Ala Ser Ala Leu Val Ala Leu Thr Pro Leu Pro Val
               20                  25                  30
   Ile Ala Thr Ala Ala Thr Ala Ala Val Ser Gly Pro Ala Pro Val Thr
           35                  40                  45
   Gly Arg Pro Asp Val His Arg Val Thr Leu Leu Thr Gly Asp Val Val
       50                  55                  60
   Thr Tyr Glu Arg Asp Gly Ala Gly His Gly Thr Ala Gln Val Glu Pro
   65                  70                  75                  80
   Ala Val Arg Pro Asp Arg Pro Ser Pro Ala Phe Gln Ile Thr Thr Thr
                   85                  90                  95
   Ala Glu Gly Leu Met Val Tyr Pro Ser Asp Ala Leu Pro Phe Val Gly
               100                 105                 110
   Lys Gly Leu Leu Ser Pro Asp Leu Phe Asn Val Thr Thr Leu Val Gly
           115                 120                 125
   Asp Arg Arg Asp Asp Ala Ala Ser Thr Thr Ile Pro Val Ile Val Arg
       130                 135                 140
   Tyr Gly Asp Arg Ser Gly Leu Ser Ala Arg Thr Leu Asp Ala Lys Ala
   145                 150                 155                 160
   Lys Ala Leu Pro Gln Thr Gln Arg Leu Thr Pro Leu Val Thr Ala Asn
                   165                 170                 175
   Gly Val Gly Val Gln Val Lys Lys Ala Gly Thr Gly Glu Phe Trp Arg
               180                 185                 190
   Ser Leu Val Ala Phe Asp Ala Ser Gly Lys Pro Val Leu Asn Gln Gly
           195                 200                 205
   Ile Ser Gln Val Arg Leu Asp Arg Lys Val Arg Val Thr Leu Asp Lys
       210                 215                 220
   Ser Thr Ala Arg Val Gly Ala Pro Ser Ala Trp Ser Gly Gly Trp Thr
   225                 230                 235                 240
   Gly Lys Gly Thr Thr Val Ala Val Val Asp Thr Gly Ile Asp Glu Asn
                   245                 250                 255
   His Pro Asp Leu Ala Gly Lys Thr Val Ala Ser Ala Asp Phe Ser Gly
               260                 265                 270
   Glu Gly Asp Val Val Asp Arg His Gly His Gly Thr His Val Ala Ser
           275                 280                 285
   Ile Val Ala Gly Thr Gly Ala Ala Ser Gly Gly Arg Tyr Lys Gly Val
       290                 295                 300
   Ala Pro Asp Ala Asn Leu Val Val Ala Lys Val Phe Asp Ala Ser Gly
   305                 310                 315                 320
   Glu Gly Asp Thr Ala Gln Val Met Ala Gly Ile Asp Trp Ala Val Ala
                   325                 330                 335
   Gln Gly Ala Lys Ile Val Asn Leu Ser Leu Gly Ala Gly Val Ser Asp
               340                 345                 350
   Gly Ala Asp Pro Met Ser Glu Gln Ile Asp Ser Leu Ser Ala Lys Ser
           355                 360                 365
   Gly Thr Leu Phe Val Val Ala Ala Gly Asn Ser Gly Pro Gly Asp Arg
       370                 375                 380
   Thr Val Thr Thr Pro Gly Ala Ala Thr Ser Ala Leu Thr Val Gly Ala
   385                 390                 395                 400
   Leu Asp Arg Glu Asp Lys Ile Ala Trp Phe Ser Ser Arg Gly Pro Arg
                   405                 410                 415
   Leu Arg Asp Ala Ser Val Lys Pro Glu Ile Thr Ala Pro Gly Val Gly
               420                 425                 430
   Ile Val Ala Ala Arg Ala Ala Asp Thr Ala Leu Gly Glu Pro Val Asp
           435                 440                 445
   Asp Ser Tyr Thr Ala Ala Ser Gly Thr Ser Met Ala Thr Pro His Val
       450                 455                 460
   Ala Gly Ala Ala Ala Ile Leu Leu Gln Gln Arg Pro Gly Leu Thr Gly
   465 470 475 480
   Gln Ala Leu Lys Asn Thr Leu Val Thr Thr Ala Lys Asp Val Gly Leu
   485 490 495
   His Trp Phe Glu Gln Gly Ala Gly Ile Leu Asp Val Ala Arg Ala Val
   500 505 510
   Ser Gln Lys Ala Thr Gly Thr Ala Val Ala Ser Phe Gly Arg Asn Glu
   515 520 525
   Arg Thr Thr Asn Ala Ser Ala Gln Val Val Arg Gln Leu Ser Tyr Thr
   530 535 540
   Asn Thr Gly Asp Gln Pro Leu Asn Leu Asn Leu Lys Leu Thr Val Arg
   545 550 555 560
   Pro Trp Asp Gly Gly Ala Ala Pro Gly Thr Gly Met Arg Leu Ala Lys
   565 570 575
   Thr Asp Leu Ser Ile Pro Pro Lys Ser Ser Thr Thr Val Asp Leu Ala
   580 585 590
   Val Asn Pro Asn Glu Gly Thr Ala Gly Val Tyr Gly Gly Ala Val Val
   595 600 605
   Ala Thr Thr Ala Asp Gly Thr Asn Ala Val Arg Thr Pro Val Ser Thr
   610 615 620
   Tyr Asn Ala Ala Glu Leu Phe Pro Val Thr Ile Arg Val Arg Asp Ser
   625 630 635 640
   Ala Gly Gly Pro Ala Gln Ser Ala Ser Ala Gln Leu Ile Asp Asp Ala
   645 650 655
   Ala Gly Ala Gly Asn Arg Asn Asp Pro Phe Leu Asp Gln Val Ser Gln
   660 665 670
   Gln Val Gly Leu Val Asp Gly Val Gly Arg Val Leu Val Pro Ala Gly
   675 680 685
   Arg Tyr Ser Ala Leu Gly Trp Val Met Glu Arg Gly Leu Thr Val Arg
   690 695 700
   Arg Trp Ser Ala Met Ser Ala Thr Gln Val Ala Val Ser Gly Pro Ala
   705 710 715 720
   Glu Ile Ala Leu Asn Ala Ala Asn Ala Val Pro Ala Gly Leu Ile Thr
   725 730 735
   Pro Thr Pro Thr Asp Leu Arg Asp Arg Thr Val Met Leu Arg Arg Val
   740 745 750
   Ile Pro Gly Gly Ala Gly Val Asn Gly Tyr Val Gly Glu Ala Gly Leu
   755 760 765
   Ala Ser Gly Ala Gly Trp Glu Val Arg Val Thr Pro Ala Ala Ala Thr
   770 775 780
   Ser Ala Gly Ala Ile Ser Leu Gln Asp Ser Ala Thr Leu Ser Gln Thr
   785 790 795 800
   Ala Val Glu Met Arg Ile Val Gly Gly Gly Pro Ala Val Leu Asn Pro
   805 810 815
   Ala Tyr Asp Val Pro Thr Leu Thr Ala Lys Ser Pro Gly Glu Arg Thr
   820 825 830
   Leu Pro Val Val Phe Gly Gly Ala Gly Asp Leu Thr Gly Leu Asp Val
   835 840 845
   Arg Gly Lys Ala Val Leu Val Arg Ile Pro Val Pro Ala Gly Ala Pro
   850 855 860
   Asp Pro Val Ser Ala Val Ser Ala Gly Ile Ala Asn Ala Ser Arg Ala
   865 870 875 880
   Val Ser Ala Ala Gly Gly Ala Ala Leu Ile Pro Tyr Ala Gly Ser Pro
   885 890 895
   Gly Ser Leu Ser Val Pro Gly Leu Ser Ser Ala Val Val Pro Thr Leu
   900 905 910
   Ser Leu Gly Trp Asp Glu Gly Glu Lys Leu Arg Thr Ala Gly Ala Val
   915 920 925
   Ser Val Arg Leu Leu Val Arg Ser Ala Pro Asp Ala Met Tyr Asn Leu
   930 935 940
   Ser Tyr Leu Asp Ala Asn Gly Val Pro Lys Glu Leu Val Arg Arg Val
   945 950 955 960
   Asp Gln Lys Thr Leu Val Ala Thr Lys Thr Gly Tyr Gln Ala Glu Lys
                   965                 970                 975
   Pro Gly Leu Thr Gly Gln Lys Asn Trp Tyr Ala Phe Pro Thr Gly Leu
               980                 985                 990
   Trp Lys Thr Gln Ala Val Gln Gly Thr Lys Ile Pro Val Pro Gly Ser
           995                 1000                1005
   Trp Thr Glu Tyr Thr Gly Pro Ala Asn Asp Arg Leu Val Trp Lys Arg
       1010                1015                1020
   Val Val Thr Leu Ser Gly Asn Asp Thr Ala Gly Arg Arg Ala Ala Leu
   1025                1030                1035                1040
   Ser Met Asn Ala Gln Asn Val Tyr Arg Ala Gly Glu Arg Ser Arg Pro
                   1045                1050                1055
   Asp Glu Phe Trp Phe Arg Ala Pro Leu His Ser Gly Thr Val Glu Leu
               1060                1065                1070
   Pro Ala Asp His Pro Ala Arg Tyr Pro Ala Thr Ala Ser Gly Trp Ala
           1075                1080                1085
   Val Leu Cys Ser Met Cys Arg Gly Gly Thr Asp Pro Asp Leu Phe Leu
       1090                1095                1100
   Pro Gly Leu Gln Trp Met Asp Gly Thr Thr Gly Ala Gly Gly His Tyr
   1105                1110                1115                1120
   Ala Asn Pro Tyr Glu Asn Ala Arg Tyr Phe Ala Ala Thr Thr Ala Arg
                   1125                1130                1135
   Leu Tyr Arg Asp Gly Thr Glu Ile Pro Arg Ser Asn Ala Asp Asp Pro
               1140                1145                1150
   Leu Ala Leu Ile Pro Glu Phe Arg Leu Ala Pro Thr Pro Ala Thr Tyr
           1155                1160                1165
   Arg Leu Asp Val Thr Asp Val Leu Pro Gly Gln Ala Gln Val Gly Ile
       1170                1175                1180
   Pro Ser Gly Ala Leu Phe Gln His Ala Pro Arg Thr Asp Thr Ser Trp
   1185                1190                1195                1200
   Thr Phe Ala Ser Ala Arg Ser Asp Ala Ala Ala Pro Leu Gly Phe Ser
                   1205                1210                1215
   Cys His Asn Ala Gly Lys Ser Cys Ser Phe Gln Pro Leu Ile Gln Leu
               1220                1225                1230
   Asp His Arg Leu Pro Leu Asp Leu Gly Gly Arg Ala Pro Ala Gly Arg
           1235                1240                1245
   Pro Phe Thr Phe Asp Val Ser Ala Gly Ser His Ser Gly Ala Arg Gly
       1250                1255                1260
   Gly Gly Pro Val Thr Arg Leu Gln Val Ser Ala Ser Thr Asp Gly Gly
   1265                1270                1275                1280
   Arg Thr Trp Thr Ala Ala Thr Val Arg Pro Gly Ala Asn Gly Leu Trp
                   1285                1290                1295
   Ser Val Thr Val Ala His Pro Pro Leu Ala Ala Thr Asp Gly Phe Val
               1300                1305                1310
   Trp Leu Arg Ser Glu Ala Arg Asp Ser Ser Gly Asn Thr Val Thr Gln
           1315                1320                1325
   Thr Val Gln Arg Ala Tyr Ala Leu Thr Asp Val Ala Lys Ala Asn Pro
       1330                1335                1340
   Arg Ala Arg
   1345
<210> 30
   <211> 4044
   <212> DNA
   <213> Amycolatopsis japonicum
<220>
   <223> orf 1653
<400> 30
   atgttccccc gttcccctca cctgacgaga cacccctggc acccggtggc gatcctggcg 60
   gcctccgcac tggtcgccct caccccctta ccggtgatcg cgaccgccgc gaccgccgcc 120
   gtatccggcc ccgctcccgt caccgggcgg ccggacgtcc atcgcgtcac gttgctcacc 180
   ggcgacgtgg tgacctacga acgagacggc gccgggcacg gcaccgcaca ggtcgaaccg 240
   gcggtgcggc cggaccggcc gagcccggcg ttccagatca cgaccacggc cgaagggctg 300
   atggtgtatc cgtcggacgc gcttccgttc gtcggcaagg gactgctgag cccggacctg 360
   ttcaacgtca ccacgctcgt cggagaccgg cgtgacgacg cggcgagcac cacgatcccc 420
   gtcatcgtgc gttacggcga ccggtcgggg ctgtccgccc ggactctgga cgcgaaggcg 480
   aaggcgctcc cgcagaccca gcggctcacc ccgctggtca cggccaacgg agtgggggtt 540
   caggtcaaga aagcggggac gggcgaattc tggcgttccc tcgtcgcgtt cgatgcgagc 600
   gggaaacccg ttttgaacca agggatttcg caagtccggc tggacaggaa ggtccgggtg 660
   acgctggaca agagcaccgc gcgggtcggt gcgccgtcgg cgtggagcgg tgggtggacc 720
   gggaagggga ccacggtcgc ggtggtggac accgggatcg acgagaacca tcccgacctg 780
   gcgggcaaga cggtcgcctc cgcggacttc agcggcgagg gcgacgtcgt ggaccgccac 840
   ggccacggca cgcacgtcgc gtcgatcgtc gccggtaccg gcgcggcatc gggcggccgg 900
   tacaagggtg tggcaccgga cgcgaatctg gtggtggcca aggttttcga cgcgtccggc 960
   gagggtgaca ccgcacaggt gatggccggg atcgactggg ccgtcgcgca gggcgcgaag 1020
   atcgtgaacc tcagcctcgg cgccggcgtc agcgacgggg ccgacccgat gagcgagcag 1080
   atcgactcgc tttcggcgaa gtccggcacg ctgttcgtgg tcgcggccgg gaactcgggg 1140
   ccgggcgacc ggacagtcac gacaccgggg gcggcgacgt ccgcgctcac cgtcggagcg 1200
   ctcgaccggg aggacaagat cgcgtggttc agcagccgcg gcccgcggtt gcgggacgcc 1260
   tcggtcaaac cggagatcac cgcgcccggc gtcgggatcg tcgccgccag ggctgcggac 1320
   acggcgctgg gcgaaccggt ggacgactcc tacaccgccg cgtcgggcac ctcaatggcg 1380
   acaccgcatg tcgcgggcgc cgcggcgatc ctgctgcagc agcgaccggg actgaccggg 1440
   caggcactga agaacaccct cgtcaccacg gccaaggacg tcggtcttca ctggttcgag 1500
   cagggcgccg gaatcctcga cgtcgcgcgg gccgtgtctc agaaggcgac cggcaccgcc 1560
   gtcgcgagct tcggccgcaa cgaacggacg accaacgcct cggcacaggt cgtccggcag 1620
   ctgtcctaca ccaacaccgg ggaccagccg ctgaacctca acctgaagct gaccgtgcga 1680
   ccttgggacg gcggcgccgc gccgggtacc gggatgcgcc tggcgaagac ggatctgtcg 1740
   atcccgccga agtcgagtac caccgtcgac ctcgccgtga acccgaacga aggaacggcg 1800
   ggcgtctacg gcggcgccgt ggtggccacg accgcggacg ggaccaacgc ggtgcggact 1860
   cccgtgagca cctacaacgc ggccgaactg ttcccggtga cgatccgggt ccgggactcg 1920
   gcgggcgggc ccgctcagtc cgcgtcggcc cagctgatcg acgacgcggc cggtgcgggt 1980
   aaccgcaacg acccgttcct cgaccaggtc agccagcagg tcggcctggt ggacggggtc 2040
   ggccgggtgc tcgtgccagc cggacgctat tcggcgctcg gctgggtgat ggagcgcggg 2100
   ctgacggtgc gccgctggtc ggcgatgagc gcgacgcagg tcgctgtcag cggcccggcg 2160
   gagatcgcgc tgaacgcggc gaacgccgtc ccggcggggc tgatcacgcc cactcccacc 2220
   gatctgcggg atcgcacggt gatgctgcgg cgcgtgatcc ccggcggagc cggtgtgaac 2280
   ggttacgtcg gcgaagccgg gctggcttcg ggcgcgggct gggaggtgcg cgtgaccccc 2340
   gcggccgcca cctcggcggg cgcgatttcg ttgcaggaca gcgcgacact gtcccagacc 2400
   gcggtcgaga tgcggatcgt cggtggcggt ccggccgtgc tgaacccggc gtacgacgtg 2460
   ccgacgttga cggcgaagtc gcccggtgag cgaacgctgc cggtcgtgtt cggtggcgcg 2520
   ggtgatctca ccggtctcga cgtccgcgga aaggccgtcc tggtaaggat tccggtcccg 2580
   gccggggcac cggacccggt gagcgcggtc tccgcgggca tcgcgaacgc ttcacgcgcg 2640
   gtgtccgccg cgggtggggc ggcgttgatc ccctacgccg gttctccggg ttcgctgagc 2700
   gttccgggtc tcagcagcgc ggtggtgccg accttgtccc tcggctggga cgaaggcgaa 2760
   aagttgcgga ccgccggtgc cgtttcggtg cgcttgctgg tgcgctcggc tccggacgcg 2820
   atgtacaacc tcagctatct cgacgccaac ggcgtgccga aggagctcgt ccggcgggtc 2880
   gaccagaaga ccttggtcgc cacgaaaacc ggctaccagg ccgaaaaacc gggactgacc 2940
   ggacagaaga actggtacgc cttcccgacc ggcctgtgga agacacaggc ggtgcagggc 3000
   acgaagatcc cggttcccgg ctcctggacc gaatacaccg gcccggcgaa cgatcggctg 3060
   gtctggaaac gggtcgtcac cctgtccggc aacgacaccg cggggcgccg cgccgcgctg 3120
   agcatgaacg cgcagaacgt ctaccgcgcg ggcgagcggt cgagaccgga cgagttctgg 3180
   ttccgcgcgc cactgcacag cggcacggtg gaactgcccg ccgatcatcc ggcccggtac 3240
   ccggcgacgg cctccggctg ggcggtgctg tgctcgatgt gccgtggtgg cacggatccc 3300
   gacctgttcc tccccggcct gcagtggatg gacggcacga ccggggcggg cgggcattac 3360
   gcgaatccct acgagaacgc gcggtacttc gcggcgacca cggcacggct gtaccgggac 3420
   gggaccgaga tcccgcggtc caatgcggac gatccgctcg cgctcatccc ggagttccgg 3480
   ctcgcgccga cccccgccac ctaccggctg gacgtgaccg acgtgctgcc cggccaggcg 3540
   caggtcggca tcccgagcgg ggcgctgttc cagcacgccc cgaggacgga cacgtcgtgg 3600
   acgttcgcct cggcgcggtc agacgccgcg gcaccgctcg gcttctcctg ccacaacgcg 3660
   ggcaagtcgt gttccttcca gccactcatc cagctggacc accggttgcc gctcgatctc 3720
   ggcggccgcg ccccggccgg acgcccgttc accttcgacg tgtccgccgg ctcgcacagc 3780
   ggcgcgcggg gcggcggacc ggtgacgcgg ctgcaggtgt cggcgtcgac ggacggcggg 3840
   cgtacctgga cggcggcgac cgtccggccg ggcgcgaacg ggctgtggtc ggtgacggtg 3900
   gcccacccgc cactcgcggc gacggacggg ttcgtctggc tgcgctcgga agcacgggac 3960
   agctcgggca acaccgtgac gcagacggtc cagcgggcct acgcgctcac ggacgtggcg 4020
   aaggcgaacc cgcgcgctcg ctga 4044
<210> 31
   <211> 197
   <212> PRT
   <213> Amycolatopsis japonicum
<220>
   <223> ORF 1654
<400> 31
   Met Leu Ala Met Gly Met Ala Gly Gly Val Ala Asn Ala Ala Gly Glu
   1               5                   10                  15
   Ser Glu Gly Thr Glu Ala Ala Pro Leu Val Ser Ser Thr Val Val Ile
               20                  25                  30
   Asn Glu Val Ser Thr Arg Gly Val Asn Gly Leu Leu Asp Glu Phe Ile
           35                  40                  45
   Glu Leu Arg Asn Val Ser Asn Gln Pro Gln Asp Val Ser Gly Tyr Val
       50                  55                  60
   Ile Arg Ile Tyr Ser Pro Ser Asn Val Val Thr Asp Thr Ile Val Leu
   65                  70                  75                  80
   Pro Ala Gly Thr Ile Leu Gln Pro Lys Gly Asn Ala Gly Gln Tyr Ala
                   85                  90                  95
   Val Leu Val Gly Gln Asn Phe Ser Gly Thr Val Val Asp Gln Thr Tyr
               100                 105                 110
   Val Ile Pro Phe Thr Leu Thr Gly Ala Glu Gly Ile Pro Thr Ala Gly
           115                 120                 125
   Gly Leu Ser Leu Gln Asn Met Ala Ala Ala Lys Ile Asp Gly Val Ala
       130                 135                 140
   Phe Ser Asn Ala Val Val Ala Pro Arg Glu Gly Gln Ala Ala Ile Pro
   145                 150                 155                 160
   Glu Asn Val Ile Thr Asp Gln Leu Asn Ala Ala Asn Thr Arg Asn Ile
                   165                 170                 175
   Leu Ser Thr Asp Thr Asp Asn Asn Arg Gln Asp Phe Ser Leu Gln Leu
               180                 185                 190
   Arg Thr Pro Gly Leu
           195
<210> 32
   <211> 594
   <212> DNA
   <213> Amycolatopsis japonicum
<220>
   <223> orf 1654
<400> 32
   atgctggcga tgggtatggc cggtggtgtc gcgaacgcgg caggcgagag cgagggcacc      60
   gaggccgccc cgctcgtgtc gtccaccgtg gtcatcaacg aggtttcgac ccgcggcgtg     120
   aacggtctgc tcgacgagtt catcgagctg cgcaacgttt cgaaccagcc gcaggacgtg     180
   agcggctacg tgatccggat ctactcgccg tcgaacgtcg tgaccgacac gatcgtcctg     240
   ccggcgggca cgatcctgca gccgaagggc aacgcgggcc agtacgcggt gctcgtcggg     300
   cagaacttct ccggcaccgt cgtcgaccag acctacgtca tccccttcac cctgaccggc     360
   gccgagggca tcccgacggc cggtggcctt tcgctgcaga acatggcggc cgcgaagatc     420
   gacggtgtgg ccttcagcaa cgccgtcgtg gccccgcgtg aaggccaggc cgcgatcccg     480
   gagaacgtca tcaccgatca gctcaacgcg gcgaacaccc gcaacatcct gagcaccgac     540
   accgacaaca accggcagga cttctcgctg cagctgcgca ccccgggtct gtaa           594
<210> 33
   <211> 94
   <212> PRT
   <213> Amycolatopsis japonicum
<220>
   <223> ORF 1655
<400> 33
   Met Thr Ala Asn Ser Glu Arg Gly Ala Ala Ala Pro Leu Cys Glu Leu
   1               5                   10                  15
   Leu Ala Glu Gln Arg Ile Ala Lys Gly Trp Ser Gln Gly Asp Leu Val
               20                  25                  30
   Val Lys Leu His Thr Arg Ser Gly Asn Asp Ser Val Thr Arg Glu Glu
           35                  40                  45
   Ile Ser Arg Trp Glu Arg Gly Lys Arg Ile Pro Gly Pro Tyr Trp Arg
       50                  55                  60
   Gln Trp Leu Ser Asp Val Leu Asp Thr Ser Ser Arg Glu Leu Glu Leu
   65                  70                  75                  80
   Ala Ala Ala Val Ala Arg Arg Arg Arg Arg Asn Ile Ile Ala
                   85                  90
<210> 34
   <211> 285
   <212> DNA
   <213> Amycolatopsis japonicum
<220>
   <223> orf 1655
<400> 34
   gtgaccgcga acagcgaacg cggggccgcg gccccgcttt gcgaactgct cgccgaacaa     60
   cgaatcgcga aaggatggtc acaaggcgat ctggtcgtga agttgcacac ccgctccggc    120
   aacgacagtg tcaccaggga agagatttcc cgctgggagc ggggaaaacg cattcccggt    180
   ccctattggc ggcagtggct cagcgatgtg ctggacacct ccagccgcga gctcgaactc    240
   gccgccgcgg tcgcgagaag gcggcgccgg aacattatcg cgtga                    285
<210> 35
   <211> 166
   <212> PRT
   <213> Amycolatopsis japonicum
<220>
   <223> ORF 1656
<400> 35
   Met Thr Lys Pro Gly Ser Leu Phe Leu Leu Leu Ala Leu Ala Gly Thr
   1               5                   10                  15
   Pro Ala Pro Asp Thr Val Arg Ala Ser Ser Ala Lys Ile His Glu Val
               20                  25                  30
   Leu Gly Gly Pro Ala Gly Tyr Ile Asp Leu His Asn Thr Gly Ala Thr
           35                  40                  45
   Thr Leu Asp Ile Gly Gly Trp Ser Val Gln Ala Cys Thr Gly Gly Ala
       50                  55                  60
   Thr Pro Val Glu Leu Ala Ala Met Pro Ala Gly Ser Glu Ile Pro Ala
   65                  70                  75                  80
   Gly Asp His Phe Leu Ile Thr Ala Gln Gly Phe Gly Gly Thr Met Gln
                   85                  90                  95
   Gln Val Val Val Glu Ala Ile Val Gly Asp Gly Glu Ile Leu Leu Asp
               100                 105                 110
   Arg Arg Gly Ala Arg Val Asp Ser Val Gly Trp Ala Pro Ser Ser Pro
           115                 120                 125
   Cys Arg Glu Asn Gln Ala Ala Val Ser Cys Pro Gly Leu Ala Gln Ser
       130                 135                 140
   Arg Asp Ala Val Ser Arg Asp Thr Asp Asn Asn Lys Ala Asp Phe Gly
   145                 150                 155                 160
   Cys Val Arg Pro Ala Gly
                   165
<210> 36
   <211> 501
   <212> DNA
   <213> Amycolatopsis japonicum
<220>
   <223> orf 1656
<400> 36
   atgacgaagc caggttccct gttcctgctc ctcgcactcg ccggaacccc ggccccggac      60
   accgtccggg cttcgtcggc caagatccac gaggtgctcg gcggcccggc aggctacatc     120
   gacctgcaca acaccggcgc caccaccctt gacatcggcg gctggtccgt tcaggcgtgc     180
   acgggcgggg cgacacccgt cgaactggcc gccatgcccg ccgggagcga gatccccgcc     240
   ggtgaccatt tcctcatcac cgcacagggt ttcggcggga cgatgcagca ggtggtcgtc     300
   gaggctatcg tcggggacgg cgagatactg ctggaccggc gcggggcgag ggtcgacagc     360
   gtcggatggg cgccgtcgtc gccctgccgc gagaaccagg ccgccgtctc ctgccccggg     420
   ctcgcccagt cgagggacgc cgtcagccgc gacaccgaca acaacaaggc cgacttcggc     480
   tgtgtacggc cggccggctg a                                               501
<210> 37
   <211> 231
   <212> PRT
   <213> Amycolatopsis japonicum
<220>
   <223> ORF 1657
<400> 37
   Met Asn Arg Val Thr Thr Leu Val Val Asp Asp Gln Pro Leu Ile Arg
   1               5                   10                  15
   Tyr Ala Leu Ser Arg Leu Leu Arg Asp Ser Ala Gly Val Gly Ala Val
               20                  25                  30
   Ala Glu Ala Ala Ser Glu Pro Glu Ala Leu Trp Gln Cys Glu Lys Ser
           35                  40                  45
   His Pro Asp Met Val Ile Thr Glu Leu Ser Ile Trp Gly Glu Pro Ala
       50                  55                  60
   Gly Thr Ala Ile Cys Arg Phe Val Lys Glu Gln Phe Glu Arg Ala Ala
   65                  70                  75                  80
   Val Leu Val Phe Ala Gly Asp Ser Ser Pro Ser Ala Ile Ala Met Ala
                   85                  90                  95
   Leu Asn Ala Gly Ala Asp Ser Phe Val His Lys Ser Ala Ser Gly Gly
               100                 105                 110
   Gln Val Met Ser Ala Val Arg Ser Thr Leu Ala Gly Gln Arg Val Cys
           115                 120                 125
   Leu Gln Ala Gly Gly Thr Pro Pro Ala Val Thr Glu Ala Val Arg Tyr
       130                 135                 140
   Ala Ser Ala Met Thr Arg Arg Glu Glu Glu Ile Leu Ala Leu Ile Leu
   145                 150                 155                 160
   Tyr Arg Trp Ser Asn Asp Glu Ile Ala Glu Glu Leu His Leu Ala Thr
                   165                 170                 175
   Gln Thr Val Lys Asn Tyr Val Ser Arg Ile Leu Gln Lys Leu Gly Phe
               180                 185                 190
   Gly Ser Arg Arg Asp Leu Phe Arg Ala Leu Arg Phe Arg Pro Gly Gly
           195                 200                 205
   Asn Leu Leu Pro Arg Leu Glu Thr Thr Glu Arg Glu Gly Glu Leu His
       210                 215                 220
   Thr Ala Asp Gly Tyr Arg Gly
   225                 230
<210> 38
   <211> 696
   <212> DNA
   <213> Amycolatopsis japonicum
<220>
   <223> orf 1657
<400> 38
   atgaaccgcg tcaccacgct cgtagtagac gatcaacccc tcatcagata cgcgctcagc      60
   aggctgttgc gggattcggc gggggtgggg gcggtggccg aagccgcgag tgagcctgaa     120
   gcgttgtggc agtgcgaaaa gagccatccc gacatggtca tcaccgagct gtcgatctgg     180
   ggtgagcctg ccgggaccgc gatctgccgg ttcgtgaagg aacagttcga acgcgctgcc     240
   gtcctggtct tcgccggtga ttcgtcgcca tcggccatcg cgatggcgct gaacgcgggt     300
   gcggacagct tcgtgcacaa gtcggccagc ggcggccagg tgatgtcggc ggtgcgctcg     360
   acgctcgccg ggcagcgggt gtgcctgcag gcgggcggaa ccccgccggc ggtcaccgag     420
   gccgtgcgct acgcgagcgc gatgaccaga cgggaggagg agatcctcgc gctgatcctc     480
   taccggtggt cgaacgacga gatcgccgag gaactgcatc tcgccacgca gaccgtgaag     540
   aactacgtca gccggatcct gcagaagctg ggcttcggca gcagacgcga cctcttccgg     600
   gcgctgcgtt tccggccggg cggaaacctg ttgccgcgcc tggagacgac cgagcgcgaa     660
   ggcgagttgc acacggcgga cgggtaccgg ggctag                               696
<210> 39
   <211> 139
   <212> PRT
   <213> Amycolatopsis japonicum
<220>
   <223> ORF 1658
<400> 39
   Met Asp Asp Ser Tyr Arg Leu Glu Ala Gly Ser Pro Ser Val Asp Asp
   1               5                   10                  15
   Tyr Leu Arg Leu Arg Arg Glu Ala Gly Leu Ser Glu Pro Pro Arg Glu
               20                  25                  30
   Gln Ala Glu Arg Gly Val Arg Gly Ala Trp Thr Ser Val Arg Val Val
           35                  40                  45
   His Arg Pro Thr Gly Glu Thr Val Gly Met Gly Arg Val Ile Ser Asp
       50                  55                  60
   Gly Gly Trp Tyr Phe His Ile Val Asp Met Ala Val Leu Pro Ala His
   65                  70                  75                  80
   Gln Arg Arg Gly Ile Gly Asp Ala Val Leu Glu Ala Leu Leu Ala Ala
                   85                  90                  95
   Ile Asp Thr Ala Ala Pro Gly Ala Tyr Val Asn Leu Leu Gly Asp Pro
               100                 105                 110
   Pro Gly Trp Arg Leu Tyr Lys Arg His Gly Phe Val Glu Thr Ala Pro
           115                 120                 125
   Gly Thr Ile Gly Met Arg Arg Gln Asn Ala Gly
       130                 135
<210> 40
   <211> 420
   <212> DNA
   <213> Amycolatopsis japonicum
<220>
   <223> orf 1658
<400> 40
   atggacgact cttaccgcct ggaagcaggc tccccgtcgg tcgacgacta cctgcggctg      60
   cgccgcgagg ccggtctcag cgaaccgccg agggaacagg cggagcgcgg ggttcgcggc     120
   gcctggacgt cggtccgggt ggtccaccgg cccaccggcg agaccgtcgg gatgggccgg     180
   gtgatcagcg acggtgggtg gtacttccac atcgtcgaca tggccgttct gcccgcccat     240
   cagcgccgcg gcatcgggga cgccgtcctc gaggcccttc tcgcggcgat cgacaccgcg     300
   gcgccgggtg cgtacgtcaa tctgctcggc gatccacccg gatggcggct ctacaaacgc     360
   cacggtttcg tggagaccgc tcccgggacc atcggcatgc ggcgccaaaa cgccggataa     420
<210> 41
   <211> 174
   <212> PRT
   <213> Amycolatopsis japonicum
<220>
   <223> ORF 1659
<400> 41
   Met Thr Val Thr Thr Leu Arg Pro Gly Leu Pro Glu Leu Ile Lys Arg
   1               5                   10                  15
   Leu Arg Gln Cys Thr Gly Arg Pro Ser Glu Pro Leu Val Thr Ala Gln
               20                  25                  30
   Ala Val Ala Asn Val Leu Thr Glu Leu Arg Pro Thr Pro Gln Leu Leu
           35                  40                  45
   Thr Ala Thr Glu Arg Ala Gly Ser Pro Asp Gly Tyr Thr Arg Thr Thr
       50                  55                  60
   Leu His Ala Glu Ser Ala Phe Ser Ile Val Gly Leu Val Trp Arg Pro
   65                  70                  75                  80
   Gly Gln Leu Thr Glu Ile His Asp His Leu Val Trp Cys Thr Phe Leu
                   85                  90                  95
   Val Leu Gln Gly Thr Glu Thr Glu Thr Ile Phe Asp Ile Asp Asp Asp
               100                 105                 110
   Arg Leu Val Arg Lys Ala Gln Arg Gln Arg Pro Ala Gly Ser Val Ser
           115                 120                 125
   Gly Val Ala Pro Pro Asp Asp Ile His Gln Val His Asn Ala Gly Asp
       130                 135                 140
   Thr Val Ala Ile Thr Leu His Val Tyr Gly Ala Asp Leu Ser Lys Gly
   145                 150                 155                 160
   Thr Ser Val Arg Arg Asn Tyr Arg Met Arg Ala Thr Ser Arg
                   165                 170
<210> 42
   <211> 525
   <212> DNA
   <213> Amycolatopsis japonicum
<220>
   <223> orf 1659
<400> 42
   atgaccgtca cgaccctcag accaggcctt ccggagctga tcaaacgtct gcgccaatgc      60
   accggaagac catccgaacc ccttgtcacc gcacaagccg tggcgaacgt tctcaccgaa     120
   ctccgcccga ctccgcaact gctcaccgcg acggaacgcg ccggctcccc cgacggctac     180
   acccgcacca ccctgcacgc cgagtccgcc ttttcgatcg tcggactggt ctggcggccc     240
   ggtcagctca ccgagatcca cgatcacctg gtgtggtgta cattccttgt cctgcaagga     300
   accgagaccg agaccatctt cgacatcgac gacgatcggc tggtccggaa ggcccagcgg     360
   caacgcccgg cggggtcggt gagcggcgtc gcgccgcccg acgacatcca ccaggtgcac     420
   aacgcgggcg acaccgtcgc catcaccttg cacgtgtacg gcgccgacct gagcaaggga     480
   accagcgtgc gccgcaatta ccggatgcgc gccacttcgc ggtga                     525
<210> 43
   <211> 288
   <212> PRT
   <213> Amycolatopsis japonicum
<220>
   <223> ORF 1660
<400> 43
   Met Leu Asn Leu Val Gln Leu Thr Val Leu Ala Ala Val Ala Arg His
   1               5                   10                  15
   Gly Ser Met Thr Ala Ala Ala Lys Glu Leu His Tyr Thr Gln Pro Ala
               20                  25                  30
   Val Ser His His Leu Ala Arg Leu Glu Ala Ala Thr Gly Ala Lys Leu
           35                  40                  45
   Val Gln Arg Ile Gly Arg Gly Ile Arg Leu Thr Pro Glu Gly Glu Leu
       50                  55                  60
   Leu Ala Ala Arg Ala Ala Glu Ile Val Gly Arg Val Glu Gly Ala Glu
   65                  70                  75                  80
   Ala Glu Leu Ala Ala Gln Val Gly Leu Arg Ala Gly Arg Val Arg Met
                   85                  90                  95
   Ala Gly Phe Gln Ser Ile Leu Ser Thr Ile Val Pro Asp Ala Ala Ala
               100                 105                 110
   Thr Leu Ala Arg Ala His Pro Gly Leu Glu Leu Gly Leu Val Asp Glu
           115                 120                 125
   His Pro Gly Glu Ala Leu Arg Met Leu Arg Glu Gly Arg Ile Asp Val
       130                 135                 140
   Ala Leu Ile Phe Arg Tyr Ala Asp Thr Pro Arg Glu Glu Gln Gly Leu
   145                 150                 155                 160
   Arg Leu Val His Leu Leu Glu Asp Pro Ile Tyr Leu Leu Thr Ser Glu
                   165                 170                 175
   Pro Gly Gln Thr Ile Ala Asp His Arg Asp Ser Thr Trp Ile Gly Gly
               180                 185                 190
   Cys Ala Arg Cys Gln Asp Glu Leu Val Thr Ile Cys Gly Arg Ala Gly
           195                 200                 205
   Phe Ala Pro Arg Ile Ser Met Val Ser Asp Asp Ile Val Val Met Gln
       210                 215                 220
   Ala Leu Val Ala Ala Arg Met Gly Val Thr Thr Leu Pro Arg Leu Ala
   225                 230                 235                 240
   Leu Arg Ala His Arg Met Pro Gly Val His Ala Thr Glu Leu Ala Glu
                   245                 250                 255
   Asp Pro Arg Gln Ile Tyr Ala Val Thr Tyr Gly Asp Pro Pro Asp Pro
               260                 265                 270
   Pro Ala Ala Ala Ala Leu Ile Asp Ala Leu Gln Thr Ser Ile Arg Ala
           275                 280                 285
<210> 44
   <211> 867
   <212> DNA
   <213> Amycolatopsis japonicum
<220>
   <223> orf 1660
<400> 44
   atgctgaatc tggtgcagct cacggtgctg gcggcggtcg cgcggcacgg ttcgatgacc      60
   gcggcggcga aggaactgca ctacacgcag cccgcggtga gccaccatct cgcgaggttg     120
   gaggcggcga cgggcgcgaa gctggtccag cggatcggcc gcggcatccg gctcacaccg     180
   gaaggggaac tgctcgccgc ccgcgcggcc gagatcgtcg gccgggtcga aggcgccgaa     240
   gccgagttgg ccgcgcaggt gggcttgcgc gccgggcggg tccggatggc ggggttccag     300
   tcgatcctga gcacgatcgt ccccgacgcg gccgcgacgc tggcccgtgc gcatcccgga     360
   ctcgaactcg ggctggtgga cgaacatccg ggcgaggcgc tgcgcatgct gcgggagggg     420
   cggatcgacg tcgccctgat cttccggtac gcggacacgc cccgcgagga acaggggctc     480
   cggctcgtgc acctgctgga ggacccgatc tacctgctca ccagcgaacc ggggcagacc     540
   atcgccgatc atcgtgactc gacctggatc ggcggctgcg cccgatgcca ggacgaactc     600
   gtcacgatct gcgggcgggc cgggttcgcc ccgcgtattt cgatggtcag cgacgacatc     660
   gtggtcatgc aggcgctggt cgcggcccgg atgggcgtca ccacgctgcc ccggctggcg     720
   ctgcgtgcgc atcggatgcc cggtgtgcac gccacggagc tggccgaaga cccgcgtcag     780
   atctatgccg tcacctatgg cgacccgccc gatcctccgg ccgcagcggc gttgatcgac     840
   gcgcttcaga cgagcatccg ggcttag                                         867
<210> 45
   <211> 411
   <212> PRT
   <213> Amycolatopsis japonicum
<220>
   <223> ORF 1661
<400> 45
   Met Arg His Leu Asn Ala Ile Gly His Leu Ala Pro His Asp His Ala
   1               5                   10                  15
   Ala Ala Pro Ala Gly Pro Leu Ser Gly Ile Pro Leu Val Val Lys Asp
               20                  25                  30
   Asn Ile His Val Ala Gly Met Pro Asn Thr Ala Gly Thr Pro Ala Leu
           35                  40                  45
   Ala Gly His Val Pro Arg Glu His Ala Thr Val Val Arg Arg Leu Thr
       50                  55                  60
   Asp Ala Gly Ala Val Val Val Gly Lys Ala Thr Met His Glu Leu Ala
   65                  70                  75                  80
   Leu Gly Ile Thr Cys Asp Thr Thr Pro Leu Gly Pro Val Arg Asn Ala
                   85                  90                  95
   Cys Asp Pro Ser Arg Phe Ala Gly Gly Ser Ser Gly Gly Thr Ala Val
               100                 105                 110
   Ala Val Ala Ala Gly Ile Val Pro Ala Gly Leu Gly Thr Asp Thr Gly
           115                 120                 125
   Gly Ser Ala Arg Val Pro Ala Ala Leu Asn Gly Val Cys Gly Phe Arg
       130                 135                 140
   Pro Thr Thr Gly Arg Tyr Pro Ser Asp Gly Met Thr Pro Leu Ser Ser
   145                 150                 155                 160
   Thr Arg Asp Thr Ala Gly Pro Ile Ala Arg Thr Val Ala Asp Leu Ala
                   165                 170                 175
   Leu Leu Asp Ala Val Leu Ala Ala Glu Glu Pro Thr Pro Leu Ile Glu
               180                 185                 190
   Ser Thr Ser Val Arg Leu Gly Val Pro His Gly Phe Leu Thr Gly Asp
           195                 200                 205
   Leu Ser Glu Asp Val Glu Glu Leu Trp Glu Ala Ala Leu Ala Arg Leu
       210                 215                 220
   Gly Ala Ala Gly Val Thr Leu Val Pro Leu Asp Asp Thr Pro Leu Ala
   225                 230                 235                 240
   Glu Leu Val Val Asp Gln Gly Met Pro Leu Val Ile His Glu Ala Gly
                   245                 250                 255
   Val Gly Leu Arg Ser Tyr Leu Ala Glu His Val Pro Glu Val Ser Phe
               260                 265                 270
   Glu Arg Leu Val Arg Glu Ile Ala Ala Pro Asp Val Arg Ala Ile Phe
           275                 280                 285
   Ala Glu Ala Val Val Pro Gly Val Glu Pro Ala Val Tyr Glu Ala Ala
       290                 295                 300
   Ile Thr Thr Arg Ser Ala Leu Arg Arg Ala Tyr Ala Lys Ile Phe Asp
   305                 310                 315                 320
   Glu Ser Gly Ile Asp Ala Leu Ala Phe Pro Thr Thr Pro Ala Thr Ala
                   325                 330                 335
   Arg Asp Phe Ser Ala Val Gly Ser Phe Val His Arg Gly Arg Glu Val
               340                 345                 350
   Pro Thr Phe Pro Thr Phe Ile Arg Asn Cys Gln Pro Gly Ser Ile Ala
           355                 360                 365
   Gly Phe Pro Gly Leu Thr Val Pro Met Gly Arg Ala Arg Asp Gly Leu
       370                 375                 380
   Pro Ala Gly Leu Ala Leu Asp Gly Leu Val Gly Asp Asp Arg Lys Leu
   385                 390                 395                 400
   Leu Gly Val Gly Ala Phe Val Glu Arg Val Leu
                   405                 410
<210> 46
   <211> 1236
   <212> DNA
   <213> Amycolatopsis japonicum
<220>
   <223> orf 1661
<400> 46
   gtgcggcatc tcaacgcgat cggccacctg gcgccgcacg atcacgcggc cgcgccggcc      60
   ggcccgctca gcgggatccc gttggtggtc aaggacaaca tccacgtcgc cgggatgccg     120
   aacaccgcgg gtaccccggc gctggcgggc cacgttccgc gcgagcacgc gaccgtggtc     180
   cgacggctga ccgacgcggg cgccgtcgtc gtcggcaagg cgaccatgca cgaactcgcc     240
   ctcggcatca cctgcgacac gaccccgctc ggcccggtgc gcaacgcgtg cgacccgtcc     300
   cggttcgccg ggggcagcag cggcgggacc gcggtcgccg tcgcggcggg catcgtgccc     360
   gcgggtctgg gcaccgacac cggcggttcg gcccgtgttc ccgccgcgct caacggggtt     420
   tgcggcttcc ggccgaccac gggccgctac ccgtccgacg gcatgacgcc gctgagcagc     480
   acgcgggaca cggccgggcc gatcgcccgc acggtcgccg atctcgcgct cctcgacgcg     540
   gtcctcgcgg cggaagagcc gacacccctg atcgagagca cgtcggtcag gctgggtgtg     600
   ccgcacggtt tcctgaccgg cgacctgtcc gaggacgtcg aagaactctg ggaagccgcg     660
   cttgcccgct tgggcgcggc aggcgtcacc ctggtgccgc tcgacgacac gccgctcgcc     720
   gaactcgtcg tcgatcaggg aatgccgctg gtgatccacg aagcaggcgt cgggctccgc     780
   tcctacctcg ccgagcacgt acccgaggtt tcgttcgagc ggctcgttcg cgagatcgcc     840
   gcgcccgacg tccgggcgat cttcgccgaa gcggtcgtgc cgggcgtcga acccgccgtc     900
   tacgaagcgg ccatcaccac ccgctccgcc ttgcgtcgcg cctacgcgaa gatcttcgac     960
   gagagcggca tcgacgcgct ggccttcccc accacaccgg cgacggcccg ggacttctcg    1020
   gccgtcggga gcttcgtgca ccgagggcgc gaggtcccga ccttccccac cttcatccgc    1080
   aactgccagc ccggcagtat cgcgggattc cccggcctga ccgtcccgat ggggcgcgca    1140
   cgcgacggcc tgcccgccgg gctggcgctc gacggcctgg tcggggacga ccggaagttg    1200
   ctgggcgtgg gggctttcgt cgaacgggtt ctgtag                              1236
<210> 47
   <211> 327
   <212> PRT
   <213> Amycolatopsis japonicum
<220>
   <223> ORF 1662
<400> 47
   Met Ser Ser Gly Pro Ser Leu His Val Leu Thr Arg Ser Asp Leu His
   1               5                   10                  15
   Ser Leu Glu Leu Thr Pro Gly Glu Ala Ile Thr Met Val Glu Asp Gly
               20                  25                  30
   Tyr Leu Ala Tyr Ala Ala Gly Ala Ser Arg Asn Pro Ala Lys Leu Met
           35                  40                  45
   Val Pro Ile Pro Asp Pro Ala Arg Asp Ala Val Ala Tyr Ser Met Leu
       50                  55                  60
   Gly Tyr Asp Gly Ser Leu Glu Gln Ala Ala Phe Lys Thr Ser Tyr Arg
   65                  70                  75                  80
   Gln Gly Ser Thr Ser Ala Glu Lys Tyr Tyr Thr Thr Ile Thr Leu Tyr
                   85                  90                  95
   Asp Asp Thr Thr Gly Leu Pro Phe Ala Leu Met Asp Cys His Arg Val
               100                 105                 110
   Gly Ala Thr Arg Thr Pro Ala Ser Thr Ala Leu Ile Ala Arg Ser Cys
           115                 120                 125
   Ala Arg Pro Gly Ala Arg Ser Ala Leu Met Val Gly Thr Gly Ala Gln
       130                 135                 140
   Gly Ile Arg Thr Leu Pro Tyr Leu Leu Thr Ala Leu Pro Glu Leu Glu
   145                 150                 155                 160
   Arg Leu Arg Leu Phe Gly Thr His Pro Asp Gly Leu Arg Asp Ser Val
                   165                 170                 175
   Ala Ala Leu Lys Glu Arg Phe Pro Asp Arg Glu Val Glu Leu Val Asp
               180                 185                 190
   Asp Val Glu Ala Ala Ala Arg Glu Ser Asp Ile Val Val Ala Ala Ser
           195                 200                 205
   Gly Arg Ala Ala His Pro Lys Ile Arg Leu Gly Trp Leu Pro Pro Gly
       210                 215                 220
   Gly Leu Leu Ile Ser Val Ala Ser Lys Gly Val Gln Glu Gly Thr Leu
   225                 230                 235                 240
   Ala Glu Ala Asp Tyr Thr Val Ala Thr Ser Gly Ala Gln Val Glu Val
                   245                 250                 255
   Thr Gly Gln Arg Met Ala Gly Pro Asp Gly Val Phe Arg Ile Asp Ala
               260                 265                 270
   Glu Leu Pro Glu Ile Leu Ala Gly Lys Ala Pro Gly Arg Arg Gly Asp
           275                 280                 285
   Glu Asp Arg Val Phe Ala Phe Ser Ser Gly Met Ile Ile Thr Asp Ile
       290                 295                 300
   Pro Val Ala His Ala Leu Ala Ala Arg Ala Ile Ala Ala Gly Arg Gly
   305                 310                 315                 320
   Arg Glu Val Ala Leu Trp Thr
                   325
<210> 48
   <211> 984
   <212> DNA
   <213> Amycolatopsis japonicum
<220>
   <223> orf 1662
<400> 48
   atgagctccg gtccctcgct gcacgtactc acgcgctcgg atctgcacag cctcgaactc      60
   acccctggcg aggcgatcac gatggtcgaa gacggctatc tggcctacgc ggccggggcc     120
   tcgcggaacc cggcgaagct gatggtgccg atcccggatc ccgcgcggga cgccgtcgcg     180
   tactcgatgc tcggctacga cggttccctc gaacaggccg cgttcaagac gagctaccgc     240
   caaggcagca cgtcggccga gaagtactac acgaccatca ccctgtacga cgacaccacg     300
   ggcctgccgt tcgcgctcat ggactgccac cgcgtcggcg ccacccgcac gcccgcgagc     360
   accgcgttga tcgcgcggtc ctgcgctcgg ccgggcgcgc gatccgcgct catggtcggc     420
   accggcgcgc aggggatccg gacgctgccg tacctgctca ccgcgctgcc ggagctggaa     480
   cggctgcggc tgttcggcac gcatcccgac ggtcttcgcg acagcgtcgc cgcgctgaag     540
   gagcgcttcc cggaccgcga ggtcgaactc gtcgacgacg tggaggccgc ggcgcgcgag     600
   tccgacatcg tggtcgccgc gtcgggccgg gcggcccacc cgaagatccg tctcggctgg     660
   ctgccgccgg gcgggctgct gatctccgtc gccagcaagg gagtccagga aggcacgctc     720
   gccgaggccg actacacggt ggccaccagc ggcgcgcagg tcgaggtgac cgggcagcgg     780
   atggcgggtc cggacggcgt gttccggatc gacgccgaac tcccggagat cctcgcgggc     840
   aaggcccccg gccgccgcgg cgacgaagac cgggtgttcg ccttcagcag cggcatgatc     900
   atcaccgaca tcccggtggc gcacgcgctg gccgcccgcg ccatcgccgc cggccgcggc     960
   cgcgaggtgg ccctgtggac ctga                                            984
<210> 49
   <211> 448
   <212> PRT
   <213> Amycolatopsis japonicum
<220>
   <223> ORF 1663
<400> 49
   Met Asp Leu Ile Asp Thr Leu Ser Pro Pro Leu Pro Ala Leu Glu Arg
   1               5                   10                  15
   Pro Trp Ala His Arg Leu Arg Ala Ser Glu Thr Leu Trp Glu Ile Ala
               20                  25                  30
   Arg Gly Val Gly Gly Gly Pro Phe His Val Val His Pro Ala Thr Phe
           35                  40                  45
   Ala Glu Asn Leu Gly Glu Met Val Asp Ala Leu Ala Ala Glu Arg Val
       50                  55                  60
   Glu Gly Thr Val Tyr Tyr Gly Lys Lys Ala Asn Lys Ala Ala Ala Trp
   65                  70                  75                  80
   Leu Arg Glu Cys Thr Arg Pro Gly Thr Gly Val Asp Val Ala Ser Val
                   85                  90                  95
   Pro Glu Leu Val His Ala Leu Gly Asn Gly Leu Arg Gly Glu Ala Ile
               100                 105                 110
   Gly Val Thr Gly Ala Ala Lys Pro Asp Gly Leu Leu Trp Leu Ala Leu
           115                 120                 125
   Arg His Arg Cys Leu Ile Ala Val Asp Ala Ala Asp Glu Leu Glu Arg
       130                 135                 140
   Val Ala Arg Leu Ala Thr Glu Leu Gly Glu Thr Ala Glu Val Leu Leu
   145                 150                 155                 160
   Arg Val Arg Pro Pro Ser Ala Pro Glu Ser Arg Phe Gly Phe Ala Pro
                   165                 170                 175
   Asp Ala Val Lys Ala Ala Val Arg Gln Cys Gly Gly Pro Val Val Leu
               180                 185                 190
   Arg Gly Phe Ser Phe His Leu Asp Gly Tyr Asp Pro Val Pro Arg Ala
           195                 200                 205
   Glu Leu Ala Ala Tyr Leu Ile Asp Leu Cys His Asp Ala Arg Ala Leu
       210                 215                 220
   Gly His Pro Ala Ser Lys Ile Ser Ala Gly Gly Gly Ile Ala Val Ser
   225                 230                 235                 240
   Tyr Val Asp Ala Gly Asp Trp Ala Arg Phe Glu Ser Gly Arg His Asp
                   245                 250                 255
   Gly Trp Phe His Ala Gly Arg Asn Pro Ala Arg Thr Tyr Pro Tyr His
               260                 265                 270
   Gln Ala Pro Thr Gly Ala Ala Met Val Thr Ala Ile Leu Arg His Glu
           275                 280                 285
   Ile Ala Gly Lys Thr Leu Ala Glu Arg Leu Arg Ala Ala Gly Ile Glu
       290                 295                 300
   Leu Leu Leu Glu Pro Gly Arg Ala Leu Val Asp Gly Ala Gly Phe Ser
   305                 310                 315                 320
   Val Phe Pro Val Leu Gly Cys Lys Pro Ala Glu Asp His Leu Ile Thr
                   325                 330                 335
   Thr Val Ala Gly Leu Ser Met Ser Leu Ser Glu Gln Trp Lys Gly Ser
               340                 345                 350
   Glu Phe Leu Pro Asp Pro Leu Leu Val Arg Arg Asp Gly Pro Gly Gly
           355                 360                 365
   Thr Pro Val Arg Thr Ile Val Ala Gly Ser Ser Cys Met Glu Tyr Asp
       370                 375                 380
   Val Leu Thr Trp Arg Ala Val Glu Leu Pro Ala Gln Pro Arg Thr Gly
   385                 390                 395                 400
   Asp Leu Leu Val Tyr Pro Asn Thr Ala Gly Tyr Gln Met Asp Lys Asn
                   405                 410                 415
   Glu Ser Gly Phe His Gln Leu Pro Leu Pro Pro Lys Val Val Val Asp
               420                 425                 430
   Gly Asp Arg Trp His Leu Asp Thr Asp His Pro Ile Gln Glu Ile Arg
           435                 440                 445
<210> 50
   <211> 1347
   <212> DNA
   <213> Amycolatopsis japonicum
<220>
   <223> orf 1663
<400> 50
   gtggacctga tcgacaccct gtccccgccc ctgcccgcgc tcgaacgtcc ctgggcgcac      60
   cggctgcgtg cttcggagac gctgtgggag atcgcccgcg gggtcggcgg cggcccgttc     120
   cacgtggtcc atccggccac tttcgccgag aacctcgggg aaatggtcga cgcgctcgcc     180
   gccgaacggg tcgaaggcac cgtctactac gggaagaagg cgaacaaggc ggcggcctgg     240
   ctgcgggaat gcacgcgtcc cggcacgggg gtcgacgtcg cgagtgtgcc cgaactggtg     300
   cacgccctcg gaaacgggct gcgcggcgaa gcgatcgggg tcaccggcgc cgccaaaccc     360
   gacggcctcc tttggctcgc cctgcggcat cgctgtctca tcgcggtgga cgcggccgac     420
   gagctggaac gcgtcgcccg gctggccacc gaactcgggg agaccgccga agtgctgctg     480
   cgagtgcggc cgccgtccgc gccggagagc cggttcggtt tcgcgcccga cgcggtgaaa     540
   gcggccgtgc gacagtgcgg cggcccggtc gtcctgcggg gtttctcgtt ccacctcgac     600
   ggctacgacc ccgtgccgag ggcggaactc gccgcatacc tgatcgatct gtgtcacgac     660
   gcccgcgcgc tcggacatcc cgcgtcgaag atcagtgcgg gcggcgggat cgctgtgtcc     720
   tatgtggacg ccggtgactg ggcgcggttc gagtccggca ggcacgacgg ctggttccac     780
   gccggccgta atccggccag gacctatcca taccaccagg cgccgaccgg ggcggcgatg     840
   gtgaccgcga tcctccggca cgagatcgcc gggaagacac tggccgagcg cctgcgcgcg     900
   gccgggatcg agctgctgct cgaaccgggc cgagccctcg tcgacggcgc cgggttctcg     960
   gtgttcccgg tgctgggctg caaaccggcc gaagaccacc tgatcaccac ggtcgcgggc    1020
   ctgtccatga gcctgtccga acagtggaag ggcagcgagt tcctgcccga cccgctgctc    1080
   gtgcgcaggg acggaccggg cggtacgcca gtgcggacca tcgtcgcggg gtcgagctgc    1140
   atggagtacg acgtgctgac ctggcgcgcg gtggagctgc ccgcgcaacc gaggaccggc    1200
   gacctgctcg tgtaccccaa cacggccggc taccagatgg acaagaacga gagcggtttc    1260
   caccagcttc cgttgccgcc gaaagtggtg gtcgacggcg accgctggca cctcgacacc    1320
   gaccacccga tccaggagat ccgatga                                       1347
<210> 51
   <211> 352
   <212> PRT
   <213> Amycolatopsis japonicum
<220>
   <223> ORF 1664
<400> 51
   Met Thr Ile Val Arg Arg Ala Ser Asp Leu Ile Gly Asn Thr Pro Leu
   1               5                   10                  15
   Leu Glu Leu Ala Arg Thr Gly Thr Gly Thr Arg Leu Leu Leu Lys Leu
               20                  25                  30
   Asp His Leu Asn Pro Thr Gly Ser Cys Lys Val Arg Met Ala Arg Gln
           35                  40                  45
   Met Ile Asp Glu Ala Glu Leu Asp Gly Arg Leu Arg Pro Gly Gly His
       50                  55                  60
   Ile Val Glu Pro Thr Ser Gly Asn Thr Gly Asn Gly Leu Ala Leu Val
   65                  70                  75                  80
   Ala Leu Glu Arg Gly Tyr Arg Phe Thr Ala Val Val Asp His His Ala
                   85                  90                  95
   Ala Arg Glu Lys Leu Gly Met Leu Arg Ala Leu Gly Ala Glu Leu Val
               100                 105                 110
   Phe Val Glu Cys Pro Pro Asp Gly Gly Val Ser Ser Val Gln Arg Arg
           115                 120                 125
   Arg Val Ala Ala Arg Ile Ala Ala Glu Thr Gly Ala Tyr His Pro Asp
       130                 135                 140
   Gln His Asn His Pro Gly Asn Gly Asn Gly Tyr Thr Gly Leu Ala Arg
   145                 150                 155                 160
   Glu Leu Val Glu Gln Leu Gly Asp Val Asp Val Leu Val Ala Ala Ile
                   165                 170                 175
   Gly Thr Gly Gly Ser Leu Cys Gly Thr Ala Arg Ala Leu Arg Ala Ala
               180                 185                 190
   Gly Ala Ser Thr His Ala Val Ala Val Glu Pro Val Gly Ser Ile Ile
           195                 200                 205
   Phe Gly Gly Ala Pro Gly Ile Tyr His Gln Thr Gly Ala Gly Ser Pro
       210                 215                 220
   Ala Gly Phe Pro Ile Gly Asp Asn Val Asp Arg Ser Val Ile Gly Glu
   225                 230                 235                 240
   Ala His Arg Val Ser Asp Ser Asp Ala Phe Ala Gly Ala Arg Val Val
                   245                 250                 255
   Ala Arg Arg Thr Gly Val Leu Val Gly Gly Thr Thr Gly Gly Ala Ile
               260                 265                 270
   His Val Ala Leu Arg Arg Leu Tyr Ala Tyr Pro Ala Gly Ser Val Val
           275                 280                 285
   Val Val Leu Cys Asn Asp Ala Gly Glu Lys Tyr Leu Asp Ser Val Tyr
       290                 295                 300
   Asp Asp Glu Trp Leu Arg Ala Arg Gly Val Leu Asp Glu Leu Ala His
   305                 310                 315                 320
   Arg Arg Met Asp Arg Trp Phe Ala Thr Tyr Ala Glu Ser Val Lys Val
                   325                 330                 335
   Ala Thr Arg Asp Arg Ala Val Arg Arg Thr Pro Val Ala Val Gly Gly
               340                 345                 350
<210> 52
   <211> 1059
   <212> DNA
   <213> Amycolatopsis japonicum
<220>
   <223> orf 1664
<400> 52
   atgacgatcg tccggcgcgc gtccgacctg atcgggaaca cgccgctgct ggagctcgcg      60
   cgtaccggca ccgggacgcg tctgctgctc aaactcgacc acctcaaccc caccggctcg     120
   tgcaaggtac gcatggcgag gcagatgatc gacgaggcgg agctggacgg acggctgcgg     180
   ccgggcgggc atatcgtcga acccacgtcc ggcaacaccg ggaacgggct cgccctggtc     240
   gcgctggaac gcggctaccg gttcaccgcc gtggtcgatc accacgccgc ccgcgagaaa     300
   ctcgggatgc tgcgcgcgct cggcgcggaa ctggtgttcg tcgaatgccc gccggacggc     360
   ggggtcagtt cggtccagcg gcggcgggtc gcggcccgca tcgccgcgga aaccggtgcc     420
   taccatcccg atcagcacaa ccatcccggc aacggcaacg ggtacaccgg gctggcgcgg     480
   gaactcgtcg agcagctcgg cgacgtcgac gtgctcgtcg cggcgatcgg caccggcggc     540
   tccctgtgcg gtacggcccg cgccctgcgc gcggcgggag cgagcaccca cgcggtggcg     600
   gtcgaacctg tcggctcgat catcttcggt ggtgcgccgg ggatctacca ccagaccggg     660
   gcgggcagcc cggccggttt cccgatcggg gacaacgtcg accggtcggt gatcggcgaa     720
   gcccaccgcg tttccgacag cgacgcgttc gccggggccc gcgtcgtcgc caggcgcacc     780
   ggcgtgctgg tcggcgggac caccggcggc gcgatccacg tcgcgctgcg gcggctttac     840
   gcgtatccgg ccggaagcgt cgtcgtggtg ctgtgcaacg acgcggggga gaagtacctc     900
   gattcggtgt acgacgacga atggctgcgc gccaggggcg tactcgacga actggcccac     960
   cgccggatgg accgctggtt cgccacctac gccgaatccg tgaaggtcgc cacgcgcgac    1020
   cgggcggtgc gccggacccc ggtggcggtg ggcggatga                           1059
<210> 53
   <211> 460
   <212> PRT
   <213> Amycolatopsis japonicum
<220>
   <223> ORF 1665
<400> 53
   Met Ser Thr Ala Val Thr Thr Arg Gly Tyr Arg Ala Leu Ala Gly Leu
   1               5                   10                  15
   Ala Ala Pro Ile Ala Gly Ile Gln Leu Ala Gln Val Ala Leu Thr Ser
               20                  25                  30
   Val Asp Leu Ala Met Leu Gly Leu Leu Gly Val Thr Ala Val Ala Ala
           35                  40                  45
   Gly Gly Leu Ala Ile Leu Leu Tyr Asn Gln Ile Arg Thr Met Cys Val
       50                  55                  60
   Gly Met Val Thr Gly Val Gly Asn Leu Val Ala Thr Ala Ala Gly Ala
   65                  70                  75                  80
   Gly Glu Leu Arg Thr Gly Thr Gly Glu Leu Asp Glu Arg Ala Arg Asp
                   85                  90                  95
   Glu Ile Arg Ser Leu Leu Arg Ser Ala Leu Leu Val Ala Thr Leu Thr
               100                 105                 110
   Ala Ala Leu Gly Ala Ala Val Leu Cys Gly Leu Ala Val Ala Leu Pro
           115                 120                 125
   Ser Leu Gly Gln Lys Pro Glu Ile Val Ser Leu Ala Gly Pro Met Met
       130                 135                 140
   Phe Ala Leu Ala Gly Gly Leu Phe Pro Met Val Trp Leu Asn Val Val
   145                 150                 155                 160
   Arg Gln Phe Ala Val Gly Leu Arg Arg Pro Gly Ser Leu Leu Ala Val
                   165                 170                 175
   Thr Leu Val Ser Ile Ala Val Asn Ala Ala Leu Asn Ala Ala Phe Ile
               180                 185                 190
   Tyr Gly Trp Ala Gly Leu Pro Glu Leu Gly Val Ala Gly Ile Gly Leu
           195                 200                 205
   Ala Thr Thr Leu Val Gln Phe Phe Thr Leu Ala Val Phe Ala Ser Ala
       210                 215                 220
   Leu Arg Arg Asp Pro Leu Leu Arg Pro Met Val Ser Leu Ala Leu Trp
   225                 230                 235                 240
   Arg Ala Asp Arg Ala Val Val Arg Arg Ile Val Arg His Gly Thr Pro
                   245                 250                 255
   Ile Cys Phe Thr Tyr Gly Ser Glu Ala Ala Ile Thr Ser Ile Ala Thr
               260                 265                 270
   Met Leu Met Gly Ala Phe Gly Pro Ala Met Leu Ala Ala Ser Asn Val
           275                 280                 285
   Ala Asn Gln Leu Ala Tyr Ile Val Tyr Gln Ala Asn Ile Gly Leu Ser
       290                 295                 300
   Gln Gly Ser Ser Ile Leu Val Ser Arg Ala Val Ala His Gly Asp Arg
   305                 310                 315                 320
   Asp Arg Ala Pro Val Ile Ala Arg Gln Ala Phe Thr Leu Ser Trp Ser
                   325                 330                 335
   Leu Met Ala Val Val Ser Leu Ala Tyr Leu Leu Val Pro Gln Val Leu
               340                 345                 350
   Leu Trp Pro Phe Leu His Asp Glu Thr Asp Ala Val Val Leu Gly Thr
           355                 360                 365
   Ala Ser Thr Leu Leu Val Phe Ala Ile Ala Gln Gln Tyr Ser Lys Gly
       370                 375                 380
   Thr Gln Asn Ile Leu Val Gly Leu Leu Arg Gly Leu Gly Asp Thr Val
   385                 390                 395                 400
   Ser Gly Leu Arg Cys Thr Leu Val Gly Tyr Trp Ala Val Gly Val Pro
                   405                 410                 415
   Ala Met Phe Leu Cys Ala Tyr Val Phe Ala Trp Gly Gly Trp Gly Val
               420                 425                 430
   Trp Ala Gly Leu Cys Leu Gly Phe Ala Thr Thr Ala Val Leu Leu Gly
           435                 440                 445
   Arg Ser Phe Ala Gly Gln Gly Ala Gly Ser Ala Arg
       450                 455                 460
<210> 54
   <211> 1383
   <212> DNA
   <213> Amycolatopsis japonicum
<220>
   <223> orf 1665
<400> 54
   atgagcacgg cggtgacgac ccgcggctac cgggccctcg ccgggctggc cgcgccgatc      60
   gcgggaatcc agctggcaca ggtcgcgctc accagcgtcg acctggccat gctcgggctg     120
   ctcggggtga ccgcggtggc ggcgggcggg ctggcgatcc tgctctacaa ccagatccgc     180
   accatgtgcg tcggcatggt caccggcgtc gggaacctcg tggcgacggc ggccggggcc     240
   ggggaactcc gcaccggcac cggcgaactc gacgagcggg cgcgggacga gatccggtcg     300
   ctcctgcggt cggcgctgct cgtcgcgacc ctgaccgcgg cgctcggcgc ggccgtcctt     360
   tgtggactgg ccgtcgcgct gccttcgctc ggccagaaac cggagatcgt ttcgctcgcc     420
   ggtccgatga tgttcgcact ggccggcggg ctgttcccga tggtgtggct gaacgtggtg     480
   cgccagttcg ccgtcgggtt gcggcggccg ggttcgctcc tcgccgtgac gctggtgtcg     540
   atcgcggtca acgccgccct caacgccgcc ttcatctacg gctgggcggg tctgcccgag     600
   ctgggcgtcg ccggtatcgg cctggcgacg accttggtcc agttcttcac gctcgccgtg     660
   ttcgcctcgg ccctccggcg ggacccgctg ttgcgcccca tggtgtcgct cgcgctgtgg     720
   cgcgcggatc gggcggtggt ccggcgcatc gtccggcacg ggacgccgat ctgcttcacc     780
   tacggctcgg aggcggcgat cacctcgatc gcgacgatgc tgatgggtgc gttcggcccg     840
   gcgatgctcg ccgcgtcgaa cgtcgcgaac cagctcgcct acatcgtcta ccaggccaac     900
   atcgggctgt cgcaaggatc gtccattttg gtcagccgcg cggtcgccca cggtgaccgg     960
   gatcgcgcac cggtgatcgc gcgccaggcg ttcaccctct cctggtcctt gatggcggtg    1020
   gtgagcctgg cgtacctgct ggtgccgcag gtgctgctgt ggccgttcct gcacgacgag    1080
   accgacgcgg tggtgctcgg cacggcgtcg accctgctgg tgttcgccat cgcgcagcag    1140
   tacagcaagg gaacgcagaa catcctcgtc ggcctgctgc gcgggctcgg tgacaccgtc    1200
   tccggcctcc gctgcacgct cgtggggtac tgggcggtgg gcgtgcccgc gatgttcttg    1260
   tgcgcctacg tgttcgcctg gggcggctgg ggggtctggg ccgggctgtg tctcggcttc    1320
   gcgacgaccg cggtcctgct cggacggagt ttcgccggtc agggagccgg ttctgcgcga    1380
   tag                                                                  1383
<210> 55
   <211> 313
   <212> PRT
   <213> Amycolatopsis japonicum
<220>
   <223> ORF 1666
<400> 55
   Met Thr Thr Thr Gly Arg Ile Gly Ile Ala Asp Val Glu Cys Leu Glu
   1               5                   10                  15
   Asn Ala Val Asp Thr Leu Arg Ala Leu Asp Tyr Arg Gln Gly Gly Ala
               20                  25                  30
   Phe Ser His Asp Ala Val His Val Val Lys Ser Val Ser Leu Leu Leu
           35                  40                  45
   Thr Trp Gly Ala Val Pro Gly Gly Leu Arg Ser Arg Leu Leu Thr Val
       50                  55                  60
   Leu Ala Asp Leu His Asn Leu Leu Gly Trp Thr Glu Phe Asp Ile Gly
   65                  70                  75                  80
   Arg Arg Val Pro Ala Arg Leu Arg Phe Asp Arg Ala Leu Gly Leu Ala
                   85                  90                  95
   Ala Ala Ala Gly Asn Asp Asn Leu Met Ala Asn Ile Cys Tyr Arg Leu
               100                 105                 110
   Gly Arg Leu Ala Leu His His Asp Asp Val Glu Gly Gly Leu Asp His
           115                 120                 125
   Leu Arg Gln Gly Gln Ala Ala Ala His Arg Ser Gly Ser Leu Arg Ala
       130                 135                 140
   Gln Ala Ile Leu Thr Ile Asn Glu Ala Trp Ala Leu Ala Lys Ala Gly
   145                 150                 155                 160
   Asp Glu Arg Ala Ala Leu Thr Ser Leu Ala Arg Ala Glu Cys Glu Phe
                   165                 170                 175
   Glu Asn Ala Gly Thr Trp Ala Ala Pro Arg Pro Trp Glu Ala Phe Phe
               180                 185                 190
   Gly Ala Thr Asp Met Ala Ala Met Arg Gly Thr Val Leu Thr Glu Leu
           195                 200                 205
   Ala Arg Thr Val Ala Ser Arg His Ser Glu Glu Ala Ile Glu Arg Leu
       210                 215                 220
   Arg Asp Ala Val Asp Gly Tyr Gly Ala Glu Met Ser Arg Ser Leu Ala
   225                 230                 235                 240
   Leu Thr Leu Ile Met Leu Ala Gln Asn His Ala Leu Gln Gly Asp Phe
                   245                 250                 255
   Gly Glu Ala Val Arg Thr Gly Asp Glu Ala Leu Asp Leu Ala Arg Gly
               260                 265                 270
   Leu Gly Ser Thr Arg Thr Lys Asp Arg Leu Ala Pro Leu Ala Ala Leu
           275                 280                 285
   Leu Arg Gly Asp Ala Asp Pro Ala Ser Arg Asp Leu Leu Ala Arg Ile
       290                 295                 300
   Asp Arg Tyr Arg Ala Glu Pro Ala Pro
                       305                 310
<210> 56
   <211> 942
   <212> DNA
   <213> Amycolatopsis japonicum
<220>
   <223> orf 1666
<400> 56
   atgacaacga ccggccgtat cggtatcgcc gacgtcgaat gtctcgaaaa cgcggtcgat      60
   actctgcgcg cgttggacta tcggcagggc ggcgcctttt cccacgacgc ggtgcacgtc     120
   gtgaagtcgg tcagcctgct gctcacttgg ggcgccgtcc ccggcggact ccggtcacgg     180
   ttgctgaccg tgctcgcgga tctgcacaat ctgctgggct ggacggaatt cgacatcggc     240
   cgccgcgttc cggcacggct ccggttcgac cgcgcgctcg gtctcgccgc cgcggcgggc     300
   aacgacaatc tcatggccaa catctgttac cgcctcggca ggctggcgtt gcatcatgac     360
   gacgtcgaag gcgggttgga ccatctccgg caaggccaag ccgccgccca ccgctccggt     420
   tccctgcggg cccaggcgat cctgacgatc aacgaggcgt gggcactggc caaggccggt     480
   gacgagcgcg cggccctcac gagcctggcg cgggcggagt gcgagttcga gaacgcgggc     540
   acctgggcgg cgccacgacc gtgggaagcc ttcttcggcg ccacggacat ggccgcgatg     600
   cgcgggaccg tcctgaccga attggccagg acggtcgcct cccggcacag cgaagaagcg     660
   atcgagcggt tacgcgacgc cgtcgacggc tacggcgccg agatgtcccg cagcctcgcg     720
   ctcaccttga tcatgctcgc gcagaaccac gcgctccagg gcgacttcgg cgaagccgtc     780
   cgcaccggcg acgaggcgct ggacctcgcg cggggcctcg gttccacccg caccaaggac     840
   cggctcgcac cgctggcggc acttctgcgc ggagacgccg atcccgcctc ccgcgacctg     900
   ctcgcccgga tcgaccgcta tcgcgcagaa ccggctccct ga                        942
<210> 57
   <211> 140
   <212> PRT
   <213> Amycolatopsis japonicum
<220>
   <223> ORF 1667
<400> 57
   Met Ser Asp Phe Glu Ala Gln Leu Arg Ala Val Ser Leu Arg Val Thr
   1               5                   10                  15
   Arg Pro Arg Leu Ala Val Leu Ala Ala Leu Arg Asp His Pro His Val
               20                  25                  30
   Asp Thr Glu Thr Val Ile Ala Leu Val Arg Ala Asp Leu Pro Thr Val
           35                  40                  45
   Ser His Gln Ala Val Tyr Asp Val Leu Arg Ala Leu Thr Glu Thr Gly
       50                  55                  60
   Leu Ile Arg Arg Ile Gln Pro Ala Gly Ala Leu Ala Arg Tyr Glu Thr
   65                  70                  75                  80
   Arg Val Gly Asp Asn His His His Val Val Cys Arg Ser Cys Gly Ala
                   85                  90                  95
   Ile Ala Asp Val Asp Cys Ala Val Gly His Ala Pro Cys Leu Thr Ala
               100                 105                 110
   Ser Gly Asp His Gly Phe Val Ile Asp Glu Ala Glu Val Val Tyr Trp
           115                 120                 125
   Gly Leu Cys Pro Gly Cys Ala Ala Glu Pro Val Gln
       130                 135                 140
<210> 58
   <211> 423
   <212> DNA
   <213> Amycolatopsis japonicum
<220>
   <223> orf 1667
<400> 58
   atgtccgact tcgaggcaca gctgcgagcg gtttcgttgc gcgtgacgcg gcctcggctg      60
   gccgtgctcg cggcgctgcg cgatcatccg cacgtcgaca ccgaaacggt gatcgcgctg     120
   gtgcgggccg atctgccgac ggtctcgcac caggcggtgt acgacgtgct gcgggcgctc     180
   accgaaaccg gtctgatccg gcggatccag cccgccggcg cgctcgcccg ttacgagacc     240
   cgggtggggg acaaccatca ccatgtcgtg tgccgttcct gcggtgcgat cgcggacgtc     300
   gattgcgccg tcggccatgc cccctgtctc accgcttcgg gcgatcacgg gttcgtgatc     360
   gacgaggcgg aggtcgtcta ctggggcctg tgccccggct gtgcggccga acctgtccag     420
   tga                                                                   423
<210> 59
   <211> 744
   <212> PRT
   <213> Amycolatopsis japonicum
<220>
   <223> ORF 1668
<400> 59
   Met Ser Asp Asn Pro Glu Lys Gly Cys Pro Val Ala His Asp Ser Val
   1               5                   10                  15
   Thr Ala His Gly Ser Glu Ser Glu Asn Pro Ala Ile Asp Ser Pro Thr
               20                  25                  30
   Pro Lys Ser Gly Gly Arg Pro Arg Thr Asn Lys Asp Trp Trp Pro Asn
           35                  40                  45
   Gln Leu Asp Leu Ser Val Leu His Ala His Ser Ser Lys Ser Asn Pro
       50                  55                  60
   Leu Gly Ala Asp Phe Ser Tyr Ala Lys Glu Phe Ala Lys Leu Asp Val
   65                  70                  75                  80
   Glu Ala Leu Lys Arg Asp Ile Thr Glu Val Leu Thr Thr Ser Gln Asp
                   85                  90                  95
   Trp Trp Pro Ala Asp Phe Gly His Tyr Gly Gly Leu Met Ile Arg Met
               100                 105                 110
   Ser Trp His Ala Ala Gly Thr Tyr Arg Ile His Asp Gly Arg Gly Gly
           115                 120                 125
   Ala Gly Asp Gly Ala Gln Arg Phe Ala Pro Leu Asn Ser Trp Pro Asp
       130                 135                 140
   Asn Ala Asn Leu Asp Lys Ala Arg Arg Leu Leu Trp Pro Val Lys Glu
   145                 150                 155                 160
   Lys Tyr Gly Gln Gln Ile Ser Trp Ala Asp Leu Leu Val Leu Ala Gly
                   165                 170                 175
   Asn Val Ala Leu Glu Ser Met Gly Phe Lys Thr Phe Gly Phe Gly Phe
               180                 185                 190
   Gly Arg Val Asp Thr Trp Glu Pro Glu Glu Ile Phe Trp Gly Pro Glu
           195                 200                 205
   Asp Thr Trp Leu Gly Asp Glu Arg Tyr Ala Ser Asp Thr Glu Met Val
       210                 215                 220
   Pro Asp Val Gly Ala Thr Glu Met Gly Leu Ile Tyr Val Asn Pro Glu
   225                 230                 235                 240
   Gly Pro Arg Gly Asn Ala Asp Pro Ala Ala Ala Ala His Phe Ile Arg
                   245                 250                 255
   Glu Thr Phe Ala Arg Met Ala Met Asn Asp Glu Glu Thr Val Ala Leu
               260                 265                 270
   Ile Ala Gly Gly His Thr Phe Gly Lys Thr His Gly Ala Gly Ile Ala
           275                 280                 285
   Asp Asp His Val Gly Pro Glu Pro Glu Ala Ala Pro Leu Glu Thr Gln
       290                 295                 300
   Gly Leu Gly Trp Leu Ser Thr His Gly Ser Gly Lys Gly Ala Asp Ala
   305                 310                 315                 320
   Ile Thr Ser Gly Leu Glu Val Thr Trp Thr Asp Lys Pro Thr Gln Trp
                   325                 330                 335
   Ser Asn Arg Phe Phe Glu Ile Leu Phe Gly Tyr Glu Trp Glu Leu Thr
               340                 345                 350
   Thr Ser Pro Gly Gly Ala Lys Gln Tyr Val Ala Lys Asp Ala Glu Ala
           355                 360                 365
   Ile Ile Pro Asp Ala His Asp Pro Ala Lys Lys His Lys Pro Thr Met
       370                 375                 380
   Leu Thr Thr Asp Leu Ala Leu Arg Val Asp Pro Glu Tyr Glu Lys Ile
   385                 390                 395                 400
   Ser Arg Arg Phe Leu Glu Asn Pro Asp Asp Phe Ala Leu Ala Phe Ala
                   405                 410                 415
   Lys Ala Trp Tyr Lys Leu Leu His Arg Asp Met Gly Pro Val Ser Arg
               420                 425                 430
   Phe Leu Gly Pro Trp Val Pro Glu Pro Gln Leu Trp Gln Asp Pro Val
           435                 440                 445
   Pro Asp Val Asp His Glu Leu Val Gly Asp Ala Asp Ile Ala Ala Leu
       450                 455                 460
   Lys Thr Lys Val Leu Glu Ser Gly Leu Thr Val Glu Gln Leu Val Gly
   465                 470                 475                 480
   Thr Ala Trp Ala Ser Ala Ala Ser Phe Arg Ser Thr Asp Lys Arg Gly
                   485                 490                 495
   Gly Ala Asn Gly Ala Arg Ile Arg Leu Ala Pro Gln Arg Asp Trp Glu
               500                 505                 510
   Val Asn Arg Pro Glu Glu Leu Ala Ser Val Leu Glu Thr Leu Glu Gly
           515                 520                 525
   Ile Gln Arg Glu Phe Asn Asp Ala Gly Gly Ala Lys Ile Ser Leu Ala
       530                 535                 540
   Asp Leu Ile Val Leu Ala Gly Thr Ala Ala Val Glu Lys Ala Ala Arg
   545                 550                 555                 560
   Asp Ala Gly Thr Asp Val Thr Val Ala Phe His Pro Gly Arg Thr Asp
                   565                 570                 575
   Ala Thr Gln Glu Asp Thr Asp Val Glu Ser Phe Thr Leu Leu Glu Pro
               580                 585                 590
   Arg Ala Asp Gly Phe Arg Asn His Leu Arg Pro Glu Glu Lys Leu Gln
           595                 600                 605
   Pro Glu Val Leu Leu Val Glu Arg Ala Tyr Met Leu Asp Leu Thr Ala
       610                 615                 620
   Pro Glu Met Thr Val Leu Val Gly Gly Leu Arg Ala Leu Gly Ile Thr
   625                 630                 635                 640
   Ala Gly Asp Thr Arg His Gly Val Leu Thr Asp Arg Pro Gly Val Leu
                   645                 650                 655
   Thr Asn Asp Phe Phe Thr Asn Leu Leu Ser Pro Gly Thr Arg Trp Lys
               660                 665                 670
   Val Ser Glu Ser Glu Glu Asn Val Tyr Glu Ile Arg Asp Ala Gly Thr
           675                 680                 685
   Asp Ala Val Lys Trp Thr Ala Thr Pro Val Asp Leu Val Phe Gly Ser
       690                 695                 700
   Asn Ser Gln Leu Arg Ala Leu Ser Glu Val Tyr Ala Gly Gln Ala Gly
   705                 710                 715                 720
   Arg Glu Arg Phe Ala Ala Asp Phe Ala Lys Ala Trp Thr Lys Val Met
                   725                 730                 735
   Glu Leu Asp Arg Phe Asp Leu Asp
               740
<210> 60
   <211> 2235
   <212> DNA
   <213> Amycolatopsis japonicum
<220>
   <223> orf 1668
<400> 60
   atgagtgaca acccggagaa gggctgcccg gtggcccacg actccgtgac cgcgcacgga      60
   agtgagagcg agaacccggc gatcgactcg ccgacgccga aatcgggcgg ccgcccgcgt     120
   accaacaagg actggtggcc caaccagctc gacctctcgg tgctgcacgc ccactcgtcg     180
   aagtcgaacc cgctcggcgc ggacttcagc tacgccaagg aattcgccaa actcgacgtc     240
   gaggccctca aacgcgacat caccgaggtg ctcaccacct cgcaggactg gtggcccgca     300
   gacttcggcc actacggcgg cctgatgatc cggatgagct ggcacgccgc gggcacctac     360
   cgcatccacg acggccgagg cggcgccggt gacggcgcgc agcggttcgc cccgctcaac     420
   agctggcccg acaacgccaa cctcgacaag gcacggcggc tgctgtggcc ggtcaaggag     480
   aagtacggcc agcagatctc gtgggccgac ctgctcgtgc tcgccgggaa cgtcgcgctg     540
   gagtcgatgg ggttcaagac gttcggcttc ggtttcggcc gcgtggacac ctgggagccc     600
   gaggagatct tctggggtcc ggaagacacc tggctgggtg acgagcgcta cgcgagcgac     660
   accgagatgg tccccgacgt cggcgcgacc gaaatgggcc tcatctacgt caaccccgag     720
   ggaccccgag gcaacgcgga cccggccgcc gcggcgcatt tcatccggga gaccttcgcc     780
   cggatggcga tgaacgacga ggagaccgtc gcgctcatcg ccgggggcca caccttcggc     840
   aagacccacg gtgccggtat cgccgacgac cacgtcggcc cggaacccga agccgccccg     900
   ctcgagacac agggcctcgg ctggctgagc acccacggca gcggcaaggg cgccgacgcg     960
   atcaccagcg gtctcgaggt gacgtggacc gacaagccga cgcagtggag caaccggttc    1020
   ttcgagatcc tcttcggcta cgaatgggaa ctcaccacca gcccgggcgg cgccaagcag    1080
   tacgtcgcca aggacgccga ggcgatcatc ccggacgccc acgacccggc caagaagcac    1140
   aagccgacca tgctcaccac ggatctcgcg ctgcgcgtcg acccggagta cgagaagatc    1200
   tcgcgccgct tcctggagaa cccggacgac ttcgcgctcg cgttcgccaa ggcctggtac    1260
   aaactgctgc accgcgacat gggcccggtc agccgtttcc tggggccgtg ggtgcccgaa    1320
   ccgcagctgt ggcaggaccc ggtgccggac gtcgaccacg aactcgtggg ggacgccgac    1380
   atcgccgcgc tcaagacgaa ggtgctcgag tccgggctca cggtcgagca gctggtcggc    1440
   acggcgtggg cgtccgcggc gagcttccgg tccaccgaca aacgcggcgg cgccaacggg    1500
   gcccggatcc gcctggcacc ccagcgtgac tgggaggtca accggccgga agagctcgcg    1560
   agtgtgctgg agaccctgga gggcatccag cgcgagttca acgacgccgg tggcgccaag    1620
   atctcgctgg ccgacctgat cgtgctggcg ggcactgccg ccgtcgagaa ggcggcgcgc    1680
   gacgcgggca ccgacgtgac cgtggcgttc caccccggcc gcaccgacgc cacccaggag    1740
   gacaccgacg tcgagtcgtt cacgctgctc gaaccgcggg ccgacgggtt ccgcaaccac    1800
   ctgcgtcccg aggagaaact gcagccggag gtcctgctgg tcgagcgcgc ctacatgctc    1860
   gacctgaccg cgcccgagat gaccgtcctc gtcggcggcc tgcgtgcgct cgggatcacc    1920
   gccggcgaca cccggcacgg cgtcctcacc gaccggcccg gcgtgctcac caacgacttc    1980
   ttcaccaacc tcctctcgcc gggcacccgg tggaaggtgt cggagtccga ggagaacgtg    2040
   tacgagatcc gcgacgccgg cacggacgcg gtgaagtgga cggcgacccc ggtcgacctc    2100
   gtgttcggct ccaactcgca gctgagggcg ctttccgagg tctatgcggg tcaggccggg    2160
   cgggagcggt tcgccgccga cttcgccaag gcgtggacca aggtcatgga actggaccgg    2220
   ttcgacctcg actga                                                     2235
<210> 61
   <211> 137
   <212> PRT
   <213> Amycolatopsis japonicum
<220>
   <223> ORF 5768
<400> 61
   Met Ser Pro Thr Thr Ala Asn Ser Ser Ala Pro Val Pro Gly Arg Arg
   1               5                   10                  15
   Ser Thr Lys Gln Arg Ala Ala Val Val Glu Leu Leu Lys Glu Ile Asp
               20                  25                  30
   Asp Phe Arg Ser Ala Gln Glu Leu His Asp Glu Leu Arg Lys Arg Gly
           35                  40                  45
   Asp Gly Ile Gly Leu Thr Thr Val Tyr Arg Thr Leu Gln Ser Leu Ser
       50                  55                  60
   Glu Ala Gly Glu Ile Asp Val Leu Arg Thr Asp Thr Gly Glu Ala Ile
   65                  70                  75                  80
   Tyr Arg Arg Cys Ser Ser His His His His His Leu Val Cys Arg Leu
                   85                  90                  95
   Cys Gly Ser Thr Val Glu Val Glu Gly Pro Ala Val Glu Arg Trp Ala
               100                 105                 110
   Glu Lys Ile Ala Ser Glu His Gly Phe Ser Asp Ile Ser His Thr Val
                 115                 120                 125
   Glu Ile Val Gly Thr Cys Ser Asn His
       130                 135
<210> 62
   <211> 414
   <212> DNA
   <213> Amycolatopsis japonicum
<220>
   <223> orf 5768
<400> 62
   atgagtccga cgacggccaa cagttccgcg ccggtgccgg gacgccggtc gacgaaacag      60
   cgggccgccg tggtcgagct gctcaaggag atcgacgact tccgttctgc ccaggaactg     120
   cacgacgagc tgcgcaaacg cggcgacggc atcgggctca ccacggtgta ccggacgctg     180
   cagtcgctgt ccgaggccgg cgagatcgac gtcctgcgca cggacaccgg cgaggccatc     240
   taccggcgtt gctcgtcgca ccaccaccat cacctggtgt gccgtctctg cggcagcacg     300
   gtcgaggtcg agggccccgc ggtggagcgg tgggccgaga agatcgcttc ggagcacggc     360
   ttctccgaca tcagccacac cgtggagatc gtcgggacct gctcgaacca ctga           414
<210> 63
   <211> 1869
   <212> DNA
   <213> Amycolatopsis japonicum
<220>
   <223> ds orf 5768
<400> 63
   aatcatatgg tcgggacctg ctcgaaccac tgacgagcgc tatgaaaggt cctttcctgg      60
   caaattttgc aaggaaagga cctttcatag catcggctca gtactcgtaa gggtccttcg     120
   gcgccggcac ctgccgcacc gaggtgagcg tcaaatccgg catgtagctg ttctcccgta     180
   ccgcggtccc cggcacgacc gtcccggtga cctccagcca ggtgtcgttc ccgaagcccg     240
   acacgccgtc gcccaccatc cgcacggtga tcgggaaggc gtcggccgcg cagcagccga     300
   tgaccatccg cgccagcatc gtgttgccct cgttgtgcac gacgaagccg gacagccgca     360
   ccgtgcgccc gttcaacgag ccactggaat cccatcccgc gcggctgacg aactcgttga     420
   cctccagcgg gaccacgtcc ccggcgggca agggcgggaa ggcggccgcg ttgctcgccg     480
   aagcgctctg tggcgccctg gcctcggtgc gggtcaccga atcggcgccc agcgcgggcg     540
   gcgcgaccag gaacacggcc agcacgggca ccatcagcag ccaggccgag cgggtgttgt     600
   ggtcgtgcgc gtggacatcg ccggacacga cggcggcggc ccgcgcggcc agcaggtcgc     660
   ggacgatcgc gaccgcgccc agcgccacca tcaccgcgcc gcccgcgatg atccacggct     720
   gctgggcggg tttgacgtag cgcaggtagt cgccgttgat cgtgatcttc agcagcgcgc 780
   cgccgagcag gatcagcagg atgttctggg tctcgcgttt cacgcgccac ctccgaggat 840
   caacacaccc gacacgacgg cgcacgacag cgcgaccacg aacgtcaccg gcgcgaaccg 900
   gagcgcgaag gacttgccaa aggtgccggt ctgcagggcg aacagcttca cgtcgatcgc 960
   gggcccgacc accaggaaca ccagtttcgg cagcagcggc atcgcggtca gcgacgcggc 1020
   gacgaaggcg tcggcctcac tgcacagggc cagcacgacg gcgagcacgg ccatcaccag 1080
   caccccgagc acgatctgct cccccagcac tccgaaccac ttggcgggca ccagcacgtt 1140
   catcgcggcg gcgatcatcg cgccgagcac caggaaaccg ccggcctcga ccagatccgc 1200
   ccgcgcggtc tcggcgaagg tcttccagcg cgaaccgtgc tgaacctccg gaagccgccg 1260
   caaagcccgc tccgcgatcc agtcgagctt gccccacttc gcccacagcc agcccatcac 1320
   catcgcggtg gcgagcgaac cggcgaagcg ggcgaacacc atttccgggt tcccggggaa 1380
   cgccaccgcc gtcgcgacca gcaccaccgg gttcaccgcg ggcgcggcga gcaggaaggt 1440
   cagcgcggcc gcgggcgcga cgccctggcc gatcaggcgc cgcgcgaccg gcaccgacgc 1500
   gcattcacac ccggcagggc gaccccggcc agccccgcga cgccgacggc cgcaccggcc 1560
   cggcgaggga gcaccttctc cagcacccgc gcgggcacga acgccgcgat cgcgccgctg 1620
   atcagcaccc cgagcaccag gaacggcagc gcctgcacgc agacggcgac gaacacggtc 1680
   gagccggtcc gcagcgcggg cacgtcgaac acctgctgga gccagctctg cccgaggatc 1740
   gcgatcagca ggatcgcgca gagcacctcg atcgaggtga tcttgaaccg ccgggccggc 1800
   ttcttctccg gggcgcttct ggaaatggtt tccatgcgcg cgatgatgcc aggtgaccgt 1860
   tctagatta 1869
<210> 64
   <211> 1537
   <212> DNA
   <213> Amycolatopsis japonicum
<220>
   <223> us orf 5768
<400> 64
   aataagcttg tttcgcgacg aagttcttcg ggatcgagca gctcacgccg ccgagcacga      60
   gcgtcgtgcg ggcctcgcgc agatccttgc ccgcgtcgtc gcgcacgtgc gtgacgatgt     120
   ccaccttcca gcccgccacc gcggtcacgt cgccgttgcg ccagccgtcc tgccggatca     180
   gctggtcgaa ggcggtcacg cgggacacgg tcaggtcgga ggtggtctcg tccttgccga     240
   tgttctcgtt cttgatgacc gacggcgggc cgagcttgag ccccttgacg tcgacgcccg     300
   aagcgccgga cggcttgagc gcgccggttt cgtcccgcac ccagatcttg cccacgctcg     360
   tcgacgacgt cgccttgctg ggcgacgagc agtcgacggc gctcttggcg ccttcaagca     420
   ccagtacggt gttgtcggcg ggcttcgcgc ctccgcgccc gttgtcgagc agggcgtagt     480
   tgctctccgc gaccgccgtc gggaccgtgg tctgctgcag cgcggcgagc acgttgtggt     540
   cgcggctggc gataccgggc gggaacgggc tctgggcgcc gaagcggccc aggttgacag     600
   cccatttgtc gccgaaggtg ttcttctcgc cgtcgtcacg cgggacgacg gtgcggtccg     660
   gggagagttt cgactggcct ggcgtcagcg gcgagcgctg ggtctcggtg atcagcgatc     720
   ccgggttccc gatgcgcacc gacgccacat tcgcgaacga cacggggccg gtcttctcat     780
   cgacggcctg cgtcgtctgc gccgaggcga gccccggaac cgacgccaaa gcgacggccg     840
   ccgtcgcggc ggccagcttc gtcacgaaac gagtcatgtc tccccactgc ctctccgggc     900
   cctcagagcg cggtcgtcag cgtacgagaa gcgacgaaac gctccgcgac aaccttgcac     960
   cgttggcgac cacatgcgaa gtggaatggt catttctttg ctaagtcacc cgaatggagc    1020
   agtaacatcg ccgccaagtg gtcgcccggt cggcgaacag accaccgggg gcgggaacac    1080
   taggatgggc aggactgtta ctggcaatga cttcggaaat catgatggac atggaggcgg    1140
   cgatggctac ggtgaccccg gactctgccg cccccgggct ctcggaggac cccgggccgc    1200
   attcgtcggc ccgtcccccc gccgaacccg cgacgtcggc ctcgcccgcc gtcctcgcgg    1260
   acgccggcga cctgctcagg gcgctcgccg cgcccgtgcg catcgccatc gtcctgcagt    1320
   tgcgtaacgc ggacaggtgc gtgcacgaac tggtggacac actcgatgtc gcgcagccgc    1380
   tgatcagcca gcacctgcgg gtgctgaaga cggcgggtgt cgtacagggt gagcgacgcg    1440
   gccgtgaggt ggtgtaccgg ctggtcgacg atcaccttgc gcatatcgtg gtggacgccg    1500
   tagcccacgt tcaggagggg aagtgagcca tatgatt                             1537

## Beschreibung

### Anwendungsgebiet und Stand der Technik

Die vorliegende Erfindung betrifft isolierte Nukleinsäuren und Proteine bzw. Peptide für die Biosynthese von [S,S]-Ethylendiamindisuccinat ([S,S]-EDDS), Expressions- und Deletionsvektoren, Wirtszellen und Deletionsmutanten der Gattung *Amycolatopsis japonicum,* Verfahren sowie ein Kit für die Biosynthese von [S,S]-EDDS.

Ethylendiamindisuccinat (EDDS, auch als Ethylendiamindibernsteinsäure bezeichnet) ist ein sechszähniger Chelatkomplexbildner. EDDS besitzt zwei Stereozentren und tritt entsprechend in drei verschiedenen Stereoisomeren auf, nämlich [R,R]-EDDS, [S,S]-EDDS und [R,S]-(*meso*)-EDDS. Es hat sich herausgestellt, dass ausschließlich das Stereoisomer [S,S]-EDDS vollständig biologisch abgebaut werden kann (Schowanek et al., Chemosphere (1997), 34(11): 2375-91).

Bei EDDS handelt es sich zudem um ein Strukturisomer von Ethylendiamintetraacetat (EDTA), ein weit verbreiteter ebenfalls sechszähniger Chelatkomplexbildner. Beide Verbindungen sind sich in ihren chemischen Eigenschaften, insbesondere hinsichtlich ihrer Fähigkeit zur Komplexbildung von Metallionen, sehr ähnlich. So weisen EDDS und EDTA für eine ganze Reihe von Metallionen vergleichbare Komplexbildungskonstanten auf.

EDTA wird bereits seit vielen Jahrzehnten wegen seiner ausgeprägten Fähigkeit zur Komplexbildung in verschiedensten Bereichen zur Entfernung von Metallionen eingesetzt und ist heute der am häufigsten verwendete Komplexbildner. Besonders stabile Komplexe bildet es mit Kupfer(II)-, Nickel(II)-, Eisen(III)- und Cobalt(II)-Ionen, aber auch mit Schwermetallionen sowie mit Calcium- und Magnesiumionen.

EDTA wird daher insbesondere als Wasserenthärter Detergenzien zugesetzt, dient der Stabilisierung von Bleichbädern im Bereich der Papier- und Textilindustrie und wird in Form seiner Eisen-, Kupfer- und Zink-Komplexe als Düngemittel verwendet. Zudem kommt EDTA im Bereich der Medizin zur Behandlung von Schwermetallvergiftungen zum Einsatz.

Nachteilig ist, dass EDTA biologisch nicht abbaubar ist und daher mittlerweile ubiquitär in Gewässern nachgewiesen werden kann. Als ökologisch bedenklich wird es insbesondere deshalb eingestuft, weil es Schwermetalle aus Sedimenten lösen und auf diese Weise bioverfügbar machen kann.

Vor diesem Hintergrund ist es wünschenswert, EDTA im Sinne einer nachhaltigen Stoffpolitik durch gleichwertige, allerdings biologisch abbaubare Verbindungen zu ersetzen.

EDDS in Form des biologisch abbaubaren Stereoisomers [S,S]-EDDS stellt einen aufgrund der zu EDTA vergleichbaren Komplexbildungskonstanten grundsätzlich geeigneten Ersatzstoff dar.

Die chemische Synthese von [S,S]-EDDS ausgehend von L-Asparaginsäure und 1,2-Dibromethan in Gegenwart von dreiwertigem Cobalt ist bekannt (Neal and Rose, Inorganic Chemistry (1968), 7(11): 2405-12). Nachteilig ist der hierbei als toxisches Nebenprodukt auftretende Bromwasserstoff, der aufwendig entfernt werden muss. Zudem erfolgt die Synthese unter Verwendung fossiler Edukte.

Daneben ist ein nicht enantioselektives chemisches Verfahren bekannt, bei dem Maleinsäure oder Maleinsäureanhydrid mit Ethylendiamin umgesetzt wird, wobei neben 50 % *meso*-EDDS [R,R]-EDDS und [S,S]-EDDS als racemisches Gemisch erhalten werden. Aufgrund der nur geringen Ausbeute von [S,S]-EDDS und der grundsätzlich sehr aufwendigen Racemattrennung ist dieses Verfahren jedoch für eine industrielle Anwendung generell ungeeignet.

Ein biokatalysiertes Verfahren zur Herstellung von [S,S]-EDDS ist aus der EP 0 731 171 A2 bekannt. Mittels des dort beschriebenen Verfahrens kann [S,S]-EDDS ausgehend von Fumarsäure und Ethylendiamin unter Einwirkung von Mikroorganismen mit Lyaseaktivität mit einer optischen Reinheit von bis zu 97% erhalten werden. Ein weiteres biokatalysiertes Verfahren zur Herstellung von [S,S]-EDDS ist aus der EP 1 043 400 A1 bekannt, bei dem [S,S]-EDDS ausgehend von Maleinsäure und Ethylendiamin in Gegenwart von Mikroorganismen mit Malatisomeraseaktivität und Metallionen mit einer optischen Reinheit von bis zu 98% gewonnen werden kann. Beide biokatalysierte Verfahren beruhen allerdings auf dem Einsatz von synthetischen Vorstufen, die von den verwendeten Mikroorganismen selbst nicht bereitgestellt werden können.

Weiterhin bekannt ist die Biosynthese von [S,S]-EDDS mittels der Bakterienart *Amycolatopsis japonicum* (Zwicker et al., Journal of Industrial Microbiology & Biotechnology (1997); 19(4): 280-285). Nachteilig hierbei ist jedoch, dass die Biosynthese einer Zink-Abhängigkeit unterliegt und bereits eine 2 µM Zink-Konzentration im Kulturmedium eine annähernd vollständige Unterbrechung der [S,S]-EDDS Synthese bewirken kann (Cebulla I., Dissertation (1995), Universität Tübingen).

Weiterhin ist die Entwicklung eines Genklonierungssystems für Amycolatopsis japonicum bekannt (Stegmann et al.: "Development of three different gene cloning systems for genetic investigation of the new species Amycolatopsis japonicum MG417-CF17, the ethylenediaminedisuccinic acid producer", Journal of Biotechnology 92 (2001) 195-204).

Ferner ist bekannt, dass es sich bei dem Aktinomyzet-Stamm MG417-CF17 um Amycolatopsis japonicum handelt und dass dieser Stamm in der Lage ist, [S,S]-EDDS zu produzieren (Goodfellow et al.: "Amycolatopsis japonicum sp. nov., an Ac-tinomycete Producing (S,S)-N,N'-Ethylenediaminedisuccinic Acid", System. Appl. Microbiol. 20, 78-84 (1997)).

Ferner ist die Herstellung von [S,S]-EDDS ausgehend von Ethylendiamin und Fumarsäure unter Verwendung diverser Bakterienstämme bekannt (Takahashi et al.: "Production of (S,S)-Ethylenediamine-N,N'-disuccinic acid from Ethylenediamine and Fumaric Acid by Bacteria", Biosci. Biotechnol. Biochem., 63 (7), 1269-1273, 1999).

Ferner ist die Entwicklung eines Zytoscape-Plugin zur Analyse von Hochdurchsatz-Sequenzierungsdaten mikrobieller Genome bekannt (Tang et al.: "ContigScape: a Cytoscape plugin facilitating microbial genome gap closing", BMC Genomics 2013, 14:289).

Ferner ist die Sequenz eines Eisenaufnahmeregulatorproteins bekannt (EBI accession no. UNIPROT: R4SX04).

Ein Verfahren unter Zink-freien Reaktionsbedingungen zur Herstellung von [S,S]-EDDS mittels *Amycolatopsis japonicum* unter Verwendung eines optimierten Kulturmediums ist in der WO 96/36725 A1 beschrieben.

Problematisch bei den gattungsgemäßen Biosyntheseverfahren für [S,S]-EDDS ist insbesondere die Tatsache, dass sich eine Anwendung der Synthesen in größerem Maßstab aufgrund der Zink-Abhängigkeit und der damit verbundenen niedrigen Ausbeuten als schwierig bzw. unrentabel erweist. So ist es mit erheblichen Kosten verbunden bzw. nahezu unmöglich, eine Zink-freie Umgebung in Kulturmedien sowie Fermentern zu erzeugen.

### Aufgabe und Lösung

Vor diesem Hintergrund ist es daher eine Aufgabe der Erfindung, Proteine bzw. Peptide, Nukleinsäuren, Gen-Cluster, Vektoren, Wirtszellen, Bakterienzellen sowie Verfahren und ein Kit für die Biosynthese von [S,S]-EDDS bereitzustellen.

Diese Aufgabe wird gelöst durch ein Protein oder Peptid gemäß Anspruch 1, durch eine Nukleinsäure gemäß Anspruch 2, durch ein Gen-Cluster mit den Merkmalen des Anspruchs 4, durch die Verwendung eines Proteins oder Peptids gemäß Anspruch 5, durch einen Vektor gemäß Anspruch 6, durch eine Wirtszelle mit den Merkmalen des Anspruchs 7, durch ein Verfahren mit den Merkmalen des Anspruchs 8 sowie durch ein Kit mit den Merkmalen des Anspruchs 9. Bevorzugte Ausführungsformen sind in den abhängigen Ansprüchen beschrieben. Der Wortlaut sämtlicher Ansprüche wird hiermit durch ausdrückliche Bezugnahme zum Inhalt dieser Beschreibung gemacht.

Den Erfindern ist es erstmalig gelungen, am Beispiel von *Amycolatopsis japonicum* Gene und Proteine zu identifizieren und bereitzustellen, die für die Biosynthese von [S,S]-Ethylendiamindisuccinat, nachfolgend als [S,S]-EDDS bezeichnet, verantwortlich sind.

Darüber hinaus ist es den Erfindern erstmalig gelungen, den Mechanismus der dieser Biosynthese zugrundeliegenden Zink-Abhängigkeit aufzuklären.

Hierdurch ist mit besonderem Vorteil eine gegenüber gattungsgemäßen Verfahren wirtschaftlichere und effizientere Herstellung von [S,S]-EDDS, insbesondere in größeren Ausbeuten und in größerer Reinheit, möglich. Die im Folgenden beschriebenen Erfindungsgegenstände bilden hierfür die Grundlage.

In einem ersten Aspekt betrifft die Erfindung ein isoliertes Protein oder Peptid, das vorzugsweise für einen Teilsyntheseschritt der Biosynthese von [S,S]-EDDS funktional ist, d.h. die Durchführung eines solchen Teilsyntheseschrittes ermöglicht.

Der Ausdruck "Biosynthese" definiert im Sinne der vorliegenden Erfindung nicht nur eine intrazelluläre Synthese von [S,S]-EDDS, sondern umfasst auch die Aufnahme in eine Zelle sowie Ausschleusung aus einer Zelle (Transport über eine Zellmembran).

Der Ausdruck "Protein" kann im Sinne der vorliegenden Erfindung nicht nur ein einzelnes erfindungsgemäßes Protein oder Peptid, sondern auch eine Kombination von mehreren verschiedenen erfindungsgemäßen Proteinen bzw. Peptiden bedeuten.

Ein erfindungsgemäßes Protein oder Peptid kann chemisch oder rekombinant, d.h. biotechnologisch, hergestellt sein.

Alternativ kann ein erfindungsgemäßes Protein oder Peptid aus einem Bakterium, insbesondere aus einem grampositiven Bakterium, bevorzugt aus einem Bakterium der Gattung *Amycolatopsis,* besonders bevorzugt aus einem Bakterium der Art *Amycolatopsis japonicum,* stammen bzw. einem solchen Bakterium entnommen sein.

Das Protein oder Peptid enthält oder besteht aus einer Aminosäuresequenz, welche ausgewählt ist aus der Gruppe bestehend aus SEQ ID Nr. 39, SEQ ID Nr. 41, SEQ ID Nr. 43, SEQ ID Nr. 45, SEQ ID Nr. 47, SEQ ID Nr. 49, SEQ ID Nr. 51 und SEQ ID Nr. 53.

Vorzugsweise weist ein erfindungsgemäßes Protein oder Peptid, das eine der nachstehend aufgeführten Aminosäuresequenzen enthält oder aus einer solchen besteht, die jeweils zugeordnete Aktivität auf:
- SEQ ID Nr. 39 N-Acetyltransferase-Aktivität,
- SEQ ID Nr. 41 Cystein-Dioxygenase (EC 1.13.11.20)-Aktivität,
- SEQ ID Nr. 43 HTH-typ Transkriptionsregulator-Aktivität,
- SEQ ID Nr. 45 Amidase (EC 3.5.1.4)-Aktivität,
- SEQ ID Nr. 47 Ornithin-Cycloamidase (EC 1.4.1.12)-Aktivität,
- SEQ ID Nr. 49 Diaminopimelat-Decarboxylase (EC 4.1.1.20)-Aktivität,
- SEQ ID Nr. 51 Cystathionin-*β*-Synthase (EC 4.2.1.22)-Aktivität,
- SEQ ID Nr. 53 Transporter-Protein-Aktivität.

Das Protein oder Peptid enthaltend oder bestehend aus einer Aminosäuresequenz gemäß SEQ ID Nr. 53 ist insbesondere für den Transport von [S,S]-EDDS oder [S,S]-EDDS-Zink-Komplex aus einer Zelle und/ oder in eine Zelle verantwortlich oder wenigstens an diesem Transport beteiligt.

Die Erfindung betrifft des Weiteren eine isolierte Nukleinsäure, welche vorzugsweise für ein Protein oder Peptid codiert, welches für einen Teilsyntheseschritt der Biosynthese von [S,S]-EDDS funktional ist, d.h. die Durchführung eines solchen Teilsyntheseschrittes ermöglicht.

Die Nukleinsäure enthält oder besteht aus einer Nukleinsäuresequenz, die ausgewählt ist aus der Gruppe bestehend aus
a) Nukleinsäuresequenz, codierend für ein Protein oder Peptid, enthaltend oder bestehend aus einer Aminosäuresequenz, welche ausgewählt ist aus der Gruppe bestehend aus SEQ ID Nr. 39, SEQ ID Nr. 41, SEQ ID Nr. 43, SEQ ID Nr. 45, SEQ ID Nr. 47, SEQ ID Nr. 49, SEQ ID Nr. 51 und SEQ ID Nr. 53 und
b) Nukleinsäuresequenz, die dem komplementären Strang der Nukleinsäuresequenz gemäß a) entspricht.

Der Ausdruck "Nukleinsäure" kann im Sinne der vorliegenden Erfindung nicht nur ein einzelne erfindungsgemäße Nukleinsäure, sondern auch eine Kombination von mehreren verschiedenen erfindungsgemäßen Nukleinsäuren bedeuten.

Weiterhin kann der Ausdruck "Nukleinsäure" im Sinne der vorliegenden Erfindung eine DNA oder eine RNA bedeuten. Eine erfindungsgemäße Nukleinsäure kann insbesondere ausgewählt sein aus der Gruppe bestehend aus Gen bzw. offenes Leseraster (O-pen Reading Frame, ORF), cDNA und mRNA.

Eine erfindungsgemäße Nukleinsäure kann chemisch oder rekombinant, d.h. gentechnologisch, hergestellt sein.

Alternativ kann eine erfindungsgemäße Nukleinsäure aus einem Bakterium, insbesondere aus einem grampositiven Bakterium, bevorzugt aus einem Bakterium der Gattung *Amycolatopsis,* besonders bevorzugt aus einem Bakterium der Art *Amycolatopsis japonicum,* stammen bzw. einem solchen Bakterium entnommen sein.

In einem weiteren Aspekt betrifft die Erfindung eine isolierte Nukleinsäure, welche vorzugsweise für ein Protein oder Peptid codiert, welches für einen Teilsyntheseschritt der Biosynthese von [S,S]-EDDS funktional ist, d.h. die Durchführung eines solchen Teilsyntheseschrittes ermöglicht, wobei die Nukleinsäure eine Nukleinsäuresequenz enthält oder aus einer solchen Sequenz besteht, die ausgewählt ist aus der Gruppe bestehend aus SEQ ID Nr. 40, SEQ ID Nr. 42, SEQ ID Nr. 44, SEQ ID Nr. 46, SEQ ID Nr. 48, SEQ ID Nr. 50, SEQ ID Nr. 52 und SEQ ID Nr. 54.

Die oben genannten Nukleinsäuresequenzen codieren bevorzugt wie folgt:
- SEQ ID Nr. 40 für eine Aminosäuresequenz gemäß SEQ ID Nr. 39,
- SEQ ID Nr. 42 für eine Aminosäuresequenz gemäß SEQ ID Nr. 41,
- SEQ ID Nr. 44 für eine Aminosäuresequenz gemäß SEQ ID Nr. 43,
- SEQ ID Nr. 46 für eine Aminosäuresequenz gemäß SEQ ID Nr. 45,
- SEQ ID Nr. 48 für eine Aminosäuresequenz gemäß SEQ ID Nr. 47,
- SEQ ID Nr. 50 für eine Aminosäuresequenz gemäß SEQ ID Nr. 49,
- SEQ ID Nr. 52 für eine Aminosäuresequenz gemäß SEQ ID Nr. 51,
- SEQ ID Nr. 54 für eine Aminosäuresequenz gemäß SEQ ID Nr. 53.

Die Nukleinsäure enthält oder besteht aus einer Nukleinsäuresequenz, die ausgewählt ist aus der Gruppe bestehend aus SEQ ID Nr. 40, SEQ ID Nr. 42, SEQ ID Nr. 44, SEQ ID Nr. 46, SEQ ID Nr. 48, SEQ ID Nr. 50, SEQ ID Nr. 52, und SEQ ID Nr. 54.

Die Erfindung umfasst des Weiteren ein isoliertes Gen-Cluster oder ein Operon, welches vorzugsweise für die Biosynthese von [S,S]-EDDS funktional ist, d.h. die Durchführung einer solchen Biosynthese ermöglicht.

Das oben erwähnte Operon enthält in der Regel ferner einen Promotor sowie insbesondere einen oder gegebenenfalls mehrere Operatoren.

Das Gen-Cluster oder Operon enthält oder besteht aus wenigstens zwei Nukleinsäuresequenzen, die ausgewählt sind aus der Gruppe bestehend aus SEQ ID Nr. 40, SEQ ID Nr. 42, SEQ ID Nr. 44, SEQ ID Nr. 46, SEQ ID Nr. 48, SEQ ID Nr. 50, SEQ ID Nr. 52, SEQ ID Nr. 54 und Kombinationen davon.

Das Gen-Cluster oder Operon kann insbesondere alle der im vorherigen Absatz genannten Nukleinsäuresequenzen enthalten oder aus diesen Sequenzen bestehen.

Die Bezeichnung Gen-Cluster entstammt der Erkenntnis, dass Bakterien und viele Eukaryoten in der Regel einen koordinierten Mechanismus zur Regulation von solchen Genen anwenden, deren Produkte (Proteine oder Peptide) an zusammenhängenden Prozessen, beispielsweise einer Biosynthese, beteiligt sind. Derartige Gene liegen auf einem einzelnen Chromosom in Strukturen, die als Gen-Cluster bezeichnet werden, gemeinsam vor und können zusammen unter der Kontrolle einer einzelnen regulatorischen Sequenz oder mehrerer regulatorischer Sequenzen transkribiert werden. Ein Gen-Cluster kann grundsätzlich aber auch mehrere Promotoren enthalten und auch von mehreren Regulatoren kontrolliert werden. Ein Gen-Cluster, ein Promotor und gegebenenfalls weitere Sequenzen, die bei der Regulation zusammenwirken, können auch als Operon bezeichnet werden.

In einem weiteren Aspekt betrifft die Erfindung ein isoliertes Protein oder Peptid, welches für eine Inhibierung bzw. Repression der Biosynthese von [S,S]-EDDS funktional ist, d.h. eine solche Inhibierung bzw. Repression ermöglicht. Bevorzugt handelt es sich bei dem Protein oder Peptid um einen Transkriptionsinhibitor bzw. -repressor für die Biosynthese von [S,S]-EDDS.

Das Protein oder Peptid enthält oder besteht aus einer Aminosäuresequenz gemäß SEQ ID Nr. 61.

Bei dem Protein handelt es sich vorzugsweise um ein Zinkaufnahmeregulatorprotein, ein sogenanntes Zur (Zinc uptake regulator)-Protein, d.h. um ein Protein, welches eine die zelluläre Aufnahme von Zink regulierende Aktivität aufweist. Im Allgemeinen ist ein Zur-Protein befähigt, Zink-Ionen ab einer bestimmten Konzentration zu binden und den Zinkhaushalt in einer Bakterienzelle durch eine zinkabhängige Genrepression oder Genexpression zu regulieren. Diese zinkabhängige Regulation von Genen wird in vielen Bakterien vermittelt, indem ein zinksaturiertes Zur-Protein, ein sogenanntes *holo*Zur-Protein, an spezifische Nukleinsäuresequenzen vor den entsprechenden Zielgenen bindet, so dass dem für die Transkription von Zielgenen essentiellen Enzym RNA-Polymerase der Zugang zu diesen verwehrt wird. Die Transkription der Zielgene wird auf diese Weise verhindert. Bei den spezifischen Nukleinsäuresequenzen, die als Zur-Bindungsstelle fungieren, handelt es sich in der Regel um Promotoren/ Promotorregionen oder Operatoren.

Da die zinkabhängige Regulation der Genrepression oder Genexpression von der Konzentration der Zink-Ionen im Zellinneren und außerhalb der Zelle abhängt, eignen sich derartige Systeme insbesondere auch als Reportersysteme zur Bestimmung der Zink-Ionenkonzentration.

Überraschenderweise wurde nun von den Erfindern, wie im Beispielteil noch ausführlicher erläutert werden wird, am Beispiel von *Amycolatopsis japonicum* festgestellt, dass es sich bei den Zielgenen, die durch das erfindungsgemäße Protein oder Peptid, enthaltend oder bestehend aus der Aminosäuresequenz gemäß SEQ ID Nr. 61, in Anwesenheit von Zink reprimiert werden, unter anderem um Biosynthesegene für die Herstellung von [S,S]-EDDS handelt. Bezüglich der Nukleinsäuresequenzen besagter Biosynthesegene wird auf die in der bisherigen Beschreibung genannten Sequenzen Bezug genommen.

In einem weiteren Aspekt betrifft die Erfindung einen artifiziellen bzw. rekombinanten, d.h. gentechnologisch hergestellten, Expressionsvektor, d.h. ein Vehikel zur Übertragung wenigstens einer Nukleinsäure in eine Empfänger- oder Wirtszelle, die dann im Rahmen einer Genexpression wenigstens ein Protein bzw. Peptid, für welches die wenigstens eine Nukleinsäure codiert, exprimiert.

Der Expressionsvektor enthält wenigstens eine erfindungsgemäße Nukleinsäure. Es kann indes bevorzugt sein, wenn der Expressionsvektor keine Nukleinsäure, enthaltend oder bestehend aus Sequenz gemäß SEQ ID Nr. 62, oder eine hierzu homologe Sequenz enthält.

Alternativ kann der Expressionsvektor ein erfindungsgemäßes Gen-Cluster oder Operon oder ein integratives Element enthalten.

Bei dem Expressionsvektor kann es sich grundsätzlich um ein Plasmid oder ein Cosmid handeln. Bevorzugt ist ein Plasmid bzw. Cosmid, das in das Chromosom von Actinomyceten integrieren kann oder als replikatives Plasmid bzw. Cosmid in der Zelle existiert und einen entsprechenden konstitutiven oder induzierbaren (regulierbaren) Promotor enthält.

In einer bevorzugten Ausführungsform handelt es sich bei dem Expressionsvektor um ein Plasmid der pRM-Familie, insbesondere ein Plasmid des Typs pRM4, in welches die wenigstens eine erfindungsgemäße Nukleinsäure oder das erfindungsgemäße Gen-Cluster insertiert ist.

Gemäß einer besonders bevorzugten Ausführungsform enthält der Expressionsvektor einen Promotor ohne Zink-Repression (None-Zinc-Repressed Promotor), d.h. einen Promotor, der keiner Zinkregulation unterliegt.

Bei dem Promotor kann es sich grundsätzlich um einen konstitutiven oder induzierbaren (regulierbaren) Promotor handeln. Bevorzugt handelt es sich bei dem Promotor um einen stark konstitutiv exprimierten oder induzierbaren Promotor, der einen intrazellulär vorhandenen Zur-Zielpromotor ersetzt. In diesem Fall erfolgt die Expression von erfindungsgemäßen Nukleinsäuren bzw. Gen-Clustern mit besonderem Vorteil unter der Kontrolle eines zinkunabhängigen Promotors. Der Promotor weist insbesondere keine Bindungsstelle für ein erfindungsgemäßes Protein gemäß SEQ ID Nr. 61 auf. Ein bevorzugter Promotor ohne Zink-Repression ist beispielsweise der Promotor ermE (promoter of erythromycin resistance gene, PermE).

Der Expressionsvektor enthält eine erfindungsgemäße Nukleinsäure, wobei die Nukleinsäure eine Nukleinsäuresequenz enthält oder aus einer solchen besteht, die ausgewählt ist aus der Gruppe bestehend aus SEQ ID Nr. 40, SEQ ID Nr. 42, SEQ ID Nr. 44, SEQ ID Nr. 46, SEQ ID Nr. 48, SEQ ID Nr. 50, SEQ ID Nr. 52, SEQ ID Nr. 54 und Kombinationen davon.

Besonders bevorzugte Nukleinsäuresequenzen können ausgewählt sein aus der Gruppe bestehend aus SEQ ID Nr. 48, SEQ ID Nr. 50, SEQ ID Nr. 52, SEQ ID Nr. 54 und Kombinationen davon.

Der erfindungsgemäße Expressionsvektor eignet sich sowohl zur Durchführung einer homologen Expression als auch zur Durchführung einer heterologen Expression.

Bezüglich weiterer Merkmale und Vorteile des Expressionsvektors, insbesondere der erfindungsgemäßen Nukleinsäuren sowie des erfindungsgemäßen Gen-Clusters und Operons, wird vollständig auf die bisherige Beschreibung Bezug genommen.

In einem weiteren Aspekt betrifft die Erfindung eine artifizielle bzw. rekombinante, d.h. biotechnologisch hergestellte, Wirtszelle.

Die Wirtszelle ist vorzugsweise eine [S,S]-EDDS produzierende Wirtszelle.

Die Wirtszelle zeichnet sich dadurch aus, dass sie einen erfindungsgemäßen Expressionsvektor enthält. In Kombination kann die Wirtszelle ein Genom enthalten, welches aufgrund einer wenigstens teilweise, insbesondere vollständigen, Insertierung des Vektors (bzw. Vektorgenoms) modifiziert vorliegt.

Im Hinblick auf eine Biosynthese von [S,S]-EDDS ist es bevorzugt, wenn in der Wirtszelle keine Zink-Repression möglich ist.

Besonders bevorzugt enthält die Wirtszelle keine für ein Zur-Protein codierende Nukleinsäure, insbesondere keine Nukleinsäure gemäß der Nukleinsäuresequenz SEQ ID Nr. 62 oder eine hierzu homologe Nukleinsäure, und/oder kein Zur-Protein, insbesondere kein Protein oder Peptid gemäß der Aminosäuresequenz SEQ ID Nr. 61 oder ein hierzu homologes Protein bzw. Peptid.

Bei der Wirtszelle kann es sich grundsätzlich um eine eukaryotische oder prokaryotische Zelle handeln.

Des Weiteren kann es sich bei der Wirtszelle um ein homologe oder heterologe Wirtszelle handeln.

Die Wirtszelle kann insbesondere ausgewählt sein aus der Gruppe bestehend aus Bakterienzelle, Hefezelle und Pilzzelle.

In einer weitergehenden Ausführungsform handelt es sich bei der Wirtszelle um ein grampositives Bakterium, bevorzugt um ein Bakterium der Gattung *Amycolatopsis* oder *Streptomyces,* besonders bevorzugt um ein Bakterium der Art *Amycolatopsis japonicum* oder *Streptomyces coelicolor.*

Die Wirtszelle kann in einer weiteren Ausführungsform mittels Transformation, Transduktion, Transfektion oder Konjugation, insbesondere unter Verwendung eines erfindungsgemäßen Expressionsvektors, hergestellt oder herstellbar sein.

Die im vorhergehenden Absatz genannten Techniken zum Einbringen von (Fremd-)Nukleinsäuren, insbesondere von DNA und RNA, in eukaryotische und prokaryotische Zellen sind dem Fachmann als solche grundsätzlich bekannt. Es handelt sich um Standardprozeduren der molekularen Genetik. Für eine detailliertere Beschreibung sei daher auf einschlägige Fachliteratur verwiesen (bspw. Mülhardt C., Der Experimentator: Molekularbilologie/Genomics (2008); Spektrum Verlag, 6. Aufl. oder Kieser T. et al., Practical Streptomyces Genetics (2000); John Innes Foundation oder Green and Sambrook, Molecular Cloning: A Laboratory Manual (2012); Cold Spring Harbor Laboratory, 4. Aufl.).

Bezüglich weiterer Merkmale und Vorteile der Wirtszelle, insbesondere der erfindungsgemäßen Nukleinsäuren, des erfindungsgemäßen Gen-Clusters und Operons, der erfindungsgemäßen Proteine bzw. Peptide sowie der erfindungsgemäßen Expressionsvektoren, wird vollständig auf die bisherige Beschreibung Bezug genommen.

Weiterhin wird eine artifizielle bzw. rekombinante, d.h. biotechnologisch hergestellte, und vorzugsweise [S,S]-EDDS produzierende Bakterienzelle, bevorzugt der Gattung *Amycolatopsis,* insbesondere der Art *Amycolatopsis japonicum,* offenbart.

Die Bakterienzelle zeichnet sich dadurch aus, dass sie kein Protein oder Peptid, enthaltend oder bestehend aus einer Aminosäuresequenz gemäß SEQ ID Nr. 61, exprimiert und/oder keine Nukleinsäure, enthaltend oder bestehend aus einer Nukleinsäuresequenz gemäß SEQ ID Nr. 62, enthält.

Die Bakterienzelle kann insbesondere durch eine wenigstens teilweise, vorzugsweise vollständige, Deletion einer Nukleinsäure, enthaltend oder bestehend aus einer Nukleinsäuresequenz gemäß SEQ ID Nr. 62, aus dem Genom, insbesondere WildtypGenom, der Bakterienzelle hergestellt oder herstellbar sein. Die Deletion kann dabei das Ergebnis einer Transformation, Transduktion, Transfektion oder Konjugation sein. Mit anderen Worten kann es sich bei der Bakterienzelle bevorzugt um eine Deletionsmutante handeln. Alternativ zu der in diesem Absatz erwähnten Deletion kommt auch der Austausch eines Basenpaares, eine Punktmutation und/oder eine Inaktivierung durch Insertion in Betracht.

Daneben kann die Bakterienzelle auch durch Insertion einer Nukleinsäure, beispielsweise in Form eines Plasmids oder einer Genkassette, enthaltend oder bestehend aus einer Nukleinsäuresequenz gemäß SEQ ID Nr. 62, in das Genom, insbesondere Wildtyp-Genom, der Bakterienzelle hergestellt oder herstellbar sein.

Bezüglich weiterer Merkmale und Vorteile der Bakterienzelle, insbesondere der in den vorhergehenden Absätzen erwähnten Nukleinsäure sowie des in den vorhergehenden Absätzen erwähnten Proteins bzw. Peptids, wird vollständig auf die bisherige Beschreibung Bezug genommen.

In einem weiteren Aspekt betrifft die Erfindung ein Verfahren für die Biosynthese von [S,S]-EDDS, umfassend die Schritte:
a) Kultivieren einer [S,S]-EDDS produzierenden erfindungsgemäßen Wirtszelle oder einer rekombinanten Bakterienzelle der Gattung *Amycolatopsis,* insbesondere von *Amycolatopsis japonicum,* welche kein Protein oder Peptid, enthaltend oder bestehend aus einer Aminosäuresequenz gemäß SEQ ID Nr. 61, exprimiert und/oder keine Nukleinsäure, enthaltend oder bestehend aus einer Nukleinsäuresequenz gemäß SEQ ID Nr. 62, enthält, und
b) Aufreinigen von [S,S]-EDDS aus der Zelle und/oder einem für die Zelle verwendeten Kulturmedium.

In einer bevorzugten Ausführungsform wird die Bakterienzelle mittels Einbringen eines erfindungsgemäßen Deletionsvektors in die Bakterienzelle, insbesondere in einen Wildtyp der Bakterienzelle, hergestellt. Das Einbringen des Vektors kann mittels Transformation, Transduktion, Transfektion oder Konjugation erfolgen.

Die Wirtszelle wird gemäß einer weiteren Ausführungsform mittels Einbringen eines erfindungsgemäßen Expressionsvektors in die Zelle hergestellt.

Bevorzugt wird die Wirtszelle dadurch hergestellt, dass ein erfindungsgemäßer Expressionsvektor in eine Bakterienzelle der Gattung *Amycolatopsis,* insbesondere der Art *Amycolatopsis japonicum,* eingebracht wird.

Bei einer alternativen Ausführungsform wird die Wirtszelle dadurch hergestellt, dass ein erfindungsgemäßer Expressionsvektor in eine Bakterienzelle der Gattung *Streptomyces,* insbesondere der Art *Streptomyces* coelicolor, eingebracht wird.

Die Kultivierung der im Rahmen eines erfindungsgemäßen Verfahrens eingesetzten Zellen kann unter Anwendung von dem Fachmann an sich bekannten Standardprotokollen erfolgen.

Abhängig von der verwendeten Wirtszelle bzw. Bakterienzelle kann es vorteilhaft sein, die Wirts- bzw. Bakterienzelle unter zinkfreien, insbesondere zinksalzfreien, Bedingungen zu kultivieren.

Erfindungsgemäß kann es vorgesehen sein, wenigstens eine Vorläuferverbindung für die Biosynthese von [S,S]-EDDS einem für die Kultivierung vorgesehenen Kulturmedium hinzuzufügen. Beispiele für geeignete Vorläuferverbindungen können grundsätzlich proteinogene und/ oder nicht-proteinogene Aminosäuren sein. Geeignete Vorläuferverbindungen können insbesondere ausgewählt sein aus der Gruppe bestehend aus L-Ornithin, L-Serin, L-Prolin, 2,3-Diaminopropionsäure, L-Aspartat, L-Lysin und Kombinationen davon.

Erfindungsgemäß kann es zudem vorgesehen sein, dass vor der Aufreinigung von [S,S]-EDDS zunächst eine Konzentrationsbestimmung des produzierten [S,S]-EDDS erfolgt. Dies kann beispielsweise mittels HPLC (high performance liquid chromatography bzw. Hochleistungsflüssigkeitschromatographie), UV/VIS-Spektroskopie oder mittels einer Kombination beider Techniken erfolgen.

Zur weiteren Aufreinigung von [S,S]-EDDS kann ein Separatorschritt zur Abtrennung von festen (Zell-)Bestandteilen erfolgen. An diesen kann sich eine Isolierung von [S,S]-EDDS mittels lonentauscheradsorption mit nachfolgender Fällungskristallisation anschließen.

Derartige Verfahren sind als solche aus dem Stand der Technik bekannt. Es spielt für die Aufreinigung grundsätzlich keine Rolle, ob das [S,S]-EDDS intrazellulär vorliegt und beispielsweise noch durch eine Zelllyse zugänglich gemacht werden muss oder ob es von dem Exportsystem in den Überstand sekretiert wird.

Bezüglich weiterer Merkmale und Vorteile des Verfahrens, insbesondere der erfindungsgemäßen Nukleinsäuren, des erfindungsgemäßen Gen-Clusters und Operons, der erfindungsgemäßen Proteine bzw. Peptide sowie der erfindungsgemäßen Vektoren, insbesondere Deletions- und Expressionsvektoren, wird vollständig auf die bisherige Beschreibung Bezug genommen.

In einem weiteren Aspekt betrifft die Erfindung ein Kit für die Biosynthese von [S,S]-EDDS, umfassend wenigstens eine Komponente, die ausgewählt ist aus der Gruppe bestehend aus wenigstens ein erfindungsgemäßes Protein oder Peptid, wenigstens eine erfindungsgemäße Nukleinsäure, ein erfindungsgemäßes Gen-Cluster oder Operon, ein erfindungsgemäßer Vektor (Expressions- und/oder Deletionsvektor), eine erfindungsgemäße Wirtszelle und Kombinationen davon.

Gegebenenfalls kann das Kit eine weitere Komponente umfassen, die ausgewählt ist aus der Gruppe bestehend aus Kulturmedium, Puffer und Kombinationen davon.

Bezüglich weiterer Merkmale und Vorteile des Kits, insbesondere der erfindungsgemäßen Nukleinsäuren, des erfindungsgemäßen Gen-Clusters und Operons, der erfindungsgemäßen Proteine bzw. Peptide sowie der erfindungsgemäßen Vektoren, insbesondere Deletions- und Expressionsvektoren, wird ebenfalls vollständig auf die bisherige Beschreibung Bezug genommen.

Weitere Merkmale und Vorteile der Erfindung ergeben sich aus den nachfolgenden Beispielen in Verbindung mit den Figuren und Unteransprüchen. Insbesondere wird die Erfindung durch Beschreibung der Identifizierung und Annotation des [S,S]-EDDS Biosynthesegen-Clusters näher erläutert. In den Ausführungsformen können einzelne Merkmale der Erfindung allein oder in Kombination mit anderen Merkmalen verwirklicht sein.

### Figurenbeschreibung

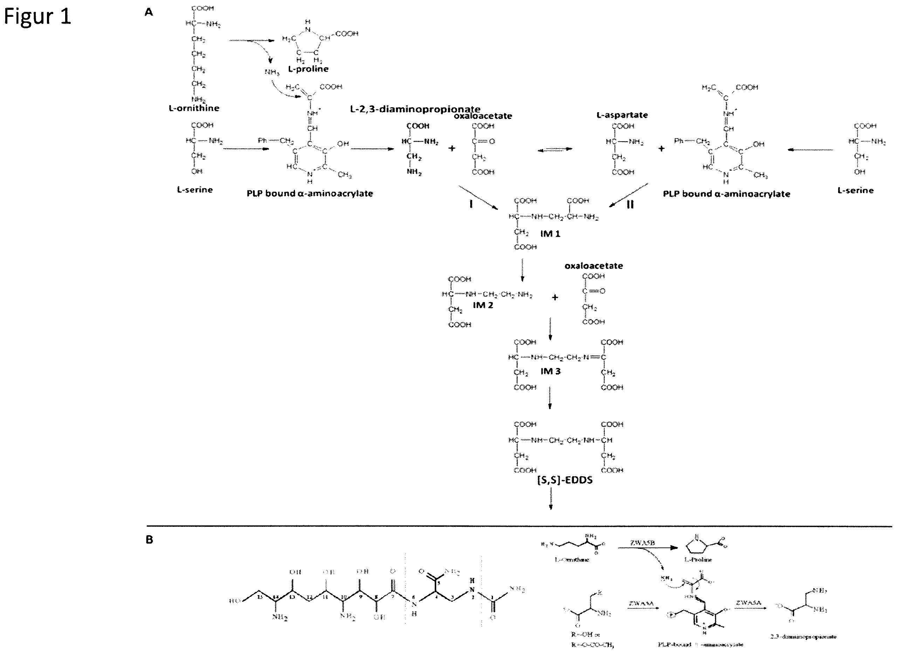

**Figur 1** zeigt schematisch den hypothetischen Biosyntheseweg von [S,S]-EDDS. (**A**) [S,S]-EDDS Biosyntheseweg. (I) Bereitstellung der Dap-Vorstufe; Umwandlung von L-Serin mit L-Ornithin als Aminodonator und PLP als Cofaktor unter Freisetzung von L-Prolin. Dap reagiert mit Oxalacetat zu IM1. (II) L-Aspartat und L-Serin reagieren unter Anwesenheit von PLP als Cofaktor zu IM1. (**B**) Chemische Struktur von Zwittermycin A (links). Der gestrichelte Kasten deutet den Dap-Baustein an. Biosyntheseweg von Dap (rechts), katalysiert durch konzertiertes Zusammenwirken von Zwittermycin A5A (ZWA5A, Homolog der Cystein Synthetase) und Zwittermycin A5B (ZWA5B, Homolog der Ornithin-Cyclodeaminase).

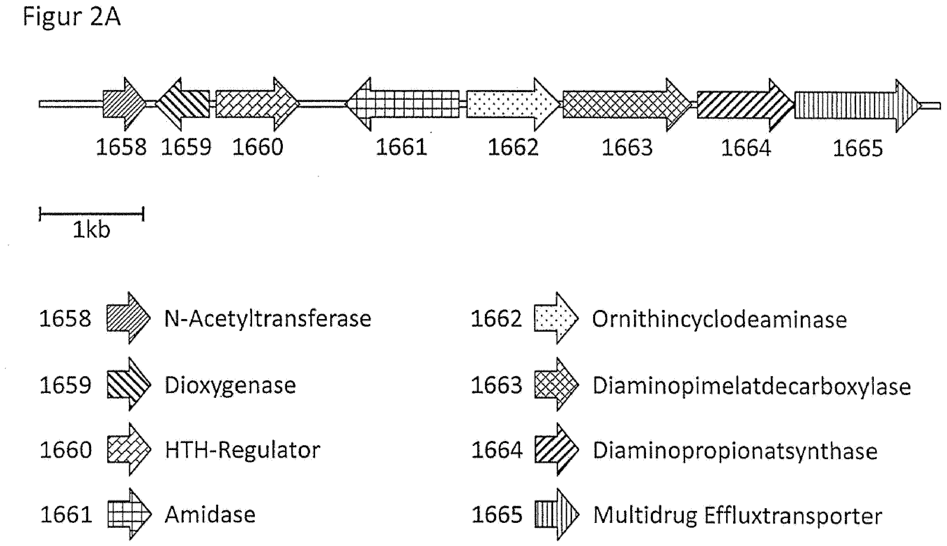

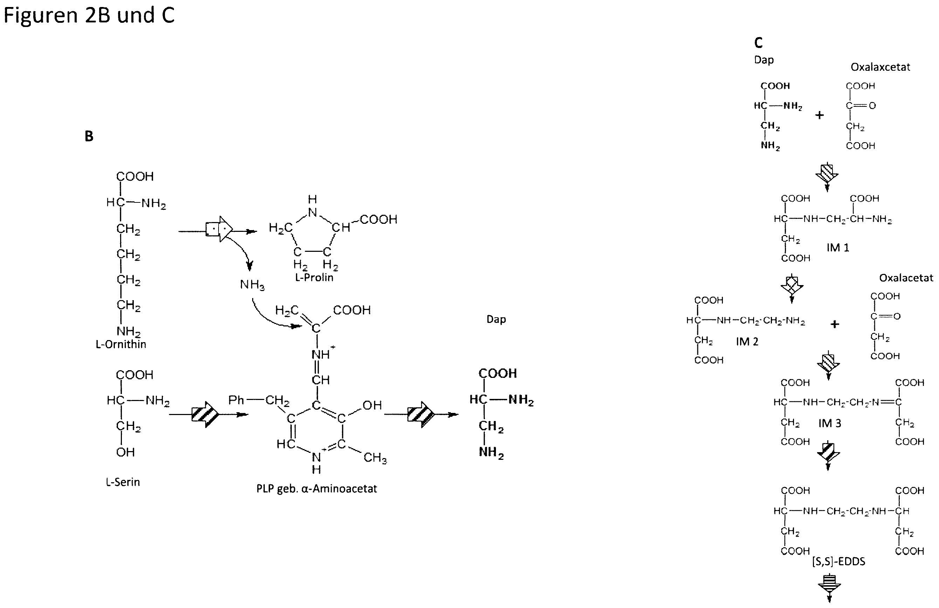

**Figur 2** zeigt den [S,S]-EDDS-Biosyntheseweg mit zugeordneten Biosynthesegenen und dem zugehörigen Gen-Cluster. (**A**) Genorganisation des [S,S]-EDDS-Biosynthesegenclusters. N-Acetyltransferase (SEQ ID Nr. 40 (orf1658)), Cysteindioxygenase (SEQ ID Nr. 42 (orf 1659)), Transkriptionsregulator vom HTH-Typ (SEQ ID Nr. 44 (orf1660)), Amidase (SEQ ID Nr. 46 (orf1661)), Ornithincyclodeaminase (SEQ ID Nr. 48 (orf1662)), Diaminopimelat-Decarboxylase (SEQ ID Nr. 50 (orf1663)), Dap-Synthase (SEQ ID Nr. 52 (orf1664)) und Multi-Drug-Effluxtransporter (SEQ ID Nr. 54 (orf1665). (**B**) Bereitstellung der Vorstufen von Dap durch Umwandlung von L-Serin mit L-Ornithin als Aminodonator und PLP als Cofaktor, katalysiert durch eine konzertierte Aktion von Ornithin-Cyclodeaminase und einer Dap-Synthase. (**C**) Zusammenstellung der Dap-Vorstufenverbindungen und zweier Oxalacetate zu [S,S]-EDDS. Die Pfeile sind mit den entsprechen Genen aus dem Gencluster (A) markiert.

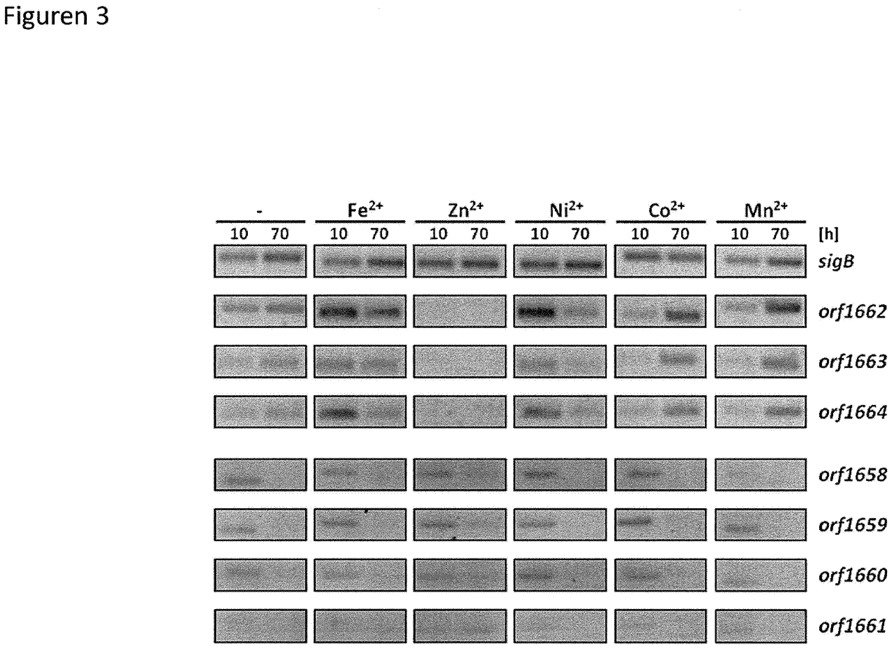

**Figur 3** zeigt das mittels RT-PCR gewonnene spurenmetallabhängige Transkriptionsmuster des [S,S]-EDDS-Biosynthesegen-Clusters in *A. japonicum.* Als Haushaltsgen (Housekeeping Gene) wurde sigB verwendet, um die RNA zu normalisieren. RNAs wurden aus Kulturen gewonnen, die in definiertem Medium in Abwesenheit jeglicher Spurenelemente (-) oder versetzt mit 25 µM Fe²⁺-, Zn²⁺-, Ni²⁺-, Co²⁺- bzw. Mn²⁺-Lösungen kultiviert wurden. Nach 10 h und 70 h Inkubation wurden Proben entnommen.

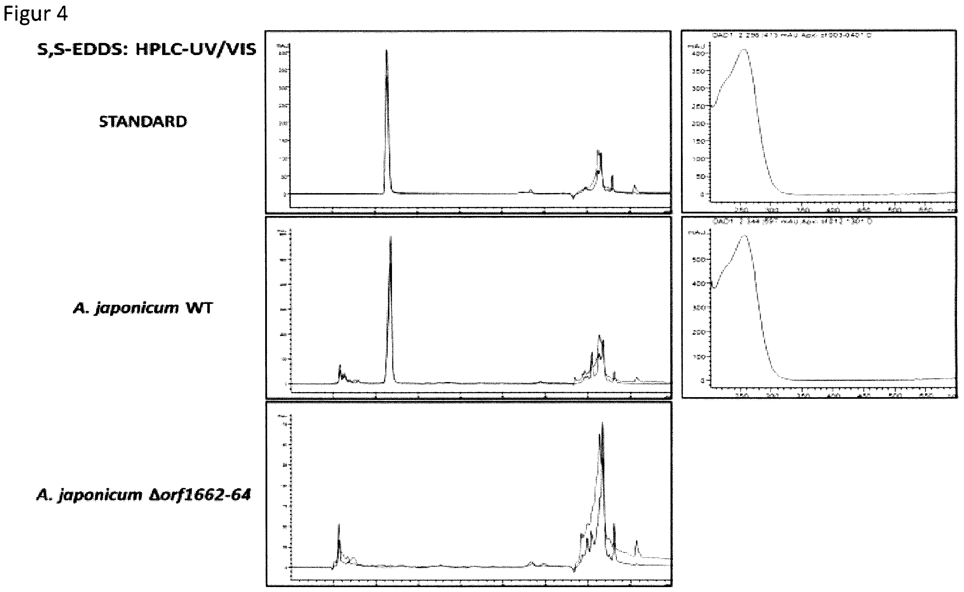

**Figur 4** zeigt die HPLC-UV/VIS-Analyse der Überstände von *A. japonicum* WT (Wildtyp) und einer Mutante *A. japonicum* Δorf1662-64 nach 72 h Kultur in M7-Medium. (Oben) [S,S]-EDDS-Standard [350 mg/L]. (Mitte) *A. japonicum* WT. (Unten) *A. japonicum* Δorf1662-64. (Rechts) Spezifische UV/VIS-Spektren von [S,S]-EDDS.

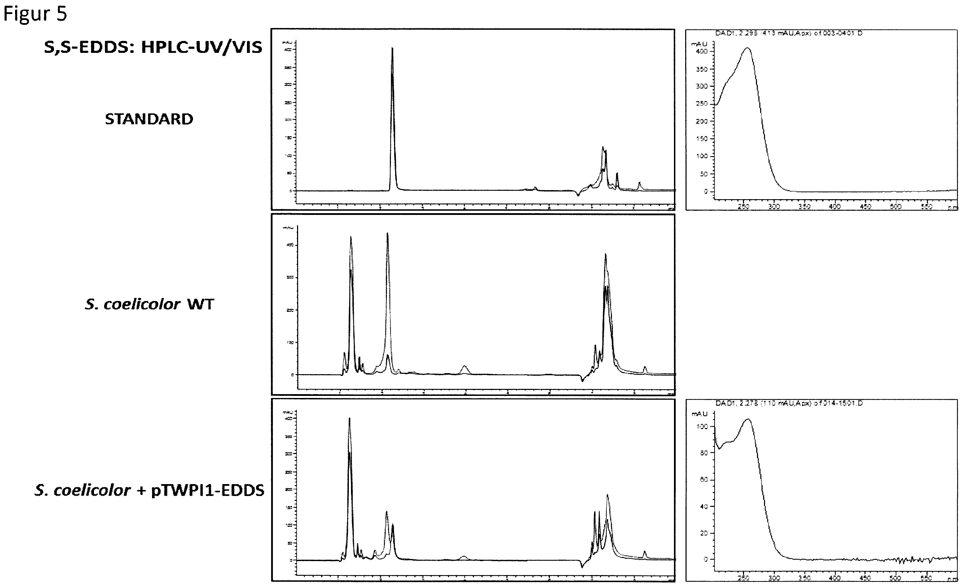

**Figur 5** zeigt die HPLC-UV/VIS-Analyse der Überstände von *S. coelicolor* WT und *S. coelicolor*-pTWPL1-EDDS nach 72 h Kultur in M7-Medium. (Oben) [S,S]-EDDS-Standard [350 mg/L]. (Mitte) *S. coelicolor* WT. (Unten) *S. coelicolor*-pTWPL1-EDDS. (Rechts) Spezifische UV/VIS-Spektren von [S,S]-EDDS.

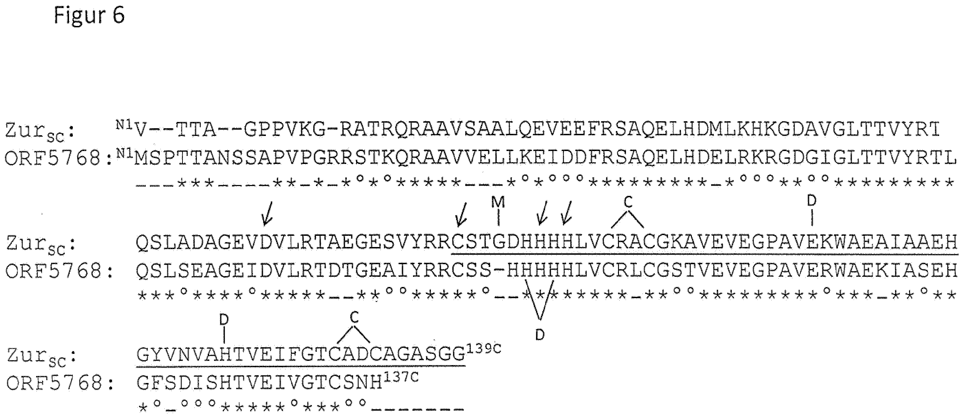

**Figur 6** zeigt den Aminosäurensequenzabgleich von *S. coelicolor* Zur (Zur_{SC}) und *A. japonicum* ORF5768 (SEQ ID Nr. 62). (Oben) Zur_{SC}-Monomer: DNA-Bindungsdomäne_{N} (Reste 1-77) nicht unterstrichen; Hinge Loop (Reste 78-85) durchgehend unterstrichen; Dimerisierungsdomäne_{C} (Reste 86-139) mit unterbrochener Unterstreichung. Identische Aminosäuren sind mit einem Sternchen (*) markiert; ähnliche Reste sind durch einen Kreis (°) markiert. Zinkbindungsstellen: Pfeil: Zur_{SC}: D65, C79, H85, H87 und ORF5768: D70, C84, H89 und H91. Abweichung relativer aa-Distanzen in einer M-Stelle (mit M gekennzeichnet). D-Stelle (mit D gekennzeichnet): Zur_{SC}: H84, H86, E105, H122 und ORF5768: H88, H90, E109, H126. C-Stelle (mit C gekennzeichnet): Zur_{SC}: C90, C93, C130, C133 und ORF5768: C94, C97, C134, H137.

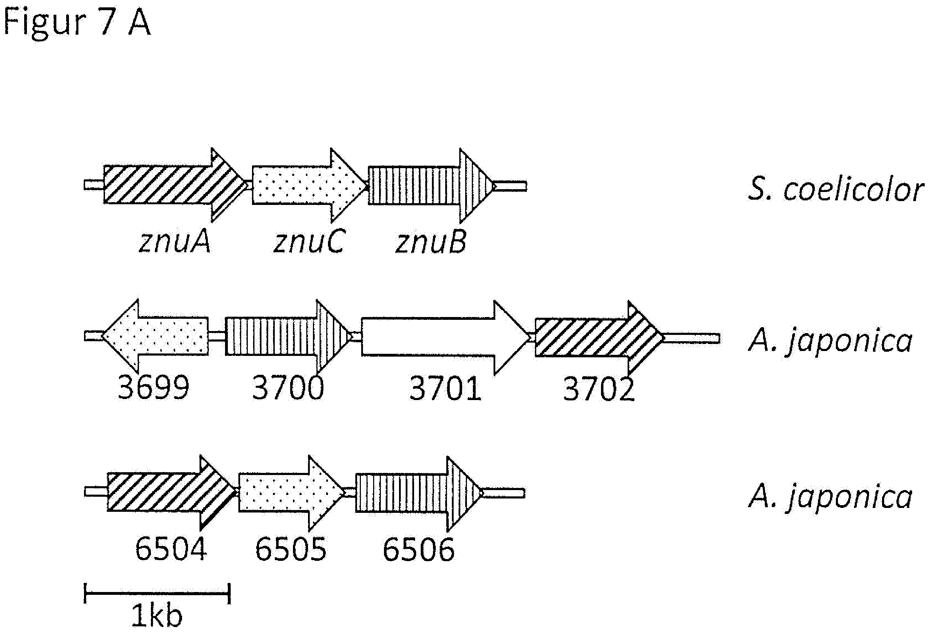

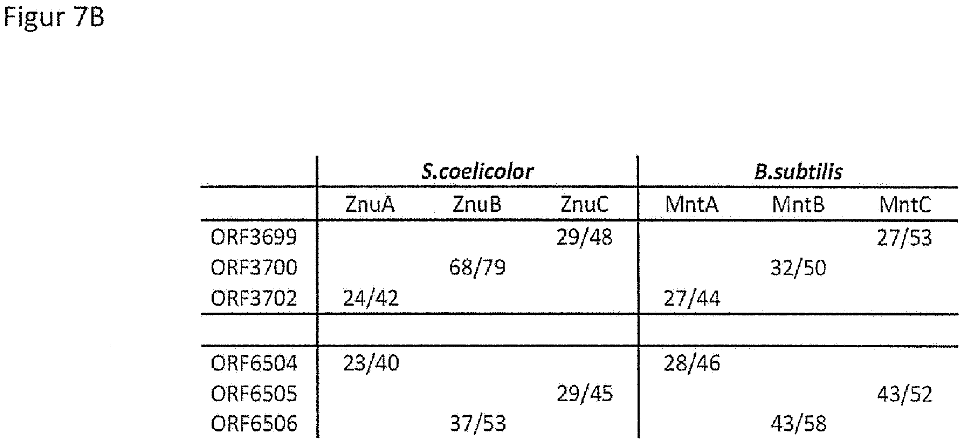

**Figur 7** zeigt den BLAST-Abgleich von *S. coelicolor* ZnuABC und *B. subtilis* MntABC with *A. japonicum* Homologen. (**A**) Genorganisation von von *S. coelicolor* znuABC, der Operon-ähnlichen Strukturen orf3699, orf3700, orf3702 und orf6504, orf6505, orf6506 von *A. japonicum.* (B) Aminosäurensequenzabgleich, (Oben) ORF3699, ORF3700 und ORF3702 (unten) ORF6504, ORF6505 und ORF6506 vs. *S. coelicolor* ZnuABC (links); vs. *B. subtilis* MntABC (rechts). X/X = % Identität/Ähnlichkeit.

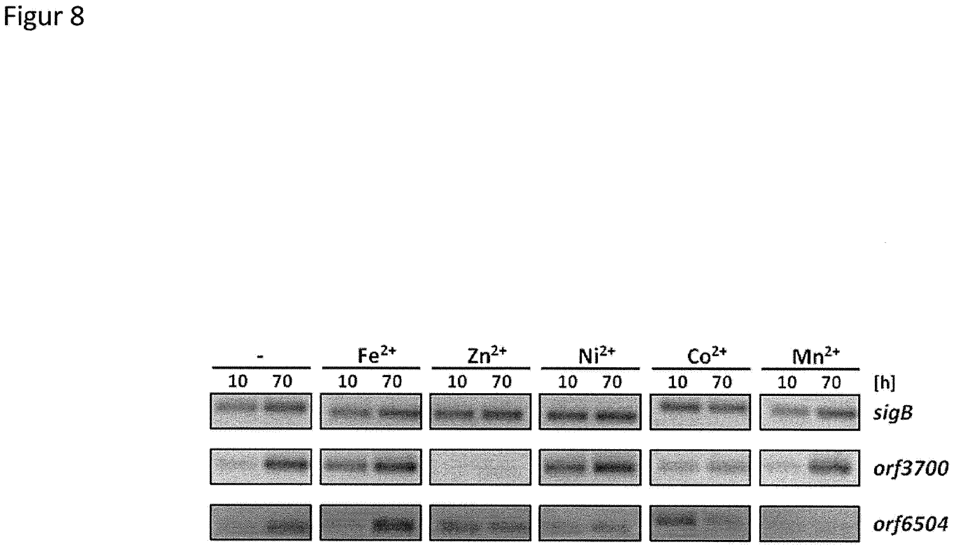

**Figur 8** zeigt das mittels RT-PCR gewonnene spurenmetallabhängige Transkriptionsmuster des Metallaufnahmesystems von *A. japonicum.* Als Haushaltsgen wurde sigB verwendet, um die RNA zu normalisieren. RNAs wurden aus Kulturen gewonnen, die in definiertem M7-Medium in Abwesenheit jeglicher Spurenelemente (-) oder versetzt mit 25 µM Fe²⁺-, Zn²⁺-, Ni²⁺-, Co²⁺- bzw. Mn²⁺-Lösungen kultiviert wurden. Nach 10 h und 70 h Inkubation wurden Proben entnommen. orf3700 und orf6504 wurden als Sonden gewählt, um die gesamten operonartigen Strukturen darzustellen.

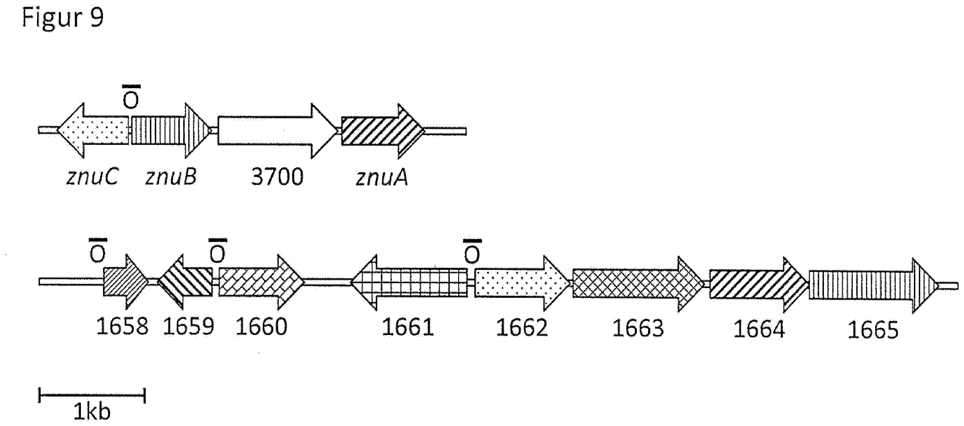

**Figur 9** zeigt schematisch die verwendeten EMSA-DNA-Sonden. (Oben) znuABC-Genloki von *A. japonicum* (orf3699-orf3702). (Unten) [S,S]-EDDS-BiosynthesegenCluster orf1658-orf1665 (entspricht SEQ ID Nr. 40, 42, 44, 46, 48, 50, 52, 54). Promotorregionen mit Zur_{AJ}-Bindungsstellen sind mit einem offenen Kreis markiert und EMSA-Sonden, die zum Prüfen der His-ORF5768-Bindung verwendet wurden, sind durch einen schwarzen Strich dargestellt.

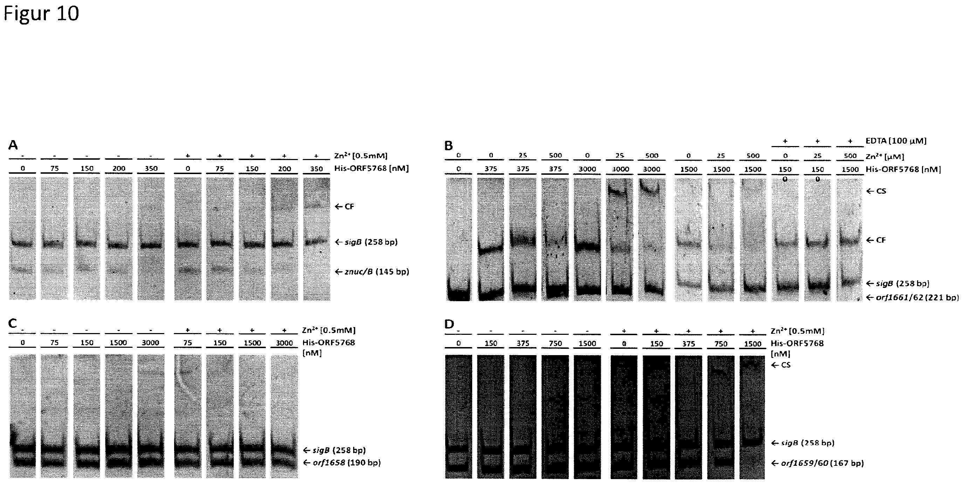

**Figur 10** zeigt die Ergebnisse des EMS Assays (Polyacryamid-Gele nach Ethidiumbromid-Färbung): Zinkabhängige Bindung von gereinigtem His₆-ORF5768 an Promotorregionen. Zwei deutliche Bindungsfälle mit unterschiedlichen Mobilitäten wurden als CF (schnell beweglicher Komplex) und CD (langsam beweglicher Komplex) bezeichnet. Zur Bestätigung der Spezifizität der Bindungskomplexe wurde dem Bindungsgemisch ein sigB-RT-Fragment zugesetzt. Gereinigtes His₆-ORF5768 wurde bei verschiedenen Konzentrationen mit ungefähr 35 nM DNA-Sonde in Abwesenheit oder in Gegenwart von Zink (ZnSO₄) inkubiert. (**A**) Bindungsassay mit znuCB-Sonde (vgl. Figur 9). Bindungsfall durch CF-Bande dargestellt. (**B**) Bindungsassay mit intergener orf1661/62-Sonde (vgl. Figur 9). Zwei Bindungsfälle sind durch CF-Bande bzw. CS-Bande dargestellt. (**C**) Bindungsassay mit orf1658-Promotorsonde und erhöhten His-ORF5768-Konzentrationen (vgl. Figur 9). (**D**) Bindungsassay mit intergener orf1659/60-Promotorsonde (vgl. Figur 9). Bindungsfall durch CS-Bande dargestellt.

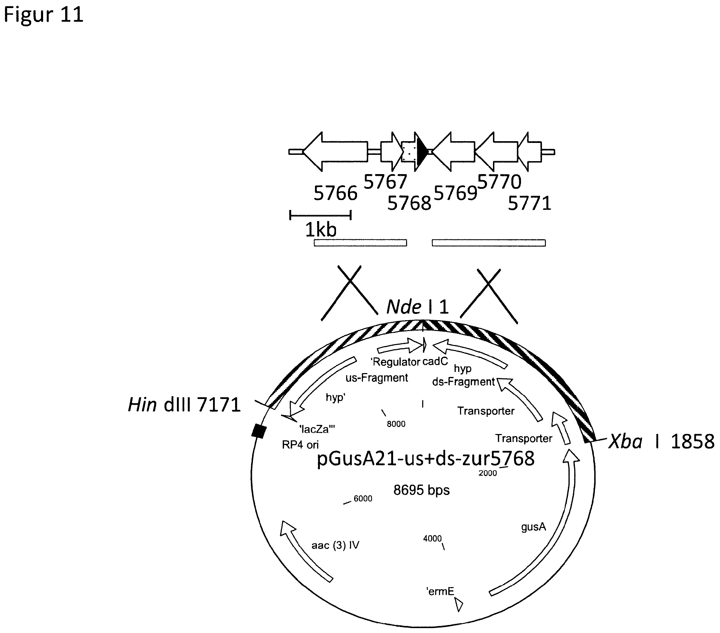

**Figur 11** zeigt eine schematische Darstellung des Deletionsvektors pGusA21Δorf5768. (Oben) orf5768 (entspricht SEQ ID Nr. 62) und umgebende Gene. (Unten) pGusA21Δorf5768. linker Balken: 5' flankierende Region von orf5768; rechter Balken: 3' flankierende Region von orf5768.

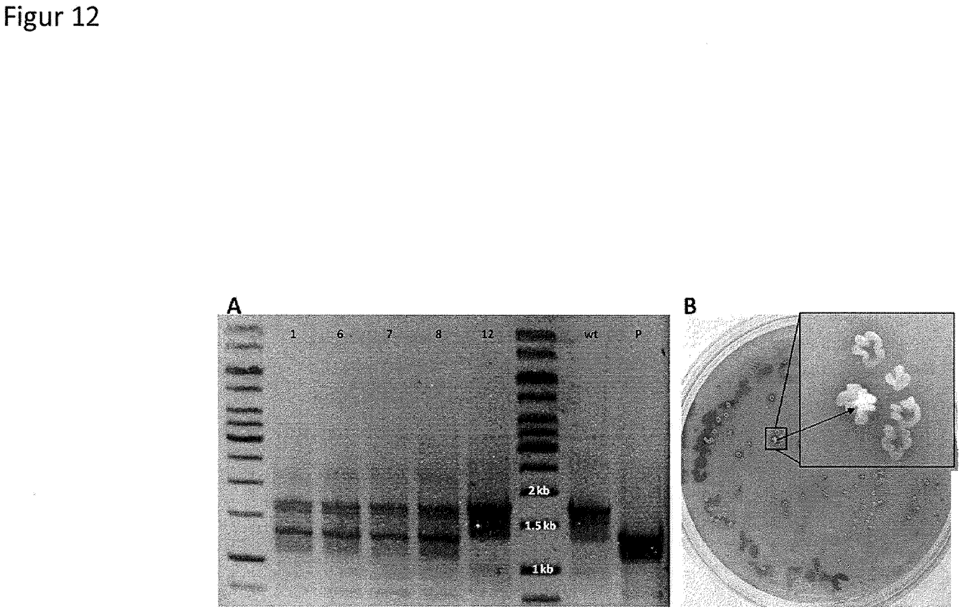

**Figur 12** zeigt die Verifizierung der Integration von pGusA21Δorf5768 in das Genom von *A. japonicum* nach direkter Transformation. (**A**) PCR-basierte Verifizierung der Integration von pGusA21Δorf5768. Erwartetes WT-Profil: 1677 bp (orf5768-SCO-wt-Frag); erwartetes integriertes pGusA21Δorf5768-Profil: 1293 bp (orf5768-SCO-single Frag). wt: Wildtyp gDNA Templat; P: isoliertes pGusA21Δorf5768 Templat. (**B**) Suche nach Klonen von *A. japonicum* mit verlorenem pGusA21Δorf5768 mit Hilfe des Gus-Reportersystems. Das Genom blauer Kolonien verfügt über das integrierte pGusA21Δorf5768-Plasmid, während es nicht-gefärbte Kolonien verloren haben.

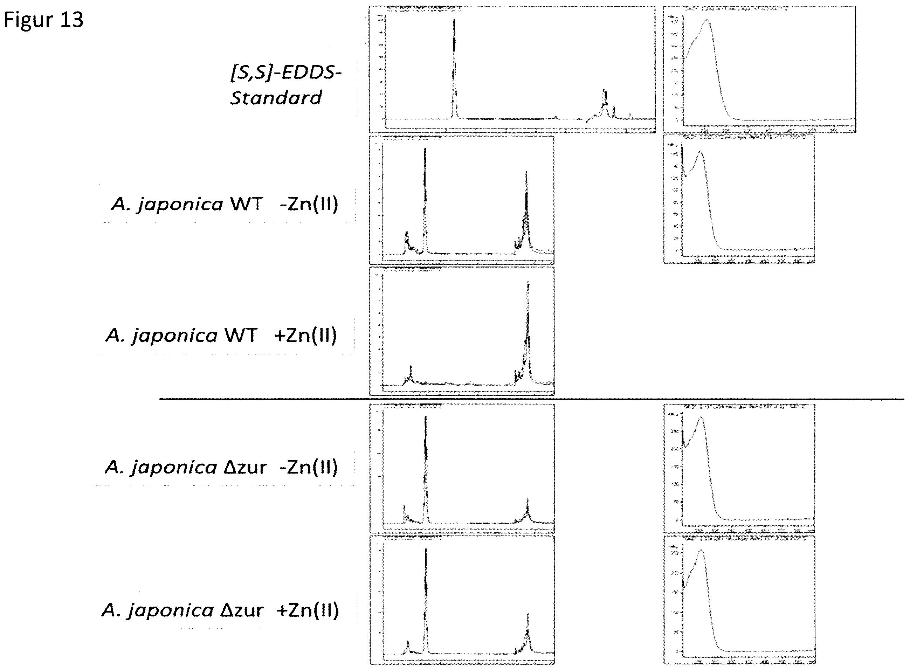

**Figur 13** zeigt die HPLC-UV/VIS-Analyse der Überstände von *A. japonicum* WT und *A. japonicum* Δzur (entspricht ΔSEQ ID Nr. 62) nach 72 h Kultur in M7-Medium mit und ohne Zusatz von 6 µM ZnSO₄. (Oben) [S,S]-EDDS-Standard [350 mg/L]. (2. und 3. von oben) *A. japonicum* WT -/+ 6 µM ZnSO₄. (Unten) *A. japonicum* Δzur -/+ 6 µM ZnSO₄. (Rechts) Spezifische UV/VIS-Spektren von [S,S]-EDDS.

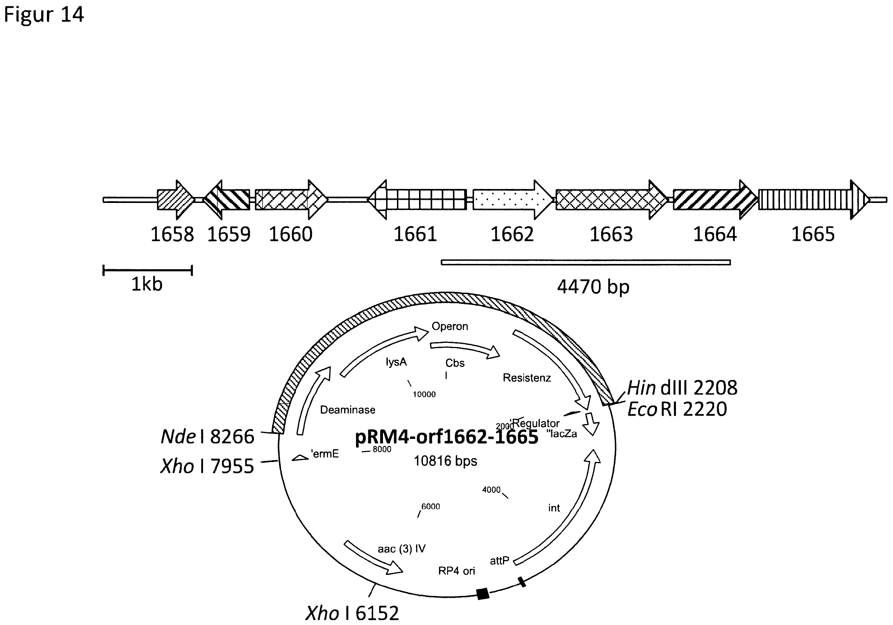

**Figur 14** zeigt schematisch die Konstruktion des Vektors pRM4-PermE*orf1662-65. (Oben) [S,S]-EDDS-Gen-Cluster orf1658-1665 (entspricht SEQ ID Nr. 40, 42, 44, 46, 48, 50, 52 und 54). (Unten) Darstellung des homologen Expressionsvektors pRM4-PermE*orf1662-65 als Plasmidkarte (orf1662 bis orf1665 unter der Kontrolle von PermE*. Grauer Balken: Operon orf1662 bis orf1665.

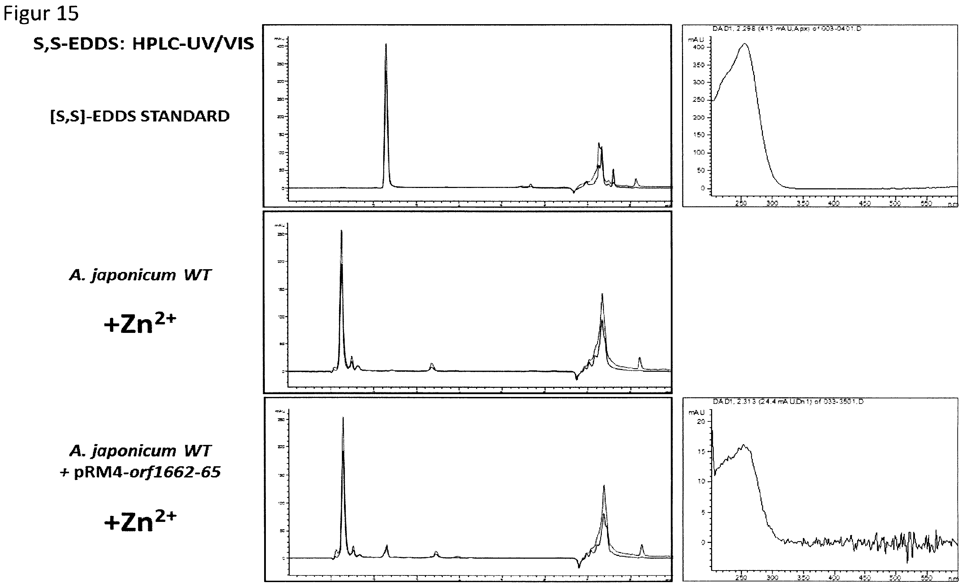

**Figur 15** zeigt die HPLC-UV/VIS-Analyse der Überstände von *A. japonicum* WT und *A. japonicum* + pRM4-PermE*orf1662-65 nach 72 h Kultur in M7-Medium mit einem Zusatz von 6 µM ZnSO₄. (Oben) [S,S]-EDDS-Standard [350 mg/L]. (Mitte) *A. japonicum* WT + 6 µM ZnSO₄. (Unten) *A. japonicum* pRM4-PermE*orf1662-65 + 6 µM ZnSO₄. (Rechts) Spezifische UV/VIS-Spektren von [S,S]-EDDS.

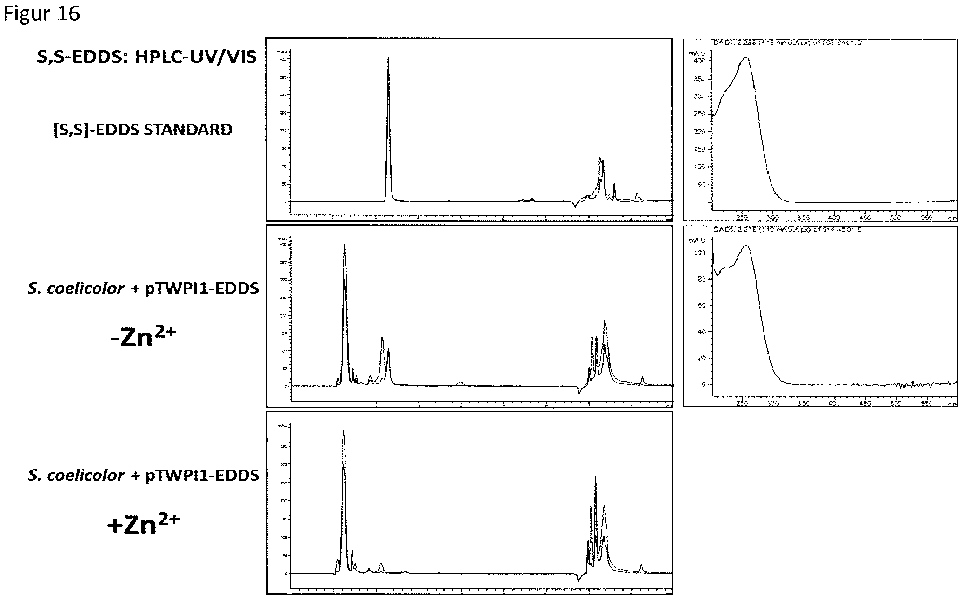

**Figur 16** zeigt die HPLC-UV/VIS-Analyse der Überstände von *S. coelicolor* + pTWPL1-EDDS nach 72 h Kultur in M7-Medium mit einem Zusatz von 6 µM ZnSO₄. (Oben) [S,S]-EDDS-Standard [350 mg/L]. (Mitte) *S. coelicolor* + pTWPL1-EDDS - 6 µM ZnSO₄. (Unten) *S. coelicolor* + pTWPL1-EDDS + 6 µM ZnSO₄. (Rechts) Spezifische UV/VIS-Spektren von [S,S]-EDDS.

### Beispielteil

### 1. Aufklärung der Biosynthese von [S,S]-EDDS

Das Genom der [S,S]-EDDS produzierenden Bakterienart *A. japonicum* wurde im Rahmen der vorliegenden Erfindung sequenziert. Das Genom von *A. japonicum* umfasst ungefähr 9,18 MB und beinhaltet 8674 vorhergesagte offene Leserahmen (Open Reading Frames, orf). Gemäß dem Webtool Antibiotics and Secondary Metabolite Analysis Shell (antiSMASH) zur schnellen Identifizierung, Annotation und Analyse von Biosynthesegen-Clustern für sekundäre Metaboliten (Medema et al., 2011, Nucleic Acids Res (2011); 39:14 und Blin et al., Nucleic Acids Res (2013); 1-9), weist das Genom von *A. japonicum* 26 einzelne Gen-Cluster für sekundäre Metaboliten auf. Diese Cluster enthalten das biosynthetische Potential zur Produktion von sekundäre Metaboliten abgeleitet von sechs NRPS (Nicht-Ribosomale Peptidsynthetasen), sechs Typ I PKS (Polyketidsynthase), zwei Typ I PKS/NRPS-Hybriden und einem Typ III PKS/NRPS-Hybriden (zur Produktion eines Glycopeptids) neben vier Terpenen, einem Ectoin, einem Aminoglycosid und einem Lantibioticum.

Es wurde zunächst ein hypothetischer Biosyntheseweg von [S,S]-EDDS postuliert (Figur 1), nach welchem die nicht-proteinogene Aminosäure 2,3-L-Diaminopropionat (Dap) eine putative Vorläuferverbindung von [S,S]-EDDS sein könnte. Dap ist unter anderem ein Sekudärmetabolit bei der Biosynthese von Viomycin durch *Streptomyces vinaceus* und der Biosynthese von Zwittermicin A durch *Bacillus thuringiensis.* Beide Synthesewege sind aufgeklärt (Thomas et al., Antimicrobial Agents and Chemotherapy (2003); 47(9): 2823-2830 und Zhao et al., FEBS Lett. (2008); 528(20): 3125-3131).

Für *B. thuringiensis* und *S. vinaceus* wurde gezeigt, dass Dap spezifisch für die Biosynthese von Zwittermicin A bzw. Viomycin bereitgestellt wird. Dap wird sowohl bei der Biosynthese von Zwittermicin A als auch von Viomycin durch die Umwandlung von L-Serin mit L-Ornithin als Aminodonator gebildet (Figur 1). Diese Reaktion wird durch die konzertierte Wirkung einer Dap-Synthase (VioB/ZWA5A) und einer Ornithin-Cyclodeaminase (VioK/ZWA5B) katalysiert, wobei Pyridoxalphosphat (PLP) als Cofaktor erforderlich ist (Thomas et al., Antimicrobial Agents and Chemotherapy (2003); 47(9): 2823-2830 und Zhao et al., FEBS Lett. (2008); 528(20): 3125-3131).

Ein Screening des Genoms von *A. japonicum* unter Verwendung von BLAST (Basic Local Alignment Search Tool, in der zum Anmeldezeitpunkt gültigen Fassung) mit den Aminosäuresequenzen von VioB/ ZWA5A bzw. VioK/ZWA5B zeigte das Vorliegen von homologen Proteinen auf, die von Genen codiert werden, die in direkter Nähe zueinander liegen. So codiert die Nukleinsäure gemäß SEQ ID Nr. 52 für ein Protein mit einer Größe von 352 aa und zeigt 32/45 %, 26/44 % aa-Identität/ Ähnlichkeit zu VioB bzw. ZWA5A. SEQ ID Nr. 48 codiert für ein Protein mit einer Größe von 327 aa und zeigt 25/39 %, 21/40 % aa-Identität/ Ähnlichkeit zu VioK bzw. ZWA5B. Die dazwischenliegende SEQ ID Nr. 50 wird als Diaminopimelat-Decarboxylase und SEQ ID Nr. 54 als Multi-Drug-Effluxtransporter bezeichnet. SEQ ID Nr. 48, SEQ ID Nr. 50, SEQ ID Nr. 52 und SEQ ID Nr. 54 zeigen eine überlappende Genanordnung und sind vermutlich als Transkriptionseinheit codiert (Figur 2).

Die Proteine oder Peptide nach SEQ ID Nr. 39, SEQ ID Nr. 41, SEQ ID Nr. 43 und SEQ ID Nr. 45 sind am 5'-Ende strangaufwärts der zuvor genannten operonartig organisierten Gene codiert und werden als N-Acetyltransferase, Cystein-Dioxygenase, Transkriptionsregulator vom HTH-Typ bzw. Amidase bezeichnet (Figur 2).

Ausgehend von den oben ermittelten Homologien wurden die folgenden Annahmen gemacht:
- Die Proteine oder Peptide gemäß SEQ ID Nr. 47 und SEQ ID Nr. 51 katalysieren in konzertierter Aktion die Synthese der Dap-Vorstufe als einen ersten Schritt der Biosynthese von [S,S]-EDDS (Figur 2).
- Die Kondensation von Dap mit Oxalacetat zur Zwischenstufenverbindung IM1 wird von einem Protein oder Peptid nach SEQ ID Nr. 39 (Nukleinsäure gemäß SEQ ID Nr. 40: als N-Acetyl-Transferase bezeichnet) katalysiert.
- Die anschließende Decarboxylierung wird von einem Protein oder Peptid nach SEQ ID Nr. 49 (SEQ ID Nr. 48: als Diaminopimelat-Decarboxylase bezeichnet) katalysiert.
- Die erneute Acetylierung mit Oxalacetat wiederum von einem Protein oder Peptid gemäß SEQ ID Nr. 39.
- Die abschließende Reduktion wird von einem Protein oder Peptid nach SEQ ID Nr. 41 (SEQ ID Nr. 42: als Cystein-Dioxygenase bezeichnet) katalysiert.
- Zum Zweck der Sekretion muss [S,S]-EDDS ferner durch die Zellmembran geschleust werden. Dieser Export wird vom putativen Multi-Drug-Effluxtransporter gemäß SEQ ID Nr. 53 vermittelt (Figur 2).

Zur Demonstration, dass die identifizierte Region für die Biosynthese von [S,S]-EDDS tatsächlich verantwortlich ist, wurde die Zinkrepression der [S,S]-EDDS-Biosynthese genutzt. Da [S,S]-EDDS ausschließlich unter im Wesentlichen zinkfreien Bedingungen gebildet wird (bereits eine 2 µM Zink-Konzentration bewirkt eine annähernd vollständige Unterbrechung der [S,S]-EDDS Synthese (Cebulla I., Dissertation (1995), Universität Tübingen), wurde das Transkriptionsmuster der putativen Biosynthesegene mittels RT-PCR (Real Time Polymerase Chain Reaction), bei Zinkanwesenheit und Zinkabwesenheit bestimmt (Figur 3).

Die putativen Dap-Synthesegene mit den Nukleinsäuresequenzen gemäß SEQ ID Nr. 48 und SEQ ID Nr. 52, die dazwischenliegende Sequenz nach SEQ ID Nr. 50 sowie die Sequenz gemäß SEQ ID Nr. 54 wurden ausschließlich während einer Kultivierung unter Zinkabwesenheit exprimiert ([S,S]-EDDS-Bildung), jedoch nicht in Gegenwart von Zink. Es konnte keine Repression dieser Gene durch andere zweiwertige Metallionen festgestellt werden. Die Nukleinsäuren gemäß SEQ ID Nr. 40, SEQ ID Nr. 42, SEQ ID Nr. 44 und SEQ ID Nr. 46 zeigen ein gemeinsames Transkriptionsmuster, das nicht durch Zink beeinflusst wird (Figur 3).

Das so identifizierte Operon der putativen [S,S]-EDDS-Biosynthese (SEQ ID Nr. 48, 50, 52 und 54) zeigt also eine zinkabhängige Transkription.

Zum Nachweis einer Beteiligung der stark durch Zink beeinflussten Gene gemäß SEQ ID Nr. 48, 50 und 52 bei der Synthese von [S,S]-EDDS wurde ferner eine Mutante mit einer Deletion im Leserahmen (In-Frame-Deletion) der codierenden Regionen SEQ ID Nr. 48, 50 und 52 erzeugt.

Insgesamt wurden 12 Mutanten von *A. japonicum* (*A. japonicum* Δ SEQ ID Nr. 48, 50 und 52) mit einer In-Frame-Deletion der codierenden Regionen SEQ ID Nr. 48, 50 und 52 erzeugt. *A. japonicum* vom Wildtyp (*A. japonicum* WT) und alle erzeugten Mutanten wurden in EDDS-Produktionsmedium (vgl. Tabelle 1) kultiviert.

**Tabelle 1: Kulturmedien, Puffer**

| | |
|---|---|
| M7-Medium (EDDS Produktionsmedium) | 11,3 g Natriumglutamat |
| | 8,0 g Kaliumdihydrogenphosphat |
| | 12,0 g Dinatriumhydrogenphosphat |
| | 1 ml Antischaumlösung |
| | 25,0 g Glycerin |
| | 1,2 Magnesiumsulfat |
| | 60 mg Eisen(III)citrat |
| M3-Medium | 20,0 g Glycerin |
| | 20,0 g Sojamehl |
| | pH 7,5 |
| M2-Medium | 50,0 g Natriumglutamat |
| | 50,0 g Saccharose |
| | 50,0 g Dextran |
| | pH 7,2 |
| TSB-Medium | 30,0 g TSB-Pulver |
| (Bacto® Tryptic Soy Broth Soybean-Casein Digest Medium; Becton, Dickinson, Co.) | |
| EMSA-Bindungspuffer | 80 mM Tris/HCl (pH 7,8) |
| | 200 mM KCL |
| | 20% Glycerol |

Während der Stamm *A. japonicum* WT unter den gewählten Bedingungen [S,S]-EDDS bildet, war keine der 12 Mutanten mehr in der Lage, noch [S,S]-EDDS zu synthetisieren (Figur 4) .

Es konnte also gezeigt werden, dass die Operon-Struktur enthaltend oder bestehend aus einer Nukleinsäuresequenz gemäß SEQ ID Nr. 48, 50 und 52 für die [S,S]-EDDS-Biosynthese in *A. japonicum* erforderlich ist.

Zur Demonstration, dass das identifizierte Gen-Cluster alle erforderlichen genetischen Informationen für die Biosynthese von [S,S]-EDDS enthält, wurde in *S. coelicolor* ein Cosmid heterolog exprimiert, das den gesamten Gen-Cluster umfasst. Dieses Cosmid (pTWPL1-EDDS) enthält die Gene nach SEQ ID Nr. 4, 6, 8, 10, 12, 14, 16, 18, 20, 22, 24, 26, 28, 30, 32, 34, 36, 38, 40, 42, 44, 46, 48, 50, 52, 54, 56 und 58 sowie Teile gemäß SEQ ID Nr. 2 und SEQ ID Nr. 60 (Tabelle 2).

**Tabelle 2: 30 zuvor nicht beschriebene Gene von A. japonicum**

| ***orf*** | **SEQ ID Nr.** | **Hypothetische Funktion** |
|---|---|---|
| *1639* | 2 | 2-Isopropylmalat-Synthase |
| *1640* | 4 | Fumarylacetoacetat-Hydrolase-Familie Protein |
| *1641* | 6 | tRNA Uridin-5-carboxymethylaminomethyl-Modifikationsenzym mnmG |
| *1642* | 8 | Phosphoglycolat-Phosphatase |
| *1643* | 10 | hypothetisches Protein |
| *1644* | 12 | hypothetisches Protein |
| *1645* | 14 | Alcohol-Dehydrogenase-Superfamilie, Zink-enthaltend; L-Threonin-3-dehydrogenase |
| *1646* | 16 | hypothetisches Protein |
| *1647* | 18 | Transkriptionsregulator, PadR-like Familie |
| *1648* | 20 | hypothetisches Protein |
| *1649* | 22 | major facilitator Superfamilie MFS_1 |
| *1650* | 24 | major facilitator Superfamilie MFS_1 |
| *1651* | 26 | Transkriptionsregulator HTH, ArsR |
| *1652* | 28 | Bialaphos-Biosyntheseweg-Regulatorprotein |
| *1653* | 30 | Furin (EC=3.4.21.75) |
| *1654* | 32 | hypothetisches Protein |
| *1655* | 34 | Helix-turn-helix type 3 |
| *1656* | 36 | hypothetisches Protein |
| *1657* | 38 | Zweikomponenten Transkriptionsregulator, LuxR Familie |
| *1658* | 40 | N-Acetyltransferase |
| *1659* | 42 | Cystein-Dioxygenase |
| *1660* | 44 | HTH-typ Transkriptionsregulator |
| *1661* | 46 | Amidase |
| *1662* | 48 | Ornithin-Cyclodeaminase |
| *1663* | 50 | Diaminopimelat-Decarboxylase |
| *1664* | 52 | Cystathionin-beta-synthase |
| *1665* | 54 | Transporter-Protein |
| *1666* | 56 | Sporulationsprotein |
| *1667* | 58 | Eisenaufnahme-Regulationprotein |
| *1668* | 60 | Katalase/Peroxidase [until nt 1512] |

Der Stamm *S. coelicolor* vom Wildtyp (*S. coelicolor* WT) und der Stamm *S. coelicolor* mit dem im Genom integrierten Cosmid pTWPL1 -EDDS (*S. coelicolor*-pTWPL1-EDDS) wurde in EDDS-Produktionsmedium kultiviert. Während der Stamm *S. coelicolor* WT nicht in der Lage ist, unter den gewählten Bedingungen [S,S]-EDDS zu produzieren, produziert *S. coelicolor-*pTWPL1-EDDS nachweisbare Mengen an [S,S]-EDDS in zinkfreiem EDDS-Produktionsmedium (Figur 5).

Die Bildung von [S,S]-EDDS durch *S. coelicolor*-pTWPL1-EDDS zeigt, dass alle für die Biosynthese von [S,S]-EDDS erforderlichen Enzyme in der Genregion orf1640-1667 bzw. SEQ ID Nr. 4, 6, 8, 10, 12, 14, 16, 18, 20, 22, 24, 26, 28, 30, 32, 34, 36, 38, 40, 42, 44, 46, 48, 50, 52, 54, 56 und 58 codiert sind.

### 2. Aufklärung der Zinkrepression der Biosynthese von [S,S]-EDDS

Die Fur (Ferric uptake regulator)-Familie der globalen metallregulatorischen Proteine ist nach dem Ferric Uptake Regulator, Fur von *E. coli* benannt (Hantke K., Mol Gen Genet (1981); 182(2): 288-292 und Bagg & Neilands, Biochemistry (1987); 26: 5471-5477). Neben dem Eisen-selektiven Fur gibt es eine große Vielfalt an Metallselektivität und biologischen Funktionen innerhalb der Fur-Familie. Darunter befinden sich beispielsweise Sensoren für Zink (Zur), Mangan (Mur) und Nickel (Nur).

Fur-Proteine sind in der Regel Transkriptionsrepressoren, die an korrespondierende Operator-DNA-Sequenzen binden, wenn sie mit ihren kognaten Metallioneneffektoren (beispielsweise wird Eisen-gebundenes Fur als *holo*Fur bezeichnet) verbunden sind, wodurch der Zugriff von RNA-Polymerase verhindert wird, was zu einer Hemmung (Repression) der Expression von nachgeordneten (strangabwärts gelegenen) Genen führt (Lee & Helmann, Nature (2006); 440(7082):363-367). Die metallfreien Proteine (beispielsweise apoFur) besitzen grundsätzlich eine geringe oder vernachlässigbare Affinität für die entsprechenden Operatorsequenz, was zu einer Enthemmung (Derepression) der Zielgene führt.

Die Fähigkeit der Fur-Familienmitglieder, physiologisch als Sensoren für Zn(II)-Ionen zu dienen, wurde übereinstimmend bei den grampositiven *B. subtilis* mit niedrigem GC-Gehalt (Zur_{BS}) und γ-Proteobakterien *E. coli* (Zur_{EC}) entdeckt (Gaballa & Helmann, J. Bacteriol. (1998); 180:5815-21 und Patzer & Hantke, MolMicrobiol (1998); 28: 1199-1210). Anschließend haben Genomanalysen vorläufige Zuordnungen von wahrscheinlichen Zur-Regulons bei zahlreichen anderen Bakterien ermöglicht, was die Verbreitung einer durch Zur vermittelten Zinkaufnahme-Regulation im Bakterienreich aufzeigt (Panina et al., Proc Natl Acad Sci U S A (2003 Aug 19); 100(17): 9912-7). In der Zwischenzeit wurden neben Zur_{BS} und Zur_{EC} eine Vielzahl von Zur-Proteinen biochemisch charakterisiert, wie für die gramnegativen *Yersinia pestis* (Zur_{YP}), *Salmonella enterica* (Zur_{SE}) und *Xanthomonas campestris* (Zur_{XC}), die grampositiven Firmicutes mit niedrigem GC-Gehalt *Staphylococcus aureus* (Zur_{SA}), *Streptococcus suis* (Zur_{SS}) und *Listeria monocytogenes* (Zur_{LM}) und grampositive Actinobakterien mit hohem GC-Gehalt *Mycobacterium tuberculosis* (Zur_{MT}), *Corynebacterium diphtheriae* (Zur_{CD}), *Corynebacterium glutamicum* (Zur_{CG}) und *S. coelicolor* (Zur_{SC}) (Li et al., BMC Microbiol. (2009 Jun 25); 9:128, Feng et al., J Bacteriol. (2008); 190(22): 7567-78, Lindsay & Foster, Microbiology (2001), 147, 1259-66, Campoy et al., Infect. Immun. (2002); 70: 4721-5, Garrido et al., FEMSMicrobiol. Lett. (2003); 221: 31-37, Tang et al., Mol. Plant-Microbe Interact. (2005); 18: 652-8, Maciag et al., J Bacteriol. (2007): 189(3): 730-40, Shin et al., Journal of Bacteriology (2007); June: 4070-7, Dalet et al., FEMS Microbiol. Lett. (1999); 174: 111-6, Schröder et al., BMC Genomics (2010); 11: 12 und Smith et al., J Bacteriol. (2009); 191(5): 1595-603).

Als Regulon wird eine Gruppe von Genen bezeichnet, deren Aktivität vom gleichen Regulator kontrolliert wird. Regulons unter der Kontrolle von Zur-Proteinen beinhalten Gene, die für Zinkakquisitionsfunktionen codieren, wie beispielsweise Zinkaufnahmesysteme mit hoher Affinität (znuABC), putative Zinkophore und zinkfreie Paraloge von Ribosomenproteinen. Diese Gene erfahren eine Repression durch Bindung der zinkgebundenen *holo*Zur-Proteine unter zinkreichen Bedingungen und Derepression durch Dissoziation des zinkfreien *apo*Zur-Proteins von der DNA.

*S. coelicolor,* ein Modellorganismus der Actinomyceten mit hohem GC-Gehalt, reguliert die Metallhomöostase und die Peroxid-Stress-Antwort unter anderem mit vier biochemisch charakterisierten Proteinen der Fur-Familie (FurA_{SC}, CatR_{SC}, Nur_{SC} und Zur_{SC}) (Hahn et al., J Bacteriol. (2000); 182(13): 3767-74, Ahn et al., Mol. Microbiol. (2006); 59:1848-58 und Shin et al., Journal of Bacteriology (2007); June: 4070-7).

Eine BLAST-Analyse von *A. japonicum* mit den aa-Sequenzen der vier einzelnen Fur-Familienproteine von *S.coelicolor* zeigte, dass das Genom von *A. japonicum* für drei Proteinhomologe der Fur-Familie codiert, entsprechend den Nukleinsäuresequenzen orf1667 (entspricht SEQ ID Nr. 58), orf3462 und orf5768 (entspricht SEQ ID Nr. 62) codiert werden.

Die Fur-Familienhomologen ORF1667 (entspricht SEQ ID Nr. 57) und ORF3462 sind *S. coelicolor* FurA_{SC} sehr ähnlich (62/75 % und 67/79 % aa-Identität/Ähnlichkeit). *S. coelicolor* FurA_{SC} ist an der adaptiven Antwort auf Peroxid-Stress beteiligt und übt eine negative Regulation auf ein Operon mit dem furA_{SC}-Gen selbst und catC aus, das für eine Catalase-Peroxidase codiert (Hahn et al., J Biol Chem. (2000); 275(49): 38254-60).

Das dritte Homologe Protein der Fur-Familie mit einer Aminosäuresequenz gemäß SEQ ID Nr. 61 ist *S. coelicolor* Zur_{SC} sehr ähnlich und zeigt zu diesem eine 67/85 % aa-Identität/Ähnlichkeit (Figur 6).

Die hohe Ähnlichkeit des biochemisch charakterisierten Zur_{SC} von *S. coelicolor* und des durch eine Nukleinsäuresequenz gemäß SEQ ID Nr. 62 (bzw. orf5768) codierten *A. japonicum* Homologen (Zur_{AJ}) impliziert, dass das Protein oder Peptid gemäß SEQ ID Nr. 61 das zinksensitive Protein der Fur-Familie von *A. japonicum* ist, welches eine zinkabhängige Repression von korrespondierenden Zielgenen vermittelt.

Zur_{SC} ist der negative Regulator des Zinkaufnahmesystems mit hoher Affinität (ZnuABC) in *S. coelicolor* (Shin et al., Journal of Bacteriology (2007); June: 4070-7). Eine BLAST-Analyse des Genoms von *A. japonicum* mit den Aminosäuresequenzen von *S. coelicolor* ZnuABC zeigte zwei Homologensysteme auf, die von orf3699, orf3700 und orf3702 bzw. orf6504, orf6505 und orf6506 codiert werden (Figur 7).

Es ist bekannt, dass die Expression des znuABC-Operons von *S. coelicolor* unter strenger Repression von Zur_{SC} steht, wobei Zink ein Cofaktor ist (Shin et al., Journal of Bacteriology (2007); June: 4070-7). Daher wurde die Transkription von orf3700 und orf6504 als Repräsentanten der gesamten Systeme in Abhängigkeit von der Gegenwart von Zink sowie verschiedener anderer Metalle untersucht, um festzustellen, welches der beiden putativen Zinkaufnahmesysteme die hochaffine Zinkaufnahme in *A. japonicum* vermittelt (Figur 8).

Der DNA-Abschnitt orf3700 wurde unter Metallmangel sowie bei Anwesenheit von Eisen, Nickel, Cobalt und exprimiert, jedoch nicht bei Anwesenheit von Zink (Figur 8). Dieses Transkriptionsmuster zeigte deutlich die strenge und ausschließlich durch Zink vermittelte Repression von orf3700 in *A. japonicum.* Daraus konnte geschlossen werden, dass das von orf3699, orf3700 und orf3702 codierte Aufnahmesystem das Zinkaufnahmesystem mit hoher Affinität (ZnuABC) von *A. japonicum* darstellt.

Um zu bestätigen, dass das zu Zur_{SC} homologe Protein gemäß SEQ ID Nr. 61 (codiert durch SEQ ID Nr. 62) das auf Zink reagierende Protein der Fur-Familie von *A. japonicum* ist und die zinkabhängige Repression des Zinkaufnahmesystems (znuABC) und der putativen [S,S]-EDDS-Biosynthesegene vermittelt, wurden Affinitätselektrophorese-Untersuchungen durchgeführt (EMS-Assays, Elektrophoretic Mobility Shift Assays)

Die Bindung des Proteins oder Peptids gemäß SEQ ID Nr. 61 wurde unter Verwendung der strangaufwärts am 5'-Ende gelegenen Promotorregion der Zinkrepressionsgene orf3700 und orf1662 (entspricht SEQ ID Nr. 46) und ebenso aller anderen vorhergesagten Promotorregionen im putativen [S,S]-EDDS-Biosynthesegen-Cluster (strangaufwärts-Region von orf1658, Zwischengenregion von orf1659 und orf1660, Zwischengenregion von orf1661 und orf1662) in Abhängigkeit von Zink als Cofaktor untersucht (Figur 9).

Um zu untersuchen, ob der homologe Zinkaufnahme-Regulator ORF5768 (Zur_{AJ}, entspricht SEQ ID Nr. 61) die zinkabhängige Repression von orf3700, orf1662 und der anderen Gene innerhalb des [S,S]-Biosynthesegen-Clusters durch Bindung an die entsprechenden Promotorregionen vermittelt (offene Kreise, Figur 9), wurden EMS Assays (Electrophoretic Mobility Shift Assay (EMS Assay) oder auch Band Shift Assay) durchgeführt. Dazu wurde der homologe Zinkaufnahme-Regulator ORF5768 mit einem Histidin₆-Tag versehen, aufgereinigt und isoliert. Ebenfalls bereitgestellt wurden die entsprechenden DNA-Sequenzen (Promotorsequenzen), die als vermeintlich zinkregulierte Promotoren untersucht werden sollten (offene Kreise, Figur 9).

Die Bindungsreaktion wurde ausgeführt, indem die Promotorsequenzen (etwa 35 nM) mit verschiedenen Mengen an His₆-ORF5768 in 10 µl Bindungspuffer (Tabelle 1) für 20 min bei 29°C mit oder ohne Zinkzusatz inkubiert wurden. Um unspezifische Bindungen von His₆-ORF5768 auszuschließen erfolgte die Zugabe eines sigB-RT-Fragments (258 bp) als eine Negativkontrolle (Figur 10).

So konnte mittels EMSA eine zinkabhängige Bindung (d.h. bei Anwesenheit von Zink) von His₆-ORF5768 an diejenigen EMSA-Sonden festgestellt werden, welche die znuABC-Promotorregion sowie die orf1659-60 und orf1661-62 Promotorregionen darstellen. Keine Bindung wurde an die orf1658 Promotorregion festgestellt (Figur 10).

### 3. Erzeugung eines [S,S]-EDDS-Produktionsstamms mit Zink-Derepression

Zur Erzeugung eines [S,S]-EDDS-Produktionsstamms von *A. japonicum* mit Zink-Derepression wurden drei Strategien verfolgt:
(1) Deletion des Zinkaufnahmeregulatorgens (und damit des Repressionsproteins Zur) SEQ ID Nr. 62.
(2) Expression der [S,S]-EDDS-Biosynthesegene unter der Kontrolle von Promotoren ohne Zink-Repression (None-Zinc-Repressed Promoters). Austausch der auf Zur gerichteten Promotoren.
(3) Heterologe Expression der [S,S]-EDDS-Biosynthesegene in Wirtszellen, bei denen die Zink-Repression nicht mehr vorhanden ist.

### 3.1 Deletion des Zinkaufnahmeregulatorgens SEQ ID Nr. 62

Unter der Annahme, dass die [S,S]-EDDS-Biosynthesegene eine Repression durch Zur_{AJ} (ORF5768 bzw. Protein oder Peptid nach SEQ ID Nr. 61) mit Zink als Cofaktor erfahren, wurde eine Deletion der codierenden Region gemäß SEQ ID Nr. 62 durchgeführt, um einen [S,S]-EDDS-Produktionsstamm mit Zink-Derepression zu erzeugen.

Um die In-Frame-Deletion der Codierungsregion nach SEQ ID Nr. 62 (entspricht orf5768) über eine homologe Rekombination zu erreichen, wurde der Deletionsvektor pGusA21Δorf5768 konstruiert, der die strangaufwärts- und strangabwärts-Region von orf5768 enthält, die pGusA21-us1662+ds1664 ähnlich ist (AG Stegmann). Ein nicht methyliertes negatives pGusA21Δorf5768 wurde von *E. coli* ET12567 gewonnen und zur direkten Transformation von *A. japonicum* verwendet (Figur 11).

Gegen Apramycin resistente Kolonien von *A. japonicum,* die nach der Transformation mit nicht methyliertem pGusA21Δorf5768 erhalten wurden, wurden auf HA-Platten übertragen. Wachsende Kolonien wurden mit X-Gluc (5-Bromo-4-chloro-3-indolyl-β-D-glucoronid)-Lösung überschichtet und blaue Kolonien (Figur 12, positiv auf das Reportergen gusA: Klon 1, 6, 7, 8, 12) wurden für eine weitere Kontrolle auf Fälle von einzelnem Crossing-over durch PCR ausgewählt.

Es wurde genomische DNA dieser Klone isoliert und Fälle von einzelnem Crossing-over der Plasmide in das Genom von *A. japonicum* wurden durch PCR auf das Vorliegen einer Apramycin-Resistenz-Kassette kontrolliert und mit Primern spezifisch deduziert, um eine Integration von pGusA21Δorf5768 in das Genom zu bestätigen. Unter Verwendung des Primerpaars aus orf5768-SCO-FP und orf5768-SCO-RP wurde ein Fragment mit 1677 bp (orf5768-SCO-wt-Frag) und/oder ein Fragment mit 1293 bp (orf5768-SCO-single Frag) amplifiziert, das das Wildtypgenom bzw. das Genom mit integriertem pGusA21Δorf5768 darstellt (Figur 12).

Die Klone von *A. japonicum* mit in das Genom integriertem pGusA21Δorf5768 (Klone 1, 6, 7 und 8) wurden vereint und zum Induzieren eines doppelten Crossing-over eingesetzt (Homologen-Rekombination), wobei die Zellen unter Temperaturbelastung kultiviert wurden.

Insgesamt wurden drei Mutanten von *A. japonicum* (*A. japonica* Δzur) in einer In-Frame-Deletion der codierenden Region SEQ ID Nr. 62 erzeugt. *A. japonicum* WT und alle erzeugten Mutanten wurden in EDDS-Produktionsmedium (vgl. Tabelle 1) kultiviert. Während der Stamm *A. japonicum* WT unter den zinkreichen Bedingungen kein [S,S]-EDDS bildete, zeigte keine der drei Mutanten eine Zink-Repression mehr (Figur 13).

Es konnte also gezeigt werden, dass die Deletion des auf Zink ansprechenden Repressors von *A. japonicum* (zur_{AJ} oder Nukleinsäuresequenz gemäß SEQ ID Nr. 62) zu einer zinkunabhängigen [S,S]-EDDS-Produktion führt.

### 3.2 Expression der [S,S]-EDDS-Biosynthesegene unter der Kontrolle von Promotoren ohne Zinkrepression

Eine zweite Strategie zur Erzeugung eines [S,S]-EDDS-Produktionsstamms mit Zink-Derepression sieht vor, die durch Zur (Proteins oder Peptids gemäß SEQ ID Nr. 61) vermittelte Zinkrepression durch einen Austausch der Zur-Zielpromotoren gegen relativ starke konstitutiv exprimierte oder induzierbare Promotoren zu vermeiden. Dazu wurde der bekannte, konstitutiv exprimierte Promotor *PermE** gewählt, um das [S,S]-EDDS-Operon (SEQ ID Nr. 48, 50, 52 und 54, entspricht orf1662-65) unter seiner Kontrolle zu exprimieren. Das erzeugte Plasmid pRM4-PermE*orf1662-65 wurde in *A. japonicum* WT transferiert und so *A. japonicum* + pRM4-PermE*orf1662-65 erhalten (Figur 14).

*A. japonicum* WT und *A. japonicum* + pRM4-PermE*orf1662-65 wurden dann in EDDS-Produktionsmedium (vgl. Tabelle 1), versetzt mit 6 µM ZnSO₄-Lösung, kultiviert. Die Daten gemäß Figur 15 zeigen, dass bei *A. japonicum* WT unter den gewählten Bedingungen keine [S,S]-EDDS-Produktion erfolgt. Dagegen ergibt sich bereits eine nachweisbare [S,S]-EDDS-Produktion in Anwesenheit von Zink durch den Stamm von *A. japonicum,* der das [S,S]-EDDS-Operon orf1662-65 unter der Kontrolle des nicht auf Zur gerichteten Promotors *ermE** exprimiert (Figur 15).

Diese Daten zeigen, dass eine deutliche zinkunabhängige [S,S]-EDDS-Produktion dadurch erreicht werden kann, dass die auf Zur_{AJ} (SEQ ID Nr. 61) gerichteten Promotoren, welche die [S,S]-EDDS-Biosynthesegene kontrollieren, ausgetauscht werden.

### 3.3 Heterologe Expression der [S,S]-EDDS-Biosynthesegene in heterologen Wirtszellen

Das [S,S]-EDDS-Biosynthese-Cluster soll in bereits biotechnologisch angewendeten Bakterienstämmen, beispielsweise *S. coelicolor,* exprimiert werden.

Es wurde daher der Stamm *S. coelicolor* mit dem im Genom integriertem Cosmid pTWPL1-EDDS in EDDS-Produktionsmedium kultiviert und auf eine [S,S]-EDDS-Produktion untersucht (Figur 16).

Die Daten bestätigen, dass [S,S]-EDDS durch eine heterologe Expression in dem Wirt *S. coelicolor* in zinkfreiem EDDS-Produktionsmedium (Figur 16, Mitte) synthetisiert werden kann. Zudem bestätigen diese Daten, dass es sich bei den im Rahmen der Erfindung identifizierten Genen um die [S,S]-EDDS-Biosynthesegene handelt.

### 4. Kultivierung und Aufbewahrung von A. japonicum

Zur Herstellung von Kulturen von *A. japonicum* wurden Lyophilisate auf HA-Platten ausplattiert und bei 27°C 4 bis 5 Tage inkubiert. Danach wurden 100 ml M3-Medium (vgl. Tabelle 1) mit dem Mycel beimpft und 48 h bei 27°C inkubiert. Die Kulturen wurden zweimal mit Salzlösung (0,9% NaCl) gewaschen. Der Niederschlag wurde in 50 ml M2-Medium (vgl. Tabelle 1) suspendiert, in Portionen von 2 ml aufgeteilt und bei -20°C bis zu 6 Monate aufbewahrt.

### 5. Kultur von A. japonicum zur DNA-Isolierung

20 ml TSB-Medium (vgl. Tabelle 1) wurden mit dem Mycel beimpft und für zwei Tage bei 27°C inkubiert. Es wurde ein disperses Wachstum und optimale Sauerstoffzufuhr in einem 100 ml Erlenmeyerkolben mit Schikane und einer Metallspirale erreicht.

### 6. Kultur von A. japonicum unter [S,S]-EDDS-Biosynthesebedingungen

*A. japonicum* wurde auf HA-Platten ausplattiert und bei 27°C für 3 bis 5 Tage inkubiert. Danach wurde etwa 1 cm² Mycel von *A. japonicum* von der Platte abgestrichen und zur Beimpfung von 100 ml M3-Medium (vgl. Tabelle 1) verwendet. Nach 48 h Inkubation bei 27°C und 180 Upm in einem Rüttler wurden 5 ml zum Beimpfen von 100 ml M7-Medium (vgl. Tabelle 1) verwendet. M7 ist ein synthetisches zinkarmes Medium, in dem *A. japonicum* [S,S]-EDDS produziert. Um eine Biosynthese von [S,S]-EDDS zu vermeiden, wurde ZnSO₄ bis zu einer Endkonzentration von 6 µM zugegeben. Für eine RT-PCR wurden ZnSO₄, FeSO₄, MnSO₄, NiSO₄ und CoCl₂ zugegeben, jeweils bis zu einer Endkonzentration von 25 µM (vgl. bspw. Figur 3).

Die in der vorliegenden Beschreibung genannten Nukleinsäure- und Aminosäuresequenzen entsprechen den im beigefügten Sequenzprotokoll aufgeführten Sequenzen.

## Patentansprüche

1. Isoliertes Protein oder Peptid, welches für einen Teilsyntheseschritt der Biosynthese von [S,S]-Ethylendiamindisuccinat funktional ist, enthaltend oder bestehend aus einer Aminosäuresequenz, die ausgewählt ist aus der Gruppe bestehend aus SEQ ID Nr. 39, SEQ ID Nr. 41, SEQ ID Nr. 43, SEQ ID Nr. 45, SEQ ID Nr. 47, SEQ ID Nr. 49, SEQ ID Nr. 51, SEQ ID Nr. 53 und Kombinationen davon.

2. Isolierte Nukleinsäure, enthaltend oder bestehend aus einer Nukleinsäuresequenz, die ausgewählt ist aus der Gruppe bestehend aus
a) eine Nukleinsäuresequenz, die für ein Protein oder Peptid nach Anspruch 1 codiert,
b) eine Nukleinsäuresequenz, die dem komplementären Strang der Nukleinsäuresequenz gemäß a) entspricht und Kombinationen davon.

3. Isolierte Nukleinsäure nach Anspruch 2, enthaltend oder bestehend aus einer Nukleinsäuresequenz, die ausgewählt ist aus der Gruppe bestehend aus SEQ ID Nr. 40, SEQ ID Nr. 42, SEQ ID Nr. 44, SEQ ID Nr. 46, SEQ ID Nr. 48, SEQ ID Nr. 50 , SEQ ID Nr. 52 , SEQ ID Nr. 54 und Kombinationen davon.

4. Isoliertes Gen-Cluster oder Operon, enthaltend oder bestehend aus wenigstens zwei Nukleinsäuresequenzen, die ausgewählt sind aus der Gruppe bestehend aus SEQ ID Nr. 40, SEQ ID Nr. 42, SEQ ID Nr. 44, SEQ ID Nr. 46, SEQ ID Nr. 48, SEQ ID Nr. 50, SEQ ID Nr. 52, SEQ ID Nr. 54 und Kombinationen davon.

5. Verwendung eines Proteins oder Peptids, enthaltend oder bestehend aus einer Aminosäuresequenz gemäß SEQ ID Nr. 61, für eine Repression der Biosynthese von [S,S]-Ethylendiamindisuccinat.

6. Expressionsvektor, enthaltend wenigstens eine Nukleinsäure nach Anspruch 2 oder 3 oder ein Gen-Cluster oder Operon gemäß Anspruch 4.

7. Rekombinante Wirtszelle, enthaltend einen Expressionsvektor nach Anspruch 6.

8. Verfahren für die Biosynthese von [S,S]-Ethylendiamindisuccinat, umfassend die Schritte:
a) Kultivieren einer [S,S]-Ethylendiamindisuccinat produzierenden Wirtszelle gemäß Anspruch 7 oder einer rekombinanten Bakterienzelle der Gattung *Amycolatopsis,* insbesondere von *Amycolatopsis japonicum,* welche kein Protein oder Peptid, enthaltend oder bestehend aus einer Aminosäuresequenz gemäß SEQ ID Nr. 61, exprimiert und/oder keine Nukleinsäure, enthaltend oder bestehend aus einer Nukleinsäuresequenz gemäß SEQ ID Nr. 62, enthält, und
b) Aufreinigen von [S,S]-Ethylendiamindisuccinat aus der Zelle und/ oder einem für die Zelle verwendeten Kulturmedium.

9. Kit für die Biosynthese von [S,S]-Ethylendiamindisuccinat, umfassend eine Komponente, die ausgewählt ist aus der Gruppe bestehend aus wenigstens ein Protein oder Peptid nach Anspruch 1, wenigstens eine Nukleinsäure nach Anspruch 2 oder 3, ein Gen-Cluster oder Operon nach Anspruch 4, einen Vektor nach Anspruch 6, eine Wirtszelle nach Anspruch 7und Kombinationen davon.

## Claims

1. Isolated protein or peptide, which is functional for a partial synthesis step of the biosynthesis of [S,S]-ethylenediamine-disuccinate, including or composed of an amino acid sequence selected from the group consisting of SEQ ID No. 39, SEQ ID No. 41, SEQ ID No. 43, SEQ ID No. 45, SEQ ID No. 47, SEQ ID No. 49, SEQ ID No. 51, SEQ ID No. 53, and combinations thereof.

2. Isolated nucleic acid, including or composed of a nucleic acid sequence selected from the group consisting of
a) a nucleic acid sequence encoding for a protein or peptide according to claim 1,
b) a nucleic acid sequence corresponding to the complementary strand of the nucleic acid sequence according to a), and combinations thereof.

3. Isolated nucleic acid according to claim 2, including or composed of a nucleic acid sequence selected from the group consisting of SEQ ID No. 40, SEQ ID No. 42, SEQ ID No. 44, SEQ ID No. 46, SEQ ID No. 48, SEQ ID No. 50, SEQ ID No. 52, SEQ ID No. 54, and combinations thereof.

4. Isolated gene cluster or operon, including or composed of at least two nucleic acid sequences selected from the group consisting of SEQ ID No. 40, SEQ ID No. 42, SEQ ID No. 44, SEQ ID No. 46, SEQ ID No. 48, SEQ ID No. 50, SEQ ID No. 52, SEQ ID No. 54, and combinations thereof.

5. Use of an isolated protein or peptide, including or composed of an amino acid sequence according to SEQ ID No. 61 for repression of the biosynthesis of [S,S]-ethylenediamine-disuccinate.

6. Expression vector, including at least one nucleic acid according to claim 2 or 3, or a gene cluster or operon according to claim 4.

7. Recombinant host cell, including an expression vector according to claim 6.

8. Method for the biosynthesis of [S,S]-ethylenediamine-disuccinate, comprising the steps:
a) cultivation of a host cell producing [S,S]-ethylenediamine-disuccinate according to claim 7 or a recombinant bacterial cell of the genus *Amycolatopsis,* in particular *Amycolatopsis japonicum,* not expressing a protein or peptide, including or composed of an amino acid sequence according to SEQ ID No. 61, and/or not including a nucleic acid, including or composed of a nucleic acid sequence according to SEQ ID No. 62, and
b) purification of [S,S]-ethylenediamine-disuccinate from the cell and/or a culture medium used for the cell.

9. Kit for the biosynthesis of [S,S]-ethylenediamine-disuccinate, comprising a component selected from the group consisting of at least one protein or peptide according to claim 1, at least one nucleic acid according to claim 2 or 3, a gene cluster or operon according to claim 4, a vector according to claim 6, a host cell according to claim 7, and combinations thereof.

## Revendications

1. Protéine ou peptide isolé qui est fonctionnel pour une étape de synthèse partielle de la biosynthèse de [S,S]-éthylènediaminedisuccinate, contenant ou composé d'une séquence d'acides aminés qui est sélectionnée dans le groupe constitué de SEQ ID N° 39, SEQ ID N° 41, SEQ ID N° 43, SEQ ID N° 45, SEQ ID N° 47, SEQ ID N° 49, SEQ ID N° 51, SEQ ID N° 53 et de combinaisons de cela.

2. Acide nucléique isolé, contenant ou composé d'une séquence d'acides nucléiques qui est sélectionnée dans le groupe composé
a) d'une séquence d'acides nucléiques, codant une protéine ou un peptide selon la revendication 1,
b) d'une séquence d'acides nucléiques correspondant au brin complémentaire de la séquence d'acides nucléiques selon a) et des combinaisons de cela.

3. Acide nucléique isolée selon la revendication 2, contenant ou composé d'une séquence d'acides nucléiques qui est sélectionnée dans le groupe constitué de SEQ ID N° 40, SEQ ID N° 42, SEQ ID N° 44, SEQ ID N° 46, SEQ ID N° 48, SEQ ID N° 50, SEQ ID N° 52, SEQ ID N° 54 et de combinaisons de cela.

4. Batterie de gènes ou opéron isolé, contenant ou composé d'au moins deux séquences d'acides nucléiques qui sont sélectionnées dans le groupe constitué de SEQ ID N° 40, SEQ ID N° 42, SEQ ID N° 44, SEQ ID N° 46, SEQ ID N° 48, SEQ ID N° 50, SEQ ID N° 52, SEQ ID N° 54 et de combinaisons de cela.

5. Utilisation d'une protéine ou d'un peptide, contenant ou composé d'une séquence d'acides aminés selon SEQ ID N° 61, pour une répression de la biosynthèse de [S,S]-éthylènediaminedisuccinate.

6. Vecteur d'expression, contenant au moins un acide nucléique selon la revendication 2 ou 3 ou une batterie de gènes ou un opéron selon la revendication 4.

7. Cellule hôte recombinante, contenant un vecteur d'expression selon la revendication 6.

8. Procédé pour la biosynthèse de [S,S]-éthylènediaminedisuccinate, comprenant les étapes :
a) Culture d'une cellule hôte produisant [S,S]-éthylènediaminedisuccinate selon la revendication 7 ou d'une cellule bactérienne recombinante de l'espèce *Amycolatopsis,* en particulier de l'espèce *Amycolatopsis japonicum,* qui n'exprime pas de protéine ou peptide, comprenant ou composé d'une séquence d'acides aminés selon SEQ ID N° 61, et/ou ne contient pas d'acide nucléique, comprenant ou composé d'une séquence d'acides nucléiques selon SEQ ID N° 62, et
b) Purification de [S,S]-éthylènediaminedisuccinate à partir de la cellule et/ou d'un milieu de culture utilisé pour la cellule.

9. Kit pour la biosynthèse de [S,S]-éthylènediaminedisuccinate, comprenant une composante sélectionnée dans le groupe constitué d'au moins une protéine ou un peptide selon la revendication 1, au moins un acide nucléique selon la revendication 2 ou 3, une batterie de gènes ou un opéron selon la revendication 4, un vecteur selon la revendication 6, une cellule hôte selon la revendication 7 et des combinaisons de cela.
